# EUROPEAN PATENT APPLICATION

(11) **EP 3 428 641 A1**
(43) Date of publication of application: **16.01.2019**
(21) Application number: 17756841.7
(22) Date of filing: 23.02.2017
(51) Int. Cl.: G01N 33/483, G01N 33/49, G01N 33/487, G01N 1/30, G01N 15/14, G01N 1/31, G01N 33/58, G01N 33/60

(54) **METHOD AND APPARATUS FOR CTC DIAGNOSIS USING PATCH**

(30) Priority: 23.02.2016 US 201662298959 P; 04.06.2016 KR 20160069936; 04.06.2016 KR 20160069937; 04.06.2016 KR 20160069938; 27.07.2016 KR 20160095739; 13.09.2016 KR 20160118462; 01.11.2016 KR 20160144551
(71) Applicant: Noul Co., Ltd., Yongin-si, Gyeonggi-do 16827 (KR)
(72) Inventor: LEE, Dong Young, Yongin-si, Gyeonggi-do 16923 (KR); LIM, Chan Yang, Seongnam-si, Gyeonggi-do 13481 (KR); KIM, Kyung Hwan, Yongin-si, Gyeonggi-do 17103 (KR)
(74) Representative: Kleine, Hubertus
(86) International application number: PCT/KR2017/002027
(87) International publication number: WO 2017/146503

(57) **Abstract**

According to an aspect of the present application, there is provided a diagnostic method for performing diagnosis on tumor cells included in a sample by using a patch which is provided as a gel type having a net-like structure forming micro-cavities, the diagnostic method including placing the sample on a plate, and using the patch that contains reagents used to detect cancer to provide the reagents contained in the patch to the plate.

## Description

### [Technical Field]

The present application relates to a method of and device for Circulating Tumor Cell (CTC) diagnosis using a patch, and more particularly, to a method of and device for CTC diagnosis using a patch for detecting tumor cells that float in blood and performing cancer diagnosis.

### [Background Art]

Circulating Tumor Cell (CTC) testing is for detecting circulating tumor cell that float in the blood, and various diagnostic methods for blood may be used.

In the case of conventional cancer diagnosis, various diagnoses are performed using a biopsy sample which is a piece of tissue removed from a person subject to diagnosis. Generally, a biopsy sample being acquired through surgery has sometimes been a cause of unwillingness to receive cancer screening felt by a person subject to diagnosis. Such a complex procedure and psychological burden make it more difficult to detect cancer at an early stage.

CTC testing is non-invasive testing for performing cancer diagnosis using a blood sample collected from the blood of a person subject to diagnosis. Therefore, a simple cancer diagnosis may be possible without procedures like surgery. Further, since CTC testing has an advantage of being able to predict whether cancer metastasis will occur, research has been more vigorously carried out on CTC testing.

In conventional CTC testing, a general immunoassay is performed on the blood to identify the onset of cancer. For example, in a process in which an antibody is injected into the blood to detect an antigen to be diagnosed, a washing process in which a large amount of washing solution is poured on a plate or the like to rinse it in order to remove unbound antibodies or other factors that interfere with the detection is necessarily required. Accordingly, there are problems in that a large amount of washing solution is wasted, and binding between an antibody and an antigen is re-separated.

Therefore, a means for improving the accuracy of diagnosis while minimizing an amount of reagents used in the diagnosis is required for CTC testing.

### [Disclosure]

### [Technical Problem]

An aspect of the present disclosure is to provide a patch capable of storing a substance.

An aspect of the present disclosure is to provide a patch capable of providing a reaction space for a substance.

An aspect of the present disclosure is to provide a patch capable of providing a substance.

An aspect of the present disclosure is to provide a patch capable of absorbing a substance.

An aspect of the present disclosure is to provide a patch capable of providing an environment.

An aspect of the present disclosure is to provide a Circulating Tumor Cell (CTC) testing method.

### [Technical Solution]

According to an aspect of the present application, there is provided a diagnostic method for performing diagnosis on tumor cells included in a sample by using a patch which is provided as a gel type having a net-like structure forming micro-cavities, the diagnostic method including placing the sample on a plate and using the patch, which contains reagents used to detect cancer, to provide the reagents contained in the patch to the plate.

According to another aspect of the present application, there is provided a diagnostic method for performing diagnosis on tumor cells included in a sample by using a patch which is provided as a gel type having a net-like structure forming micro-cavities, the diagnostic method including placing the sample on a plate, using a first patch, which contains a first reagent used to detect cancer, to provide the reagent contained in the first patch to the plate, and using a second patch, which contains a second reagent used to detect the cancer, to provide the reagent contained in the second patch to the plate.

According to still another aspect of the present application, there is provided a diagnostic device for performing diagnosis on tumor cells included in a sample by using a patch which is provided as a gel type having a net-like structure forming micro-cavities and contains reagents used to detect cancer, the diagnostic device including a relative movement adjusting module configured to relatively move the patch and a region in which the sample is provided to each other to provide the reagents contained in the patch to the sample, and an image acquiring module configured to acquire an image of the sample for cancer diagnosis.

### [Advantageous Effects]

According to the present disclosure, containing, delivery, and absorption of a substance can be easily performed.

According to the present disclosure, a reaction region for a substance can be provided, or a predetermined environment can be provided to a target region.

According to the present disclosure, delivery and absorption of a substance can be properly adjusted using a patch, and an amount of reagents used in diagnosis can be significantly reduced.

According to the present disclosure, a more accurate diagnosis result can be acquired by performing multiple diagnostic methods.

Aspects of the present disclosure are not limited to those mentioned above, and unmentioned aspects will be clearly understood by those of ordinary skill in the art to which the present disclosure pertains from the present specification and the accompanying drawings.

### [Description of Drawings]

FIG. 1 illustrates an example of a patch in detail according to the present application.
FIG. 2 illustrates an example of a patch in detail according to the present application.
FIG. 3 illustrates providing a reaction space as an example of a function of a patch according to the present application.
FIG. 4 illustrates providing a reaction space as an example of a function of a patch according to the present application.
FIG. 5 illustrates providing a substance as an example of a function of a patch according to the present application.
FIG. 6 illustrates providing a substance as an example of a function of a patch according to the present application.
FIG. 7 illustrates providing a substance as an example of a function of a patch according to the present application.
FIG. 8 illustrates providing a substance as an example of a function of a patch according to the present application.
FIG. 9 illustrates providing a substance as an example of a function of a patch according to the present application.
FIG. 10 illustrates providing a substance as an example of a function of a patch according to the present application.
FIG. 11 illustrates providing a substance as an example of a function of a patch according to the present application.
FIG. 12 illustrates providing a substance as an example of a function of a patch according to the present application.
FIG. 13 illustrates providing a substance as an example of a function of a patch according to the present application.
FIG. 14 illustrates absorbing of a substance as an example of a function of a patch according to the present application.
FIG. 15 illustrates absorbing of a substance as an example of a function of a patch according to the present application.
FIG. 16 illustrates absorbing of a substance as an example of a function of a patch according to the present application.
FIG. 17 illustrates absorbing of a substance as an example of a function of a patch according to the present application.
FIG. 18 illustrates absorbing of a substance as an example of a function of a patch according to the present application.
FIG. 19 illustrates absorbing of a substance as an example of a function of a patch according to the present application.
FIG. 20 illustrates absorbing of a substance as an example of a function of a patch according to the present application.
FIG. 21 illustrates absorbing of a substance as an example of a function of a patch according to the present application.
FIG. 22 illustrates absorbing of a substance as an example of a function of a patch according to the present application.
FIG. 23 illustrates providing an environment as an example of a function of a patch according to the present application.
FIG. 24 illustrates providing an environment as an example of a function of a patch according to the present application.
FIG. 25 illustrates providing an environment as an example of a function of a patch according to the present application.
FIG. 26 illustrates performance of absorbing and providing a substance as an embodiment of a patch according to the present application.
FIG. 27 illustrates performance of absorbing and providing a substance as an embodiment of a patch according to the present application.
FIG. 28 illustrates performance of absorbing and providing a substance as an embodiment of a patch according to the present application.
FIG. 29 illustrates performance of absorbing and providing a substance as an embodiment of a patch according to the present application.
FIG. 30 illustrates performance of absorbing and providing a substance as an embodiment of a patch according to the present application.
FIG. 31 illustrates performance of absorbing and providing a substance and providing an environment as an embodiment of a patch according to the present application.
FIG. 32 illustrates performance of absorbing and providing a substance and providing an environment as an embodiment of a patch according to the present application.
FIG. 33 illustrates an implementation of a plurality of patches as an embodiment of a patch according to the present application.
FIG. 34 illustrates an implementation of a plurality of patches and a plate having a plurality of target regions as an embodiment of a patch according to the present application.
FIG. 35 is a view for describing performance of morphological diagnosis using a staining technique in cancer diagnosis according to an embodiment of the present application.
FIG. 36 is a view for describing performance of morphological diagnosis using a staining technique in which a plurality of stains are used in cancer diagnosis according to an embodiment of the present application.
FIG. 37 is a view for describing performance of morphological diagnosis using a 4,6-diamidino-2-phenylindole (DAPI) staining technique in cancer diagnosis according to an embodiment of the present application.
FIG. 38 is a view for describing performance of immunoassay in cancer diagnosis according to an embodiment of the present application.
FIG. 39 is a view for describing a method of detecting cancer using a substrate-enzyme reaction when immunoassay is performed in cancer diagnosis according to an embodiment of the present application.
FIG. 40 is a view for describing a method of detecting cancer using a substrate-enzyme reaction when immunoassay is performed in cancer diagnosis according to an embodiment of the present application.
FIG. 41 is a view for describing a method of detecting cancer using a fluorescent substance when immunoassay is performed in cancer diagnosis according to an embodiment of the present application.
FIG. 42 is a view for describing a method of performing immunoassay using multiple types of antibodies in cancer diagnosis according to an embodiment of the present application.
FIG. 43 is a view for describing a method of performing immunoassay using a primary antibody and a secondary antibody in cancer diagnosis according to an embodiment of the present application.
FIG. 44 is a view for describing a method of performing immunoassay using a first antibody that has been applied on a plate and a second antibody that is provided to a sample in cancer diagnosis according to an embodiment of the present application.
FIG. 45 is a view for describing performance of cell culturing in cancer diagnosis according to an embodiment of the present application.
FIG. 46 is a view for describing a process in which cells, which are included in a sample, proliferate in cancer diagnosis according to an embodiment of the present application.
FIG. 47 is a view for describing a process in which cells included in a sample proliferate in cancer diagnosis according to an embodiment of the present application.
FIG. 48 is a view for describing performance of a polymerase chain reaction (PCR) process in cancer diagnosis according to an embodiment of the present application.
FIG. 49 is a view for describing a method of using a patch to decompose a cell membrane of cells included in a sample when a PCR process is performed in cancer diagnosis according to an embodiment of the present application.
FIG. 50 is a view for describing performance of a PCR process in cancer diagnosis according to an embodiment of the present application.
FIG. 51 is a view for describing multiple types of diagnostic methods for a sample in cancer diagnosis according to an embodiment of the present application.
FIG. 52 is a view for describing multiple types of diagnostic methods for a sample in cancer diagnosis according to an embodiment of the present application.
FIG. 53 is a view for describing multiple types of diagnostic methods for a sample in cancer diagnosis according to an embodiment of the present application.
FIG. 54 is a view for describing multiple types of diagnostic methods for a sample in cancer diagnosis according to an embodiment of the present application.
FIG. 55 is a view for describing multiple types of diagnostic methods for a sample in cancer diagnosis according to an embodiment of the present application.
FIG. 56 is a view for describing multiple types of diagnostic methods for a sample in cancer diagnosis according to an embodiment of the present application.
FIG. 57 is a view for describing multiple types of diagnostic methods for a sample in cancer diagnosis according to an embodiment of the present application.
FIG. 58 is a view for describing multiple types of diagnostic methods for a sample in cancer diagnosis according to an embodiment of the present application.
FIG. 59 is a block diagram of a diagnostic device according to an embodiment of the present application.
FIG. 60 is a conceptual diagram illustrating an example in which a structure of a diagnostic device is moved due to a relative movement operation of a relative position adjusting module according to an embodiment of the present application.

### [Modes of the Invention]

Since embodiments described herein are for clearly describing the spirit of the present disclosure to those of ordinary skill in the art to which the present disclosure pertains, the present disclosure is not limited to the embodiments described herein, and the scope of the present disclosure should be construed as including revised examples or modified examples not departing from the spirit of the present disclosure.

General terms currently being used as widely as possible have been selected as terms used herein in consideration of functions in the present disclosure, but the terms may be changed according to intentions and practices of those of ordinary skill in the art to which the present disclosure pertains or the advent of new technologies, etc. However, instead, when a particular term is defined as a certain meaning and used, the meaning of the term will be separately described. Consequently, the terms used herein should be construed on the basis of substantial meanings of the terms and content throughout the present specification instead of simply on the basis of names of the terms.

The accompanying drawings herein are for easily describing the present disclosure. Since shapes illustrated in the drawings may have been exaggeratedly depicted as much as necessary to assist in understating the present disclosure, the present disclosure is not limited by the drawings.

When detailed description of a known configuration or function related to the present disclosure is deemed to obscure the gist of the present disclosure in the present specification, the detailed description related thereto will be omitted as necessary.

According to an aspect of the present application, there is provided a diagnostic method for performing diagnosis on tumor cells included in a sample by using a patch which is provided as a gel type having a net-like structure forming micro-cavities, the diagnostic method including placing the sample on a plate and using the patch that contains reagents used to detect cancer to provide the reagents contained in the patch to the plate.

The placing of the sample on the plate may include providing the sample in a single layer on the plate.

The sample may be blood.

The placing of the sample on the plate may include fixating the sample on the plate.

The reagents contained in the patch may include an antibody that reacts specifically with the tumor cells.

The diagnostic method may include providing a substrate on the plate in order to identify an antibody bound to the tumor cells.

An antibody that reacts specifically with the tumor cells may be applied on the plate.

The reagents contained in the patch may include a staining reagent used in staining cells in order to stain the tumor cells.

The staining reagent may target at least one of a nucleus of the cells, a cytoplasm of the cells, and DNA distributed in the cells.

The diagnostic method may include adjusting a temperature of the sample in order to create a reaction environment for the staining reagent.

The reagents contained in the patch may include a nutrient reagent used in cell culturing in order to diagnose the cancer by culturing the tumor cells.

The providing the reagents contained in the patch may include bringing the patch and the plate into contact.

The diagnostic method may further include acquiring an image of the plate on which the sample is placed in order to perform diagnosis of the tumor cells.

The image of the plate on which the sample is placed may be a fluorescence image.

The reagents contained in the patch may include a reagent used in removal of a cell membrane of cells in order to extract DNA of the tumor cells.

According to another aspect of the present application, there is provided a diagnostic method for performing diagnosis on tumor cells included in a sample by using a patch which is provided as a gel type having a net-like structure forming micro-cavities, the diagnostic method including placing the sample on a plate, using a first patch that contains a first reagent used to detect cancer to provide the reagent contained in the first patch to the plate, and using a second patch, which contains a second reagent used to detect the cancer, to provide the reagent contained in the second patch to the plate.

The providing the reagent contained in the first patch may be performed prior to the providing the reagent contained in the second patch.

The first reagent may include an antibody that reacts specifically with the tumor cells, and the second reagent may include an antibody that binds to the antibody reacting specifically with the tumor cells.

The first reagent may include an antibody that reacts specifically with the tumor cells, and the second reagent may include a nutrient reagent that is used in cell culturing in order to diagnose the cancer by culturing the tumor cells
The first reagent may include an antibody that reacts specifically with the tumor cells, and the second reagent may include a staining reagent that is used for cell staining in order to stain the tumor cells.

The first reagent may include an antibody that reacts specifically with the tumor cells, and the second reagent may include an antibody that reacts specifically with white blood cells.

The diagnostic method may further include, between the providing the reagent contained in the first patch and the providing the reagent contained in the second patch, acquiring an image of the sample.

The first reagent may include a reagent used in removal of a cell membrane of cells in order to extract DNA of the tumor cells, and the second reagent may include a reagent used in a polymerase chain reaction (PCR).

The diagnostic method may further include adjusting a temperature of the sample in order to cause the PCR.

According to still another aspect of the present application, there is provided a diagnostic device for performing diagnosis on tumor cells included in a sample by using a patch which is provided as a gel type having a net-like structure forming micro-cavities and contains reagents used to detect cancer, the diagnostic device including a relative movement adjusting module configured to relatively move the patch and a region in which the sample is provided to each other to provide the reagents contained in the patch to the sample, and an image acquiring module configured to acquire an image of the sample for cancer diagnosis.

The diagnostic device may further include a temperature adjusting module configured to adjust the temperature of the sample.

### 1. Patch

### 1.1 Meaning of patch

In the present application, a patch for managing a liquid substance is disclosed.

The liquid substance may mean a substance which is in a liquid state and can flow.

The liquid substance may be a substance formed of a single component having fluidity. Alternatively, the liquid substance may be a mixture that includes a substance formed of a plurality of components.

When the liquid substance is a substance formed of a single component, the liquid substance may be a substance formed of a single chemical element or a compound including a plurality of chemical elements.

When the liquid substance is a mixture, a portion of the substance formed of a plurality of components may serve as a solvent, and the other portion may serve as a solute. That is, the mixture may be a solution.

A plurality of components constituting the mixture which forms the substance may be uniformly distributed. Alternatively, the mixture including the substance formed of a plurality of components may be a uniformly mixed mixture.

The substance formed of a plurality of components may include a solvent and a substance that is not dissolved in the solvent and is uniformly distributed.

A portion of the substance formed of a plurality of components may be non-uniformly distributed. The non-uniformly distributed substance may include non-uniformly distributed particle components in the solvent. In this case, the non-uniformly distributed particle components may be in a solid phase.

For example, a substance that may be managed using the patch may be in a state of 1) a liquid formed of a single component, 2) a solution, or 3) a colloid, or according to circumstances, may be in a state in which 4) solid particles are non-uniformly distributed within another liquid substance.

Hereinafter, the patch according to the present application will be described in more detail.

### 1.2 General nature of patch

### 1.2.1 Configuration

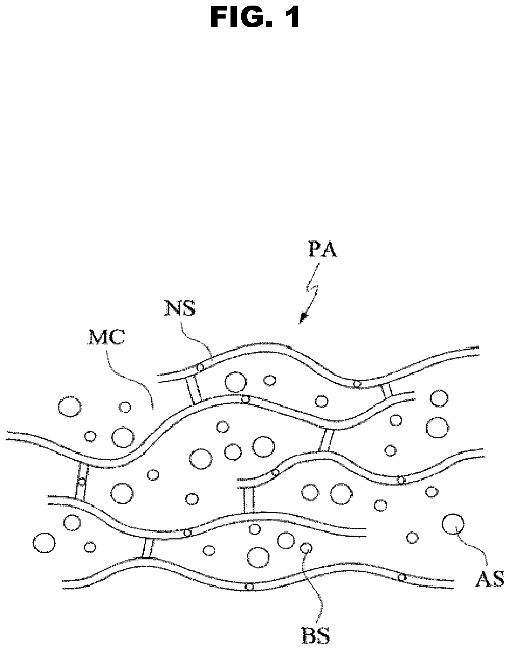

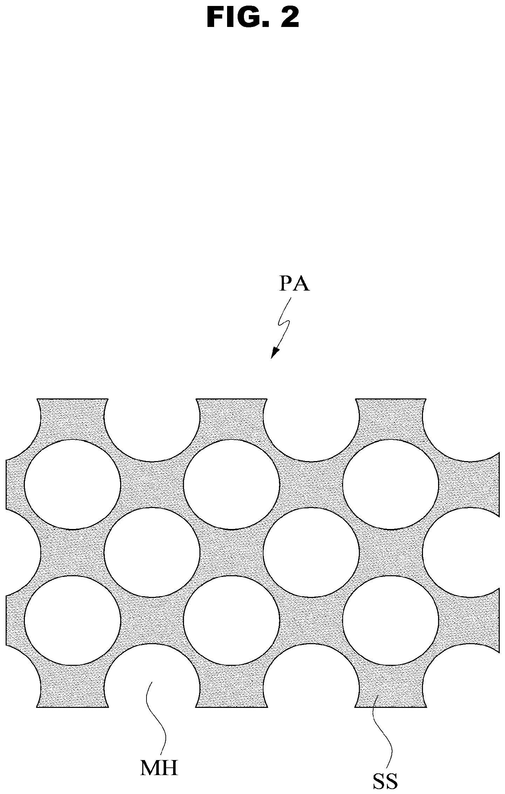

FIGS. 1 and 2 are views illustrating an example of a patch according to the present application. The patch according to the present application will be described below with reference to FIGS. 1 and 2.

Referring to FIG. 1, a patch PA according to the present application may include a mesh structural body NS and a liquid substance.

As the liquid substance, a base substance BS and an additive substance AS may be taken into consideration separately.

The patch PA may be in a gel state(gel type). The patch PA may be implemented as a gel-type structural body in which colloidal molecules are bound and mesh tissues are formed.

The patch PA according to the present application is a structure for managing a liquid substance SB, and may include a three-dimensional mesh(net-like) structural body NS. The mesh structural body NS may be a continuously distributed solid structure. The mesh structural body NS may have a mesh structure in which a plurality of micro-threads are intertwined. However, the mesh structural body NS is not limited to the mesh form in which the plurality of micro-threads are intertwined, and may also be implemented in the form of an arbitrary three-dimensional matrix that is formed by connection of a plurality of micro-structures. For example, the mesh structural body NS may be a frame structural body that includes a plurality of micro-cavities. In other words, the mesh structural body NS may form a plurality of micro-cavities MC.

FIG. 2 illustrates a structure of a patch according to an embodiment of the present application. Referring to FIG. 2, the mesh structural body of the patch PA may have a sponge structure SS. The mesh structural body of the sponge structure SS may include a plurality of micro-holes MH. Hereinafter, the terms micro-holes MH and the micro-cavities MC may be used interchangeably, and unless particularly mentioned otherwise, the term micro-cavities MC is defined as encompassing the concept of the micro-holes MH.

The mesh structural body NS may have a regular or irregular pattern. Furthermore, the mesh structural body NS may include both a region having a regular pattern and a region having an irregular pattern.

A density of the mesh structural body NS may have a value within a predetermined range. Preferably, the predetermined range may be set within a limit in which the form of the liquid substance SB captured in the patch PA is maintained in a form that corresponds to the patch PA. The density may be defined as a degree to which the mesh structural body NS is dense or a mass ratio, a volume ratio, or the like that the mesh structural body NS occupies in the patch.

The patch according to the present application may manage the liquid substance SB by having a three-dimensional mesh structure.

The patch PA according to the present application may include the liquid substance SB, and the fluidity of the liquid substance SB included in the patch PA may be limited by the form of the mesh structural body NS of the patch PA.

The liquid substance SB may freely flow within the mesh structural body NS. In other words, the liquid substance SB is placed in the plurality of micro-cavities formed by the mesh structural body NS. An exchange of liquid substance SB may occur between neighboring micro-cavities. In this case, the liquid substance SB may be present in a state in which the liquid substance SB permeating into a frame structural body that forms the mesh tissues. In such a case, nano-sized pores into which the liquid substances SB may permeate may be formed in the frame structural body.

Further, whether to the liquid substance SB is filled in the frame structural body of the mesh structure may be determined depending on a molecular weight or a particle size of the liquid substance SB to be captured in the patch PA. A substance having a relatively large molecular weight may be captured in the micro-cavities, and a substance having a relatively small molecular weight may be captured by the frame structural body and filled in the micro-cavities and/or the frame structural body of the mesh structural body NS.

In the present specification, the term "capture" may be defined as a state in which the liquid substance SB is placed in the plurality of micro-cavities and/or nano-sized holes formed by the mesh structural body NS. As described above, the state in which the liquid substance SB is captured in the patch PA is defined as including a state in which the liquid substance SB may flow between the micro-cavities and/or the nano-sized holes.

As in the following, the base substance BS and the additive substance AS may be taken into consideration separately as the liquid substance SB.

The base substance BS may be a liquid substance SB having fluidity.

The additive substance AS may be a substance that is mixed with the base substance BS and has fluidity. In other words, the base substance BS may be a solvent. The additive substance AS may be a solute that is dissolved in the solvent or may be particles that are not melted in the solvent.

The base substance BS may be a substance capable of flowing inside a matrix formed by the mesh structural body NS. The base substance BS may be uniformly distributed in the mesh structural body NS or may be distributed only in a partial region of the mesh structural body NS. The base substance BS may be a liquid having a single component.

The additive substance AS may be a substance that is mixed with the base substance BS or dissolved in the base substance BS. For example, the additive substance AS may serve as a solute while the base substance BS is a solvent. The additive substance AS may be uniformly distributed in the base substance BS.

The additive substance AS may be fine particles that are not dissolved in the base substance BS. For example, the additive substance AS may include colloidal molecules and fine particles such as microorganisms.

The additive substance AS may include particles larger than the micro-cavities formed by the mesh structural body NS. When the size of the micro-cavities is smaller than the size of the particles included in the additive substance AS, fluidity of the additive substance AS may be limited.

According to an embodiment, the additive substance AS may include a component that is selectively included in the patch PA.

The additive substance AS does not necessarily refer to a substance that is lower in quantity or inferior in function in comparison to the above-described base substance BS.

Hereinafter, characteristics of the liquid substance SB captured in the patch PA may be presumed as characteristics of the patch PA. That is, the characteristics of the patch PA may depend on characteristics of a substance captured in the patch PA.

### 1.2.2 Characteristics

As described above, the patch PA according to the present application may include the mesh structural body NS. The patch PA may manage the liquid substance SB through the mesh structural body NS. The patch PA may allow the liquid substance SB captured in the patch PA to maintain at least some of its unique characteristics.

For example, diffusion of a substance may occur in a region of the patch PA in which the liquid substance SB is distributed, and a force such as surface tension may come into action.

The patch PA may provide a liquid environment in which diffusion of a target substance is caused due to thermal motion of a substance or a difference in density or concentration thereof. Generally, "diffusion" refers to a phenomenon in which particles that constitute a substance are spread from a side at which concentration is high to a side at which a concentration is low due to a difference in concentration. Such a diffusion phenomenon may be basically understood as a phenomenon that occurs due to motion of molecules (translational motion in a gas or liquid, vibrational motion in a solid, and the like). In the present application, in addition to referring to the phenomenon in which particles are spread from a side at which a concentration is high toward a side at which a concentration is low due to a difference in concentration or density, "diffusion" also refers to a phenomenon in which particles move due to irregular motion of molecules that occurs even when a concentration is uniform. The expression "irregular motion" may also have the same meaning as "diffusion" unless particularly mentioned otherwise. The diffused substance may be a solute that is dissolved in the liquid substance SB, and the diffused substance may be provided in a solid, liquid, or gas state.

More specifically, a non-uniformly-distributed substance in the liquid substance SB captured by the patch PA may be diffused in a space provided by the patch PA. In other words, the additive substance AS may be diffused in a space defined by the patch PA.

The non-uniformly-distributed substance or the additive substance AS in the liquid substance SB managed by the patch PA may be diffused within the micro-cavities provided by the mesh structural body NS of the patch PA. A region in which the non-uniformly-distributed substance or the additive substance AS may be diffused may be changed by the patch PA being connected or coming into contact with another substance.

Even when, after the concentration of the substance or the additive substance AS has become uniform, as a result of diffusion of the non-uniformly-distributed substance or the additive substance AS within the patch PA or within an external region connected to the patch PA, the substance or the additive substance AS may continuously move due to irregular motion of molecules inside the patch PA and/or within the external region connected to the patch PA.

The patch PA may be implemented to exhibit a hydrophilic or hydrophobic property. In other words, the mesh structural body NS of the patch PA may have a hydrophilic or hydrophobic property.

When properties of the mesh structural body NS and the liquid substance SB are similar, the mesh structural body NS may be able to manage the liquid substance SB more effectively.

The base substance BS may be a polar hydrophilic substance or a nonpolar hydrophobic substance. The additive substance AS may exhibit a hydrophilic or hydrophobic property.

The properties of the liquid substance SB may be related to the base substance BS and/or the additive substance AS. For example, when both the base substance BS and the additive substance AS are hydrophilic, the liquid substance SB may be hydrophilic, and when both the base substance BS and the additive substance AS are hydrophobic, the liquid substance SB may be hydrophobic. When polarities of the base substance BS and the additive substance AS are different, the liquid substance SB may be hydrophilic or hydrophobic.

When polarities of both the mesh structural body NS and the liquid substance SB are hydrophilic or hydrophobic, an attractive force may come into action between the mesh structural body NS and the liquid substance SB. When polarities of the mesh structural body NS and the liquid substance SB are opposite, e.g., when the polarity of the mesh structural body NS is hydrophobic and the polarity of the liquid substance SB is hydrophilic, a repulsive force may act between the mesh structural body NS and the liquid substance SB.

On the basis of the above-described properties, the patch PA may be solely used, a plurality of patches PA may be used, or the patch PA may be used with another medium to induce a desired reaction. Hereinafter, functional aspects of the patch PA will be described.

However, hereinafter, for convenience of description, the patch PA is assumed as being a gel type that may include a hydrophilic solution. In other words, unless particularly mentioned otherwise, the mesh structural body NS of the patch PA is assumed to have a hydrophilic property.

However, the scope of the present application should not be interpreted as being limited to the gel-type patch PA having a hydrophilic property. In addition to a gel-type patch PA that includes a solution exhibiting a hydrophobic property, a gel-type patch PA from which a solvent is removed and even a sol-type patch PA, as long as it is capable of implementing functions according to the present application, may belong to the scope of the present application.

### 2. Functions of patch

Due to the above-described characteristics, the patch according to the present application may have some useful functions. In other words, by capturing the liquid substance SB, the patch may become involved in behavior of the liquid substance SB.

Accordingly, hereinafter, in accordance with forms of behavior of the substance with respect to the patch PA, a reservoir function in which a state of the substance is defined in a predetermined region formed by the patch PA and a channeling function in which a state of the substance is defined in a region including an external region of the patch PA will be separately described.

### 2.1 Reservoir

### 2.1.1 Meaning

As described above, the patch PA according to the present application may capture the liquid substance SB. In other words, the patch PA may perform a function as a reservoir.

The patch PA may capture the liquid substance SB in the plurality of micro-cavities formed in the mesh structural body NS using the mesh structural body NS. The liquid substance SB may occupy at least a portion of the fine micro-cavities formed by the three-dimensional mesh structural body NS of the patch PA or be penetrated in the nano-sized pores formed in the mesh structural body NS.

The liquid substance SB placed in the patch PA does not lose properties of a liquid even when the liquid substance SB is distributed in the plurality of micro-cavities. That is, the liquid substance SB has fluidity even in the patch PA, and diffusion of a substance may occur in the liquid substance SB distributed in the patch PA, and an appropriate solute may be dissolved in the substance.

The reservoir function of the patch PA will be described below in more detail.

### 2.1.2 Containing

In the present application, the patch PA may capture a target substance due to the above-described characteristics. The patch PA may have resistance to a change in an external environment within a predetermined range. In this way, the patch PA may maintain a state in which the substance is captured therein. The liquid substance SB, which is a target to be captured, may occupy the three-dimensional mesh structural body NS.

Hereinafter, for convenience, the above-described function of the patch PA will be referred to as "containing."

However, "the patch PA containing the liquid substance" is defined to encompass a case in which the liquid substance is contained in a space formed by the mesh structure and/or a case in which the liquid substance is contained in the frame structural body constituting the mesh structural body NS.

The patch PA may contain the liquid substance SB. For example, the patch PA may contain the liquid substance SB, due to an attractive force that acts between the mesh structural body NS of the patch PA and the liquid substance SB. The liquid substance SB may be bound to the mesh structural body NS with an attractive force of a predetermined strength or higher and contained in the patch PA.

Properties of the liquid substance SB contained in the patch PA may be classified in accordance with properties of the patch PA. More specifically, when the patch PA exhibits a hydrophilic property, the patch PA may be bound to a hydrophilic liquid substance SB which is polar in general and contain the hydrophilic liquid substance SB in the three-dimensional micro-cavities. Alternatively, when the patch PA exhibits a hydrophobic property, the hydrophobic liquid substance SB may be contained in the micro-cavities of the three-dimensional mesh structural body NS.

The amount of substance that may be contained in the patch PA may be proportional to a volume of the patch PA. In other words, the amount of substance contained in the patch PA may be proportional to an amount of three-dimensional mesh structural body NS that serves as a support body that contributes to the form of the patch PA. However, there is no constant proportional factor between the amount of substance that may be contained in the patch PA and the volume of the patch PA, and thus the relationship between the amount of substance that may be contained in the patch PA and the volume of the patch PA may be changed in accordance with the design or manufacturing method of the mesh structure.

The amount of substance contained in the patch PA may be reduced due to evaporation, loss, etc. with time. The substance may be additionally injected into the patch PA to increase or maintain the content of the substance contained in the patch PA. For example, a moisture keeping agent for suppressing evaporation of moisture may be added to the patch PA.

The patch PA may be implemented in a form in which it is easy to store the liquid substance SB. This signifies that, when the substance is affected by environmental factors such as humidity level, amount of light, and temperature, the patch PA may be implemented to minimize denaturalization of the substance. For example, to prevent the patch PA from being denaturalized due to external factors such as bacteria, the patch PA may be treated with a bacteria inhibitor.

A liquid substance SB having a plurality of components may be contained in the patch PA. In this case, the substance formed of a plurality of components may be placed together in the patch PA before a reference time point, or a primarily-injected substance may be first contained in the patch PA and then a secondary substance may be contained in the patch PA after a predetermined amount of time. For example, when a liquid substance SB formed of two components is contained in the patch PA, the two components may be contained in the patch PA upon manufacturing the patch PA, only one component may be contained in the patch PA upon manufacturing the patch PA and the other component may be contained therein later, or the two components may be sequentially contained in the patch PA after the patch PA is manufactured.

As described above, the substance contained in the patch may exhibit fluidity, and the substance may move irregularly or be diffused due to molecular motion in the patch PA.

### 2.1.3 Providing of reaction space

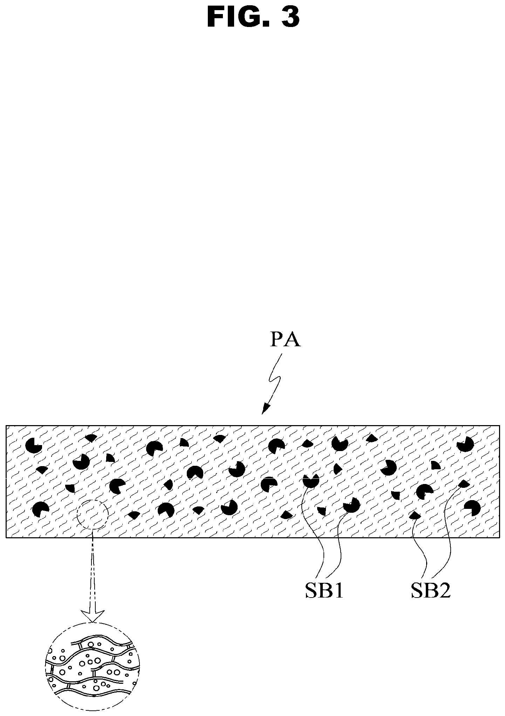

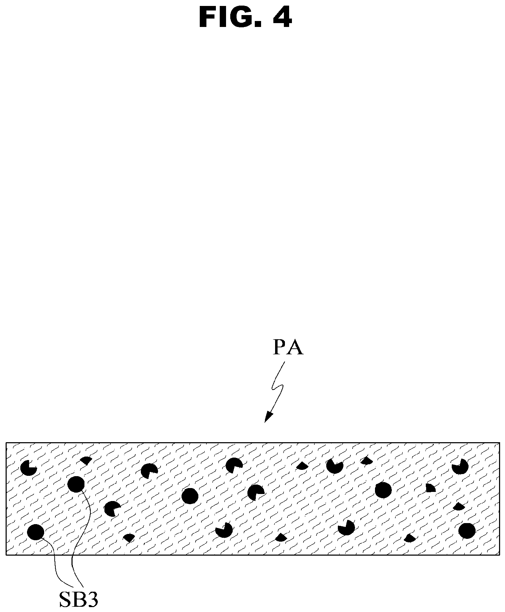

FIGS. 3 and 4 are views illustrating providing a reaction space as an example of a function of the patch according to the present application.

As illustrated in FIGS. 3 and 4, the patch PA according to the present application may perform a function of providing a space. In other words, the patch PA may provide a space in which the liquid substance SB may move through a space formed by the mesh structural body NS and/or a space constituting the mesh structural body NS.

The patch PA may provide a space for activity other than diffusion of particles and/or irregular motion of particles (hereinafter referred to as activity other than diffusion). The activity other than diffusion may refer to a chemical reaction, but is not limited thereto, and may also refer to a physical state change. More specifically, the activity other than diffusion may include a chemical reaction in which a chemical composition of the substance changes after the activity, a specific binding reaction between components included in the substance, homogenization of solutes or particles included in the substance and non-uniformly distributed therein, condensation of some components included in the substance, or a biological activity of a portion of the substance.

When a plurality of substances become involved in the activity, the plurality of substances may be placed together in the patch PA before a reference time point. The plurality of substances may be sequentially inserted into the patch PA.

By changing environmental conditions of the patch PA, efficiency of the function of providing a space for activities other than diffusion in the patch PA may be enhanced. For example, the activity may be promoted or a start of the activity may be induced by changing a temperature condition of the patch PA or adding an electrical condition thereto.

According to FIGS. 3 and 4, a first substance SB1 and a second substance SB2 placed in the patch PA may react inside the patch PA and be deformed into a third substance SB3 or generate the third substance SB3.

### 2.2 Channel

### 2.2.1 Meaning

Movement of a substance may occur between the patch PA and an external region. The substance may be moved from the patch PA to the external region of the patch PA or may be moved from the external region to the patch PA.

The patch PA may form a substance movement path or get involved in movement of the substance. More specifically, the patch PA may become involved in movement of the liquid substance SB captured in the patch PA or become involved in movement of an external substance through the liquid substance SB captured in the patch PA. The base substance BS or the additive substance AS may move out from the patch PA, or an external substance may be introduced from an external region to the patch PA.

The patch PA may provide a substance movement path. That is, the patch PA may become involved in movement of the substance and provide a substance movement channel. The patch PA may provide a substance movement channel based on unique properties of the liquid substance SB.

In accordance with whether the patch PA is connected to the external region, the patch PA may be in a state in which the liquid substance SB is movable between the patch PA and the external region or a state in which the liquid substance SB is immovable between the patch PA and the external region. When channeling between the patch PA and the external region begins, the patch PA may have unique functions.

Hereinafter, the state in which the substance is movable and the state in which the substance is immovable will be described first, and the unique functions of the patch PA will be described in detail in connection with whether the patch PA and the external region are connected.

Basically, irregular motion and/or diffusion of the substance are fundamental causes of movement of the liquid substance SB between the patch PA and the external region. However, controlling an external environmental factor (e.g., controlling a temperature condition, controlling an electrical condition, or the like) in order to control movement of a substance between the patch PA and the external region has already been described.

### 2.2.2 Movable state

In the state in which the substance is movable, a flow may occur between the liquid substance SB captured in the patch PA and/or the substance placed in the external region. In the state in which the substance is movable, substance movement may occur between the liquid substance SB captured in the patch PA and the external region.

For example, in the state in which the substance is movable, the liquid substance SB or some components of the liquid substance SB may be diffused to the external region or moved due to irregular motion. Alternatively, in the state in which the substance is movable, an external substance placed in the external region or some components of the external substance may be diffused to the liquid substance SB in the patch PA or moved due to irregular motion.

The state in which the substance is movable may be caused by contact. The contact may refer to connection between the liquid substance SB captured in the patch PA and the external region. Contact may refer to at least a partial overlap between a flow region of the liquid substance SB and the external region. The contact may refer to the external substance being connected to at least a portion of the patch PA. It may be understood that the range in which the captured liquid substance SB may flow is expanded in the state in which the substance is movable. In other words, in the state in which the substance is movable, the range in which the liquid substance SB may flow may be expanded to include at least a portion of the external region of the captured liquid substance SB. For example, when the liquid substance SB is in contact with the external region, the range in which the captured liquid substance SB may flow may be expanded to include at least a portion of the external region in contact. More specifically, when the external region is an external plate, the region in which the liquid substance SB may flow may be expanded to include a region of the external plate in contact with the liquid substance SB.

### 2.2.3 Immovable state

In the state in which the substance is immovable, substance movement may not occur between the liquid substance SB captured in the patch PA and the external region. However, substance movement may respectively occur in the liquid substance SB captured in the patch PA and in external substance placed in the external region.

The state in which the substance is immovable may be a state in which the contact is released. In other words, in the state in which contact between the patch PA and the external region is released, substance movement is not possible between the liquid substance SB remaining in the patch PA and the external region or the external substance.

More specifically, the state in which the contact is released may refer to a state in which the liquid substance SB captured in the patch PA is not connected to the external region. The state in which the contact is released may refer to a state in which the liquid substance SB is not connected to an external substance placed in the external region. For example, the state in which movement of the substance is impossible may be caused by separation between the patch PA and the external region.

In the present specification, although "movable state" has a meaning differentiated from that of "immovable state," a transition may occur between the states due to an elapse of time, an environmental change, and the like. In other words, the patch PA may be in the immovable state after being in the movable state , in the movable state after being in the immovable state, or may be in the movable state again, after being in the immovable state after being in the movable state. 2.2.4 Differentiation of functions

### 2.2.4.1 Delivery

In the present application, due to the above-described characteristics, the patch PA may deliver at least a portion of the liquid substance SB captured in the patch PA to a desired external region. The delivery of the substance may refer to separation of a portion of the liquid substance SB captured in the patch PA from the patch PA due to a predetermined condition being satisfied. The separation of the portion of the liquid substance SB may refer to the portion of the substance being extracted, emitted, or released from a region that is affected by the patch PA. This is a concept subordinate to the above-described channeling function of the patch PA, and may be understood as defining transfer(delivery) of the substance placed in the patch PA to the outside of the patch PA.

The desired external region may be another patch PA, a dried region, or a liquid region.

The predetermined condition for the delivery to occur may be set as an environmental condition such as a temperature change, a pressure change, a change in an electrical characteristic, and a change in a physical state. For example, when the patch PA is in contact with an object whose force of binding to the liquid substance SB is larger than a force of binding to the mesh structural body NS of the patch PA, the liquid substance SB may be chemically bound with the object in contact, and as a result, at least a portion of the liquid substance SB may be provided to the object.

Hereinafter, for convenience, the above-described function of the patch PA will be referred to as "delivery."

The delivery may occur between the patch PA and the external region, via the state in which the liquid substance SB is movable and the state in which the liquid substance SB is immovable between the patch PA and the external region.

More specifically, when the liquid substance SB is in the movable state, the liquid substance SB may be diffused between the patch PA and the external region or may be moved to the external region due to irregular motion. In other words, the base solution and/or the additive substance AS included in the liquid substance SB may be moved from the patch PA to the external region. In the state in which the liquid substance SB is immovable, the liquid substance SB is unable to move between the patch PA and the external region. In other words, due to a transition from the movable state to the immovable state, a portion of the substance that has moved from the patch PA to the external region due to diffusion and/or irregular motion of the liquid substance SB become unable to move back to the patch PA. Thus, a portion of the liquid substance SB may be provided to the external region.

The delivery may be performed due to a difference between an attractive force between the liquid substance SB and the mesh structural body NS and an attractive force between the liquid substance SB and the external region or the external substance. The attractive force may be caused by similarity between polarities or a specific binding relationship.

More specifically, when the liquid substance SB is hydrophilic and the external region or the external substance is more hydrophilic than the mesh structural body NS, at least a portion of the liquid substance SB captured in the patch PA may be provided to the external region via the movable state and the immovable state.

The delivery of the liquid substance SB may also be performed selectively. For example, when a specific binding relationship exists between some components included in the liquid substance SB and the external substance, some of the ingredients may be selectively delivered via the state in which the substance is movable and the state in which the substance is immovable.

More specifically, when it is assumed that the patch PA provides a substance to an external plate PL, which is in a form of a flat plate, a substance that binds specifically to a portion of the liquid substance SB captured in the patch PA (e.g., a portion of a solute) may be applied on the external plate PL. In this case, the patch PA may selectively deliver a portion of the solute that binds specifically to the substance applied on the external plate PL from the patch PA to the plate PL via the movable state and the immovable state.

The delivery as a function of the patch PA will be described below according to a few examples of different regions to which the substance is moved. However, in giving the detailed description, the concepts of "release" of the liquid substance SB and "delivery" of the liquid substance SB may be interchangeably used.

Here, a case in which the liquid substance SB is provided from the patch PA to a separate external plate PL will be described. For example, a case in which the substance is moved from the patch PA to a plate PL, such as a slide glass, may be taken into consideration.

As the patch PA and the plate PL come into contact, at least a portion of the liquid substance SB captured in the patch PA is diffused to the plate PL or moved due to irregular motion. When the contact between the patch PA and the plate PL is released, the portion of the substance that has been moved from the patch PA to the plate PL (that is, the portion of the liquid substance SB) may become unable to move back to the patch PA. As a result, the portion of the substance may be provided from the patch PA to the plate PL. In this case, the portion of the substance being provided may be the additive substance AS. For a substance in the patch PA to be "provided" by the contact and separation, an attractive force and/or binding force that acts between the substance and the plate PL should be present, and the attractive force and/or the binding force should be larger than the attractive force acting between the substance and the patch PA. Therefore, if the above-described "delivery condition" is not satisfied, delivery of a substance may not occur between the patch PA and the plate PL.

The delivery of a substance may be controlled by providing a temperature condition or an electrical condition to the patch PA.

The movement of a substance from the patch PA to the plate PL may depend on an extent of a contact area between the patch PA and the plate PL. For example, the substance movement efficiency between the patch PA and the plate PL may be increased or decreased in accordance with an extent of an area in which the patch PA and the plate PL come into contact.

When the patch PA includes a plurality of components, only some of the components may be selectively moved to the external plate PL. More specifically, a substance that binds specifically to some of the plurality of components may be fixed to the external plate PL. In this case, the substance fixed to the external plate PL may be in a liquid or solid state, or may be fixed to a different region. In this case, a portion of the substance of the plurality of components moves to the plate PL and binds specifically to the plate PL due to contact between the patch PA and the different region, and when the patch PA is separated from the plate PL, only some of the components may be selectively released to the plate PL.

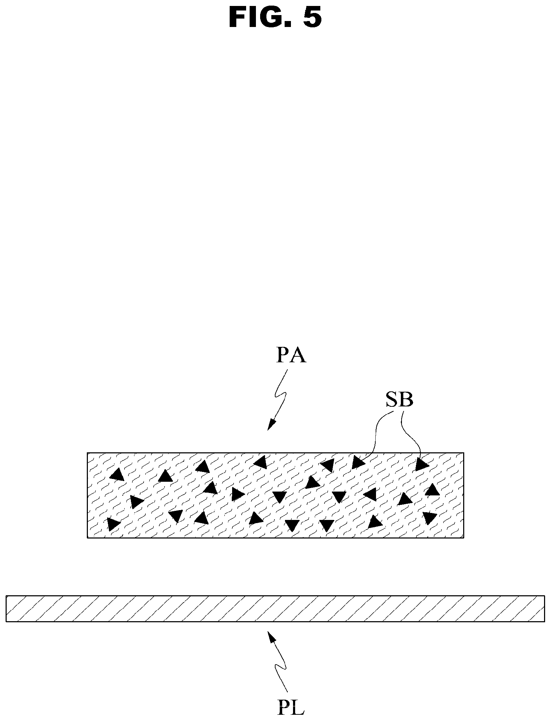

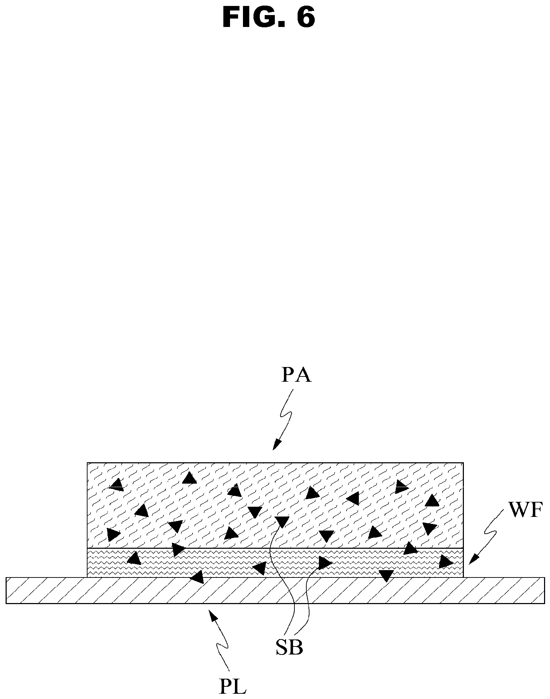

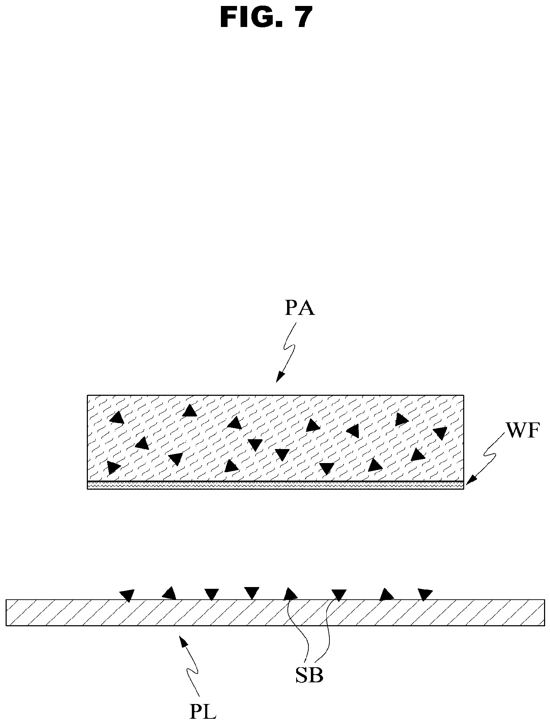

FIGS. 5 to 7 illustrate delivery of a substance from the patch PA to the external plate PL as an example of delivery of a substance from among the functions of the patch PA according to the present application. According to FIGS. 5 to 7, by the patch PA coming into contact with the external plate PL, a portion of a substance contained in the patch PA may be provided to the plate PL. In this case, providing the substance may become possible by the patch PA coming into contact with the plate so that the substance is movable. In this case, a water film WF may be formed in the vicinity of a contact surface at which the plate and the patch PA come into contact, and the substance may be movable through the formed water film WF.

Here, a case in which the liquid substance SB is provided from the patch PA to a substance having fluidity SL will be described. The substance having fluidity SL may be a liquid substance that is held in other containing space or that is flowing.

As the patch PA and the substance having fluidity come into contact (for example, the patch PA is put into a solution), at least a portion of the liquid substance SB captured in the patch PA may be diffused or moved due to irregular motion to the substance having fluidity SL. When the patch PA and the substance having fluidity SL are separated, a portion of the liquid substance SB that has been moved from the patch PA to the substance having fluidity become unable to move back to the patch PA so that a portion of the substance in the patch PA may be provided to the substance having fluidity.

The substance movement between the patch PA and the substance having fluidity SL may depend on an extent of a contact area between the patch PA and the substance having fluidity SL. For example, the substance movement efficiency between the patch PA and the substance having fluidity SL may be increased or decreased in accordance with an extent of an area at which the patch PA and the substance having fluidity SL come into contact (for example, a depth at which the patch PA is immersed into a solution or the like).

The substance movement between the patch PA and the substance having fluidity SL may be controlled through physical separation between the patch PA and the substance having fluidity.

A partial concentration of the additive substance AS in the liquid substance SB and a partial concentration of the additive substance AS in the substance having fluidity may be different, and the additive substance AS may be provided from the patch PA to the substance having fluidity.

However, in the patch PA providing the liquid substance SB to the substance having fluidity SL, the physical separation between the patch PA and the substance having fluidity SL is not essential. For example, when a force (driving force/ casual force) that causes a substance to move from the patch PA to a liquid having fluidity disappears or is decreased to a reference value or lower, the movement of the substance may be stopped.

In "delivery" between the patch PA and the substance having fluidity SL, the above-described "delivery condition" between the patch PA and the substance having fluidity SL may not be required. It may be understood that substances that have already moved to the substance having fluidity SL are diffused and/or moved due to irregular motion in the substance having fluidity SL, and the substance has been provided to the substance having fluidity SL when a distance between the moved substance and the patch PA become larger a predetermined distance. Since, while in the case of the plate PL, a movable range expanded due to the contact is extremely limited, and thus the attractive force between the patch PA and the substances that have moved to the plate PL may be significant, in the relationship between the patch PA and the substance having fluidity, a movable range expanded due to contact between the patch PA and the plate PL is relatively much wider, and thus the attractive force between the patch PA and the substances that have moved to the substance having fluidity SL is insignificant.

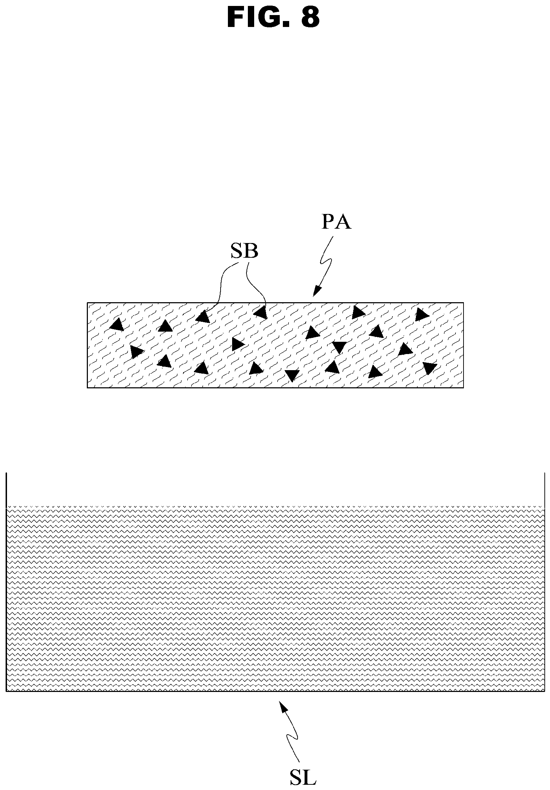

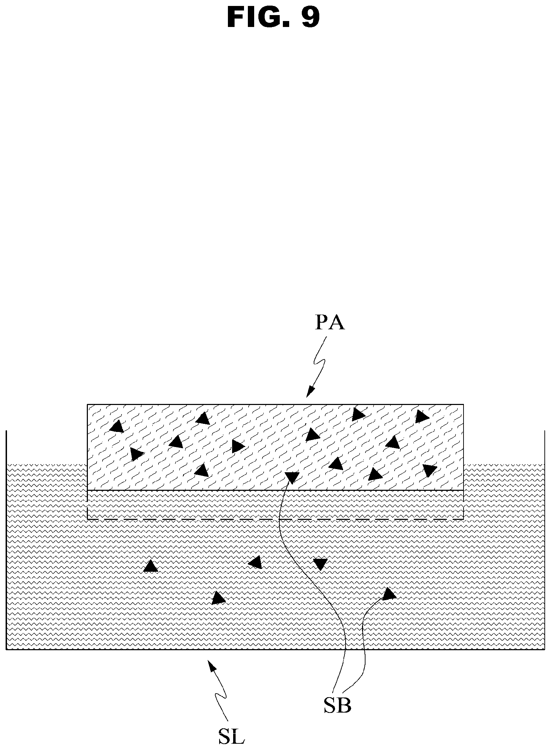

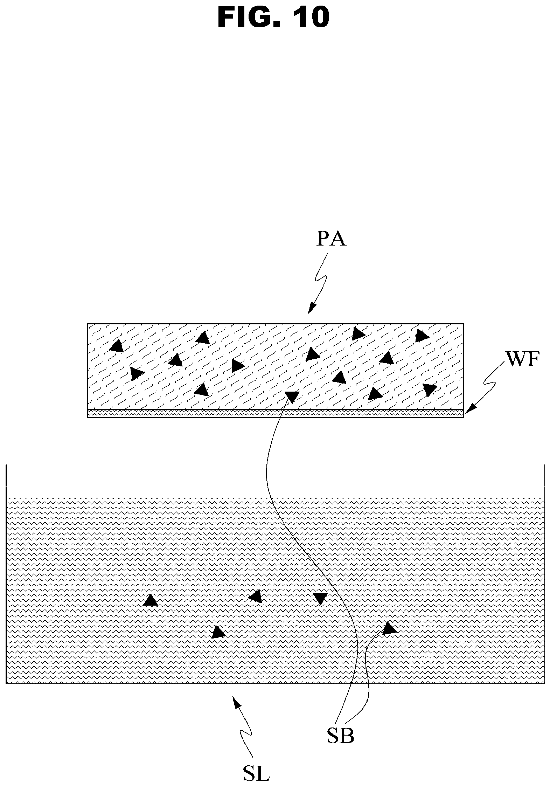

FIGS. 8 to 10 illustrate delivery of a substance from the patch PA to the substance having fluidity as an example of delivery of a substance from among the functions of the patch PA according to the present application. According to FIGS. 8 to 10, the patch PA may deliver a portion of a substance contained in the patch PA to an external substance having fluidity. The delivery of the portion of the contained substance may be performed by the patch PA being inserted into or coming into contact with the substance having fluidity so that substance movement is possible between the liquid substance SB captured in the patch PA and the substance having fluidity.

Here, it is assumed that a substance is moved from the patch PA to another patch PA. In a contact region in which the patch PA and the other patch PA are in contact, at least a portion of the liquid substance B provided in the patch PA may be moved to the other patch PA.

In the contact region, the liquid substance SB provided in each patch PA may be diffused and moved to the other patch PA. In this case, due to the movement of the substance, a concentration of the liquid substance SB provided in each patch PA may be changed. Also in the present embodiment, as described above, the patch PA and the other patch PA may be separated, and a portion of the liquid substance SB in the patch PA may be provided to the other patch PA.

The substance movement between the patch PA and the other patch PA may be performed through a change in an environmental condition including a change in a physical state.

The substance movement between the patch PA and another patch PA may depend on an extent of a contact area between the patch PA and the other patch PA. For example, the substance movement efficiency between the patch PA and the other patch PA may be increased or decreased in accordance with an extent of an area where the patch PA comes into contact with the other patch PA.

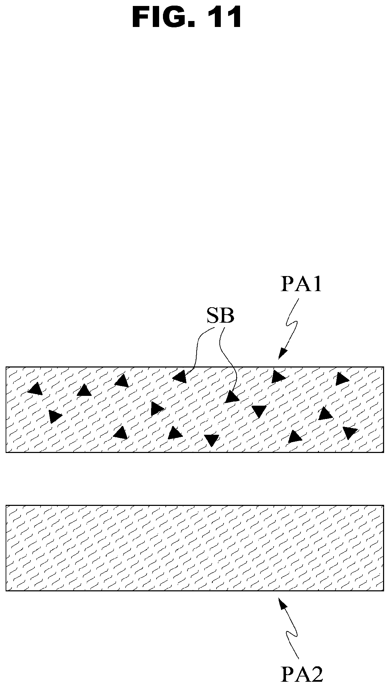

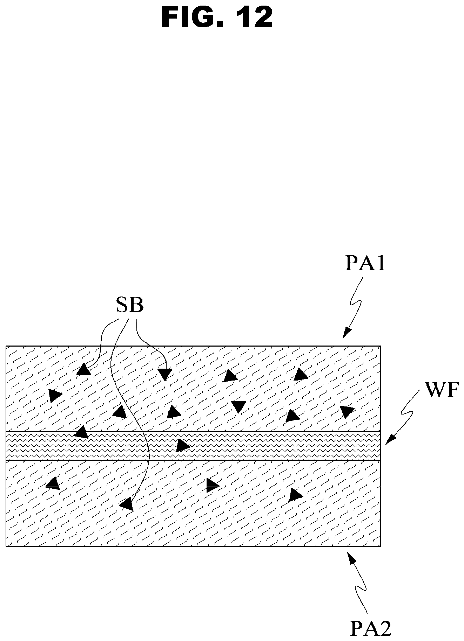

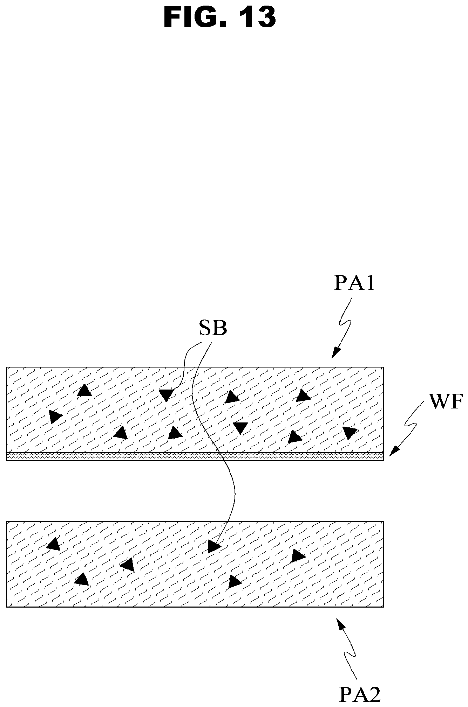

FIGS. 11 to 13 illustrate delivery of a substance from a patch PA1 to another patch PA2 as an example of delivery of a substance among the functions of the patch PA according to the present application. According to FIGS. 11 to 13, the patch PA1 may deliver a portion of a substance contained in the patch PA1 to the other patch PA2. The delivery of the portion of the substance may be performed by the patch PA1 coming into contact with the other patch PA2 and becoming a state in which a liquid substance SB captured in the patch PA1 and a substance captured in the other patch PA2 are exchangeable.

### 2.2.4.2 Absorption

Prior to description, it should be noted that, among the functions of the patch PA according to the present application, "absorption" may be managed similarly as the above-described "delivery" in some embodiments. For example, in a case in which a substance moves due to a concentration differences between substances, the "absorption" may be similar to the "delivery" in that a concentration of the liquid substance SB, particularly, a concentration of the additive substance AS, may be changed to control a direction in which the substance is moved. The "absorption" may also be similar to "delivery" in terms of controlling movement and selective absorption of a substance through a release of physical contact with the patch PA, and this may be clearly understood by those of ordinary skill in the art to which the present application pertains.

Due to the above-described characteristics, the patch PA according to the present application may capture an external substance. The patch PA may pull in an external substance present outside a region defined by the patch PA toward a region affected by the patch PA. The pulled external substance may be captured along with the liquid substance SB of the patch PA. The pulling of the external substance may be caused by an attractive force between the external substance and the liquid substance SB already captured in the patch PA. Alternatively, the pulling of the external substance may be caused by an attractive force between the external substance and a region of the mesh structural body NS not occupied by the liquid substance SB. The pulling of the external substance may be caused by a force of surface tension.

Hereinafter, for convenience, the above-described function of the patch PA will be referred to as "absorption." Absorption may be understood as a concept subordinate to the above-described channeling function of the patch PA, the concept defining movement of an external substance to the patch PA.

The absorption may occur by the patch PA via a state in which the substance is movable and a state in which the substance is immovable.

A substance that is absorbable by the patch PA may be in a liquid or solid state. For example, when the patch PA comes into contact with an external substance including a solid state substance, absorption of the substance may be performed due to an attractive force between the solid state substance included in the external substance and the liquid substance SB placed in the patch PA. As another example, when the patch PA comes into contact with a liquid external substance, the absorption may be performed due to binding between the liquid external substance and the liquid substance SB placed in the patch PA.

The external substance absorbed into the patch PA may be moved to the inside of the patch PA through the micro-cavities of the mesh structural body NS forming the patch PA or may be distributed on a surface of the patch PA. Positions at which the external substance is distributed may be set on the basis of a molecular weight or a particle size of the external substance.

While the absorption is performed, the form of the patch PA may be changed. For example, the volume, color, and the like of the patch PA may be changed. While the absorption into the patch PA is being performed, the absorption into the patch PA may be activated or delayed by adding external conditions such as a temperature change and a physical state change to an absorption environment of the patch PA.

The absorption will be described below as a function of the patch PA according to some examples of an external region that provides a substance to be absorbed into the patch PA when the absorption occurs.

Hereinafter, it will be assumed that the patch PA absorbs an external substance from a external plate PL. An example of the external plate may include a plate PL in which the external substance may be placed while the external substance is not absorbed thereinto.

A substance may be applied on the external plate PL. Particularly, a substance may be applied in a form of powder on the plate PL. The substance applied on the plate PL may be a single component or a mixture of a plurality of components.

The plate PL may have the shape of a flat plate. The shape of the plate PL may be deformed for improvement in ability to contain the substance or the like. For example, a well may be formed to improve the ability to contain the substance, a surface of the plate PL may be deformed by engraving or embossing, or a patterned plate PL may be used to improve contact with the patch PA.

The absorption of a substance from the plate PL by the patch PA according to the present application may be performed through contact between the plate PL and the patch PA. In this case, in a contact region in the vicinity of a contact surface between the plate PL and the patch PA, a water film WF may be formed due to the liquid substance SB captured in the patch PA and/or the substance applied on the plate PL. When the water film (aquaplane, hydroplane) WF is formed in the contact region, the substance applied on the plate PL may be captured by the water film WF. The substance captured in the water film WF may freely flow within the patch PA.

When the patch PA is spaced a predetermined distance or more apart and separated from the plate PL, the water film WF may be moved along with the patch PA, and the substance applied on the plate PL may be absorbed into the patch PA. The substance applied on the plate PL may be absorbed into the patch PA as the patch PA is separated a predetermined distance or more apart from the plate PL. When the patch PA and the plate PL are spaced apart and separated, the liquid substance SB provided to the patch PA may not be moved to the plate PL, or only an insignificant amount thereof may be absorbed into the patch PA.

A portion of or the entire substance applied on the plate PL may react specifically with a portion of or the entire substance captured in the patch PA. In this respect, absorption of a substance from the plate PL by the patch PA may be selectively performed. Particularly, the absorption may be performed selectively when the patch PA has a stronger attractive force than the plate PL with respect to a portion of the substance captured in the patch PA.

As an example, a portion of the substance may be fixed to the plate PL. In other words, a portion of the substance may be fixed to the plate PL while another portion of the substance is applied to have fluidity or not be fixed. In this case, when the patch PA and the plate PL are brought into contact and separated, the substance, excluding the portion of the substance fixed to the plate PL of the substance applied on the plate PL, may be selectively absorbed into the patch PA. Instead, the selective absorption may also occur due to polarities of a substance placed on the plate PL and a substance captured in the patch PA regardless of whether the substance is fixed.

As another example, when the liquid substance SB captured in the patch PA is bound specifically to at least a portion of a substance applied on the plate PL, only the portion of the substance applied on the plate PL bound specifically to the liquid substance SB may be absorbed into the patch PA when the patch PA is brought into contact with and then separated from the substance applied on the plate PL.

As yet another example, a portion of the substance applied on the plate PL may react specifically with a substance fixed to the plate PL in advance. In this case, only a remaining substance, excluding the substance that reacts specifically with the substance fixed to the plate PL in advance of the substance being applied to the plate PL, may be absorbed into the patch PA.

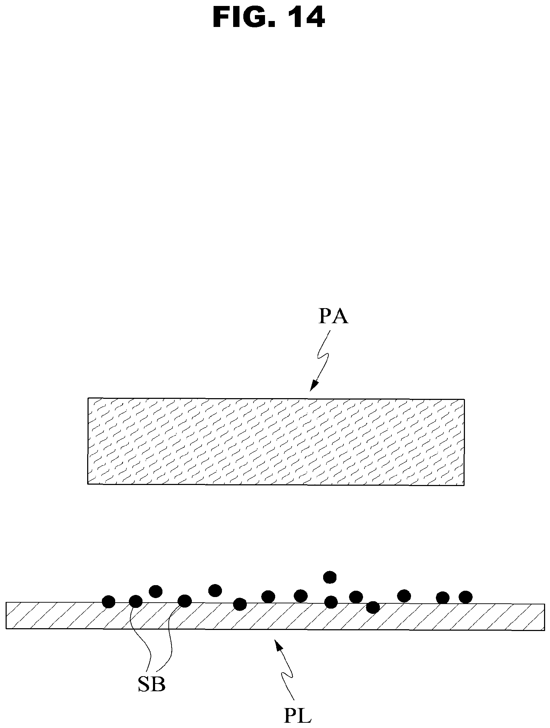

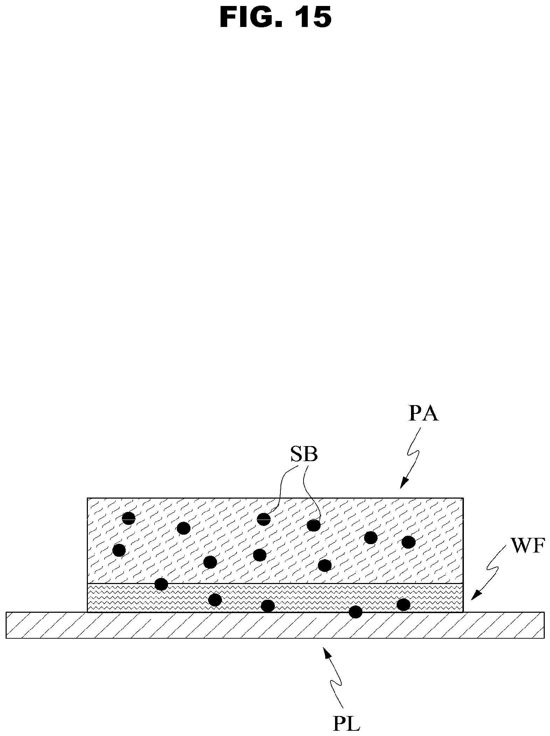

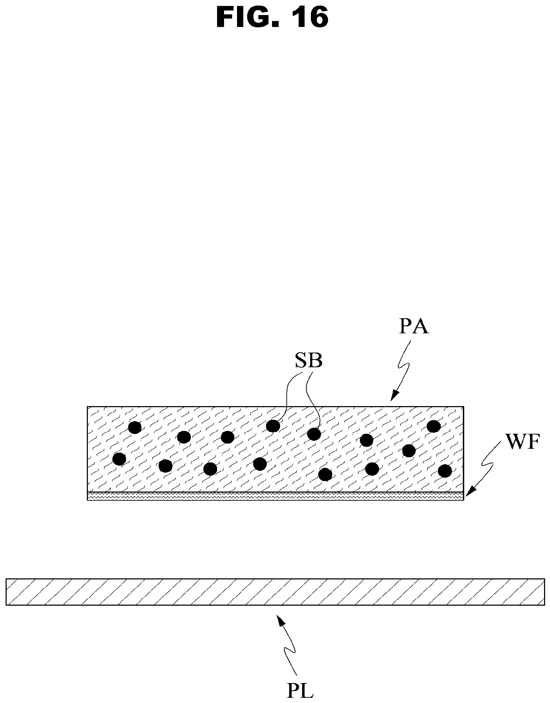

FIGS. 14 to 16 illustrate absorption of a substance from an external plate PL by the patch PA as an example of absorption of a substance from among the functions of the patch PA according to the present application. According to FIGS. 14 to 16, the patch PA may absorb a portion of a substance placed on the external plate PL from the external plate PL. The absorption of the substance may be performed by the patch PA coming into contact with the external plate PL, the water film WF being formed in the vicinity of a contact region between the external plate PL and the patch PA, and the substance being movable to the patch PA through the water film WF.

Here, it will be assumed that a substance is absorbed into the patch PA from the substance having fluidity SL. The substance having fluidity SL may refer to a liquid external substance that is held in other containing space or that is flowing. More specifically, by having an environment in which the substance having fluidity SL and the liquid substance SB captured in the patch PA may flow to and from each other, a portion of or the entire substance having fluidity SL may be absorbed into the patch PA. In this case, the environment in which the substance having fluidity SL and the liquid substance SB may flow to and from each other may be formed by the patch PA coming into contact with at least a portion of the substance having fluidity SL.

When the patch PA comes into contact with the substance having fluidity SL, the patch PA may be in a state in which a substance is movable from the substance having fluidity SL. When the patch PA is separated from the substance having fluidity SL, at least a portion of the substance having fluidity SL may be absorbed into the patch PA.

The absorption of a substance into the patch PA from the substance having fluidity SL may depend on a concentration difference between the substance captured in the patch PA and the substance having fluidity SL. In other words, when the concentration of the liquid substance SB captured in the patch PA with respect to a predetermined additive substance AS is lower than the concentration of the substance having fluidity SL with respect to the predetermined additive substance AS, the predetermined additive substance AS may be absorbed into the patch PA.

When a substance is absorbed into the patch PA from the substance having fluidity SL, in addition to the absorption depending on the concentration difference while the patch PA and the substance having fluidity SL are in contact as described above, the absorption into the patch PA may also be controlled by adding an electrical factor or changing a physical condition. Further, without direct contact between the substance captured in the patch PA and a substance to be absorbed, the absorption of a substance may also be performed through indirect contact therebetween via a medium.

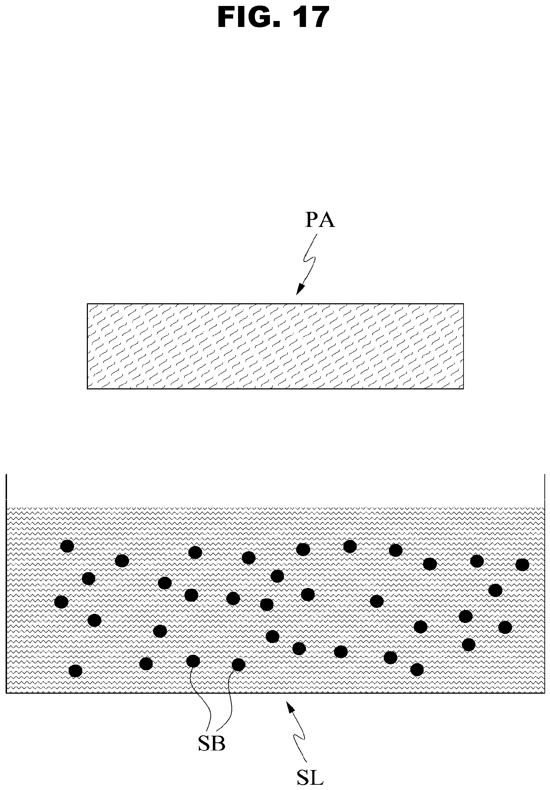

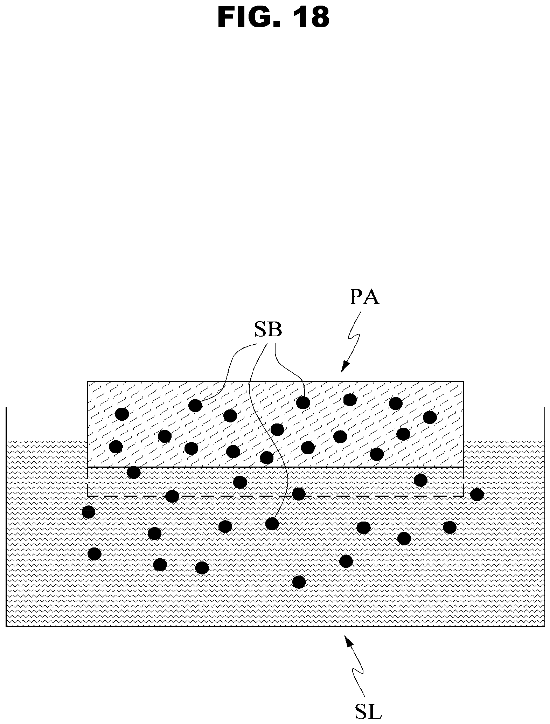

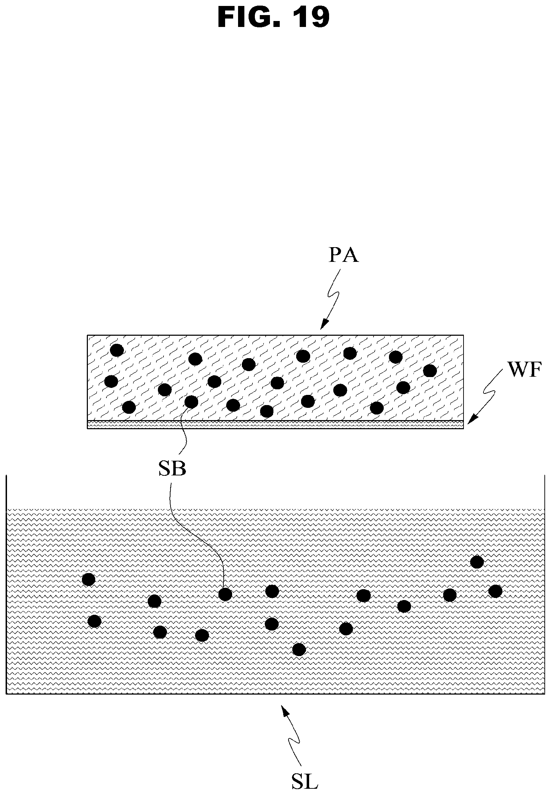

FIGS. 17 to 19 illustrate absorption of a substance from the substance having fluidity SL by the patch PA as an example of absorption of a substance from among the functions of the patch PA according to the present application. According to FIGS. 17 to 19, the patch PA may absorb a portion of the substance having fluidity SL. The absorption of a substance may be performed by the patch PA being immersed into the substance having fluidity SL or coming into contact with the substance having fluidity SL so that the liquid substance SB captured in the patch PA and the substance having fluidity SL are movable to and from each other.

Here, it will be assumed that the patch PA absorbs an external substance from another patch PA.

The absorption of an external substance from another patch PA by the patch PA may be performed due to a difference in binding force between the absorbed external substance and the substance already captured in the patch PA and between the absorbed external substance and the external substance not absorbed into the patch PA. For example, when the absorbed substance exhibits hydrophilic property, the patch PA exhibits hydrophilic property, and an attractive force between the absorbed substance and the patch PA is stronger than an attractive force between the other patch PA and the absorbed substance (that is, when the patch PA is more hydrophilic than the other patch PA), at least a portion of the external substance may be absorbed into the patch PA when the patch PA and the other patch PA are separated after being brought into contact.

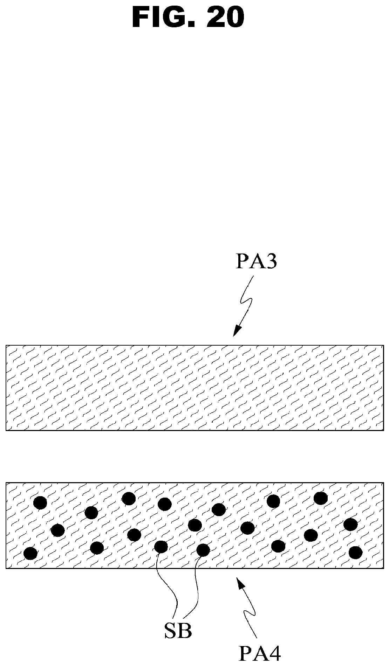

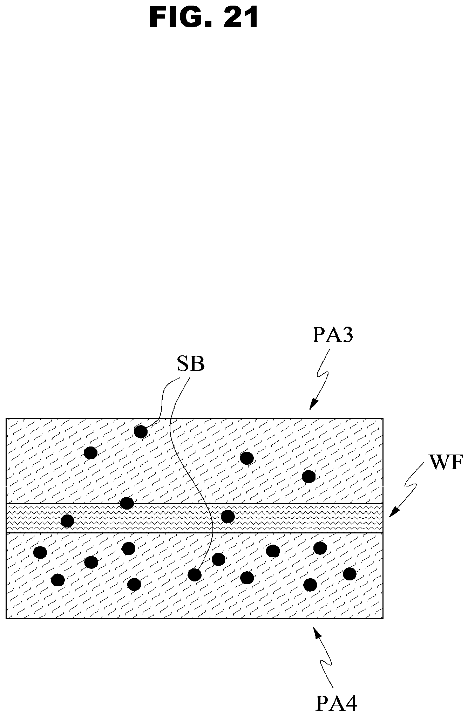

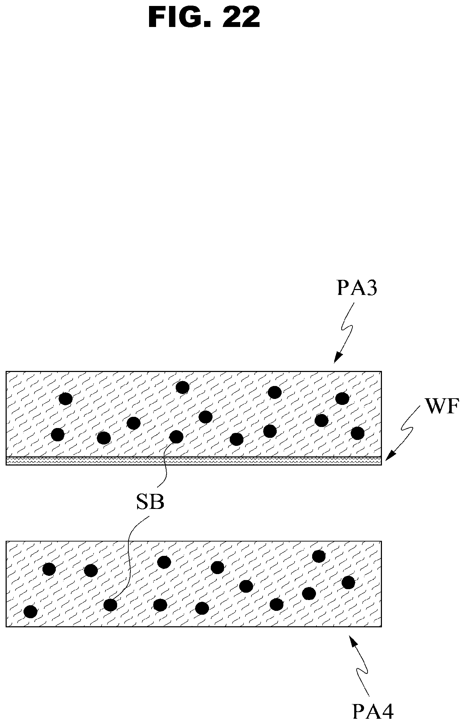

FIGS. 20 to 22 illustrate absorption of a substance from another patch PA4 by a patch PA3 as an example of absorption of a substance among the functions of the patch PA according to the present application. According to FIGS. 20 to 22, the patch PA3 may absorb a portion of a substance placed in the other patch PA4. The absorption of the substance may be performed by the patch PA3 coming into contact with the other patch PA4 so that a liquid substance SB captured in the patch PA3 and a liquid substance SB captured in the other patch PA4 are exchangeable.

A binding force of the patch PA to the external substance absorbed thereinto may be changed in accordance with a proportion of a frame structural body of the three-dimensional mesh structural body NS constituting the patch PA with respect to the total volume of the patch PA. For example, as the proportion of a volume occupied by the frame structural body in the entire patch PA increases, the amount of substance captured in the structural body may be reduced. In this case, a binding force between the patch PA and a target substance may be reduced due to a reason such as reduction in a contact area between the target substance and the substance captured in the patch PA.

In relation to this, ratios of materials that constitutes the mesh structural body NS may be adjusted during manufacturing process of the patch PA so that polarity of the patch PA is controlled. For example, in the case of a patch PA manufactured using agarose, a concentration of the agarose may be controlled to adjust a degree of the absorption.

When the certain region has a weaker binding force than the patch PA with respect to a substance provided from the patch PA, and the patch PA and another patch PA are brought into contact and then separated, the absorbed external substance may be separated from the other patch PA along with the patch PA.

### 2.2.4.3 Providing of environment

Due to the above-described characteristics, the patch PA according to the present application may perform a function of adjusting an environmental condition of a desired region. The patch PA may provide an environment due to the patch PA to the desired region.

The environmental condition due to the patch PA may depend on the liquid substance SB captured in the patch PA. The patch PA may create a desired environment in a substance placed in an external region on the basis of characteristics of a substance accommodated in the patch PA or for a purpose of making the environment correspond to characteristics of the substance accommodated in the patch PA.

The adjustment of the environment may be understood as changing an environmental condition of the desired region. The changing of the environmental condition of the desired region may be implemented in a form in which a region affected by the patch PA is expanded to include at least a portion of the desired region or a form in which an environment of the patch PA is shared with the desired region.

Hereinafter, for convenience, the above-described function of the patch PA will be referred to as "providing an environment."

The providing an environment by the patch PA may be performed in a state in which a substance is movable between the patch PA and an external region subject to provide the environment. The providing an environment by the patch PA may be performed through contact. For example, when the patch PA comes into contact with a desired region (for example, an external substance, a plate PL, or the like), a specific environment may be provided to the desired region by the patch PA.

The patch PA may adjust an environment of a target region TA by providing an environment with an appropriate pH, osmotic pressure, humidity level, concentration, temperature, and the like. For example, the patch PA may provide fluidity(liquidity) to the target region TA or a target substance. Such providing fluidity may occur due to movement of a portion of a substance captured in the patch PA. A moist environment may be provided to the target region TA through the liquid substance SB or the base substance BS captured in the patch PA.

The environmental factors provided by the patch PA may be constantly maintained in accordance with a purpose. For example, the patch PA may provide homeostasis to the desired region. As another example, as a result of providing an environment, the substance captured in the patch PA may be adapted to an environmental condition of the desired region

The providing an environment by the patch PA may result from diffusion of the liquid substance SB included in the patch PA. That is, when the patch PA and the desired region come into contact, a substance may be movable through a contact region that is formed due to contact between the patch PA and the desired region. In relation to this, an environmental change due to an osmotic pressure, an environmental change due to a change in ionic concentration, providing a moist environment, and a change in a pH level may be implemented in accordance with a direction in which the substance is diffused.

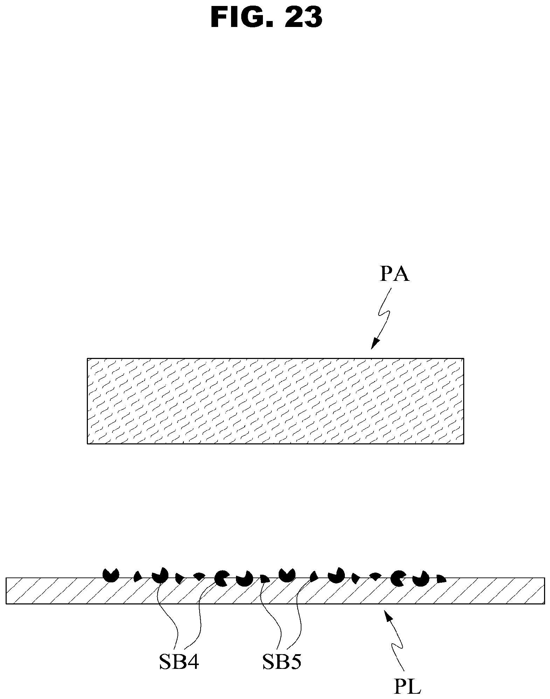

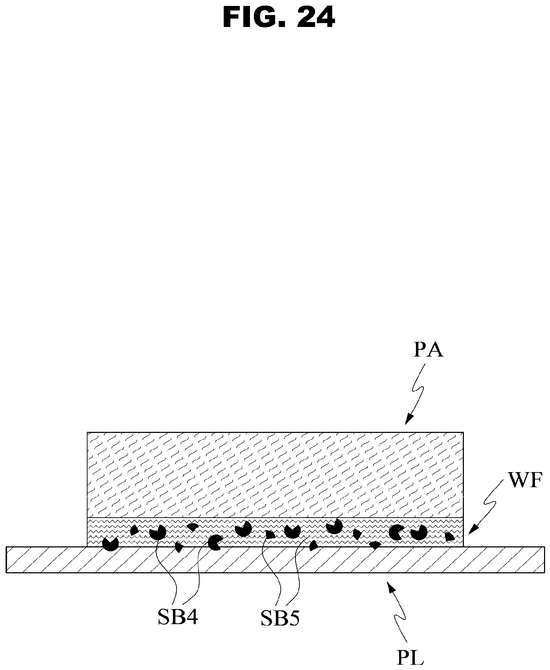

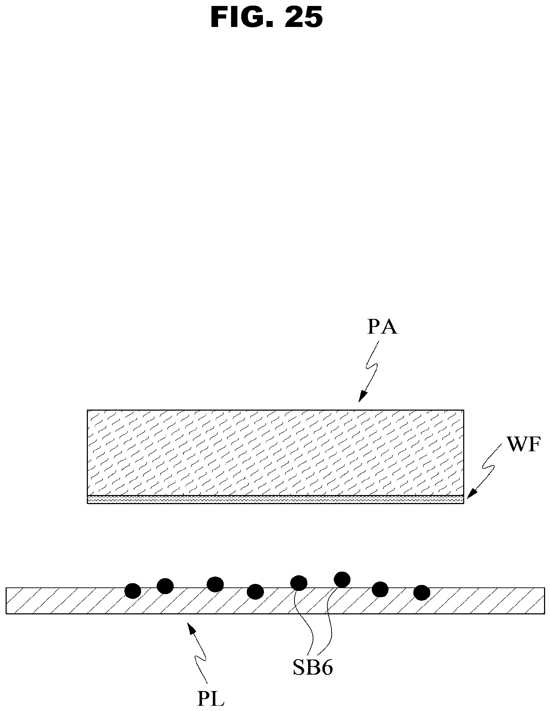

FIGS. 23 to 25 illustrate providing a predetermined environment to an external plate PL by the patch PA as an example of providing an environment among the functions of the patch PA according to the present application. According to FIGS. 23 to 25, the patch PA may provide a predetermined environment to an external plate PL on which a fourth substance SB4 and a fifth substance SB5 are placed. For example, the patch PA may provide a predetermined environment to the plate PL for the fourth substance SB4 and the fifth substance SB5 to react and form a sixth substance SB6. The providing the environment may be performed by the patch PA coming into contact with the plate PL so that a water film WF is formed in the vicinity of a contact region and the fourth substance SB4 and the fifth substance SB5 are captured in the water film WF.

### 3. Application of patch

The patch PA according to the present application may be implemented to perform various functions by suitably applying the above-described functions of the patch PA.

The technical spirit of the present application will be described below by disclosing some embodiments. However, the technical scope to which functions of the patch PA disclosed by the present application are applied may be interpreted in a broad sense within the scope that may be easily derived by those of ordinary skill in the art, and the scope of the present application should not be interpreted as being limited by the embodiments disclosed herein.

### 3.1. In-patch

The patch PA may provide a reaction region for a substance. In other words, a reaction of a substance may occur in at least a portion of a spatial region affected by the patch PA. In this case, the reaction of a substance may be a reaction between liquid substances SB captured in the patch PA and/or a reaction between the captured liquid substance SB and a substance provided from the outside of the patch PA. The providing a reaction region for a substance may activate or promote a reaction of a substance.

In this case, the liquid substance SB captured in the patch PA may include at least one of a substance added upon manufacturing the patch PA, a substance additive into the patch PA after the manufacturing of the patch PA and contained in the patch PA, and a substance temporarily captured in the patch PA. In other words, regardless of a form in which a substance is captured in the patch PA, any substance captured in the patch PA at a time point at which a reaction in the patch PA is activated may react in the patch PA. Further, a substance injected after the manufacturing of the patch PA may also act as a reaction initiator.

The providing a reaction region for a reaction related to the liquid substance SB captured in the patch PA may be a concept subordinate, in terms of embodiment, to the above-described Section 2.1.3 (that is, providing reaction space). Alternatively, the providing a reaction region for a reaction related to the liquid substance SB captured in the patch PA may consist of multiple concepts that perform combined functions of the above-described Section 2.1.3 and Section 2.2.4.2 (that is, absorption). The providing a reaction region for a reaction related to the liquid substance SB captured in the patch PA is not limited thereto and may be implemented in the form in which two or more functions are combined.

### 3.1.1 First embodiment

Hereinafter, description will be given by assuming that the function of absorption into the patch PA and the function of providing a reaction space (hereinafter referred to as "providing function") are performed by a single patch PA. In this case, the absorption function and the providing function may be simultaneously-performed functions, functions performed at different time points, or functions sequentially performed to perform another function. The patch PA further including other functions in addition to the absorption and providing functions may also be considered as belonging to the present embodiment.

As described above, the patch PA may perform a function of capturing a substance, and the substance may have fluidity even when the substance is captured. When some components of the liquid substance SB are non-uniformly distributed, the non-uniform components may be diffused. Even when components of the liquid substance SB are uniformly distributed, the liquid substance SB may have a predetermined level of mobility due to irregular motion of particles. In this case, a reaction between substances, for example, specific binding between substances, may occur inside the patch PA.

For example, in the patch PA, in addition to a reaction between captured substances, a reaction in a form in which a substance having fluidity that is newly captured in the patch PA and the substance that has been captured in the patch PA bind specifically to each other may also be possible.

The reaction between the substance having fluidity and the substance that has been captured in the patch PA may also occur after the substance patch being separated from an space that has been provided. For example, after the patch PA absorbs the substance having fluidity from an arbitrary space, the patch PA may be separated from the arbitrary space, and a reaction between the absorbed substance and the substance that has been captured in the patch PA may occur in the patch PA.

In addition, the patch PA may allow a reaction of a substance captured therein to occur by performing the absorption function with respect to a substance having fluidity,. In other words, the absorption of the substance having fluidity by the patch PA may act as a trigger for a reaction between the absorbed substance and the substance that has been captured in the patch PA. The reaction may occur inside a space defined by the patch PA.

A composition of the liquid substance SB captured in the patch PA may be changed due to the reaction occurring inside the patch PA. When, particularly, a substance captured inside the patch PA is a compound, a chemical composition thereof may be changed before and after a reaction. Alternatively, a composition distribution of a substance may be changed in accordance with a position of the substance in the patch PA. For example, this may be due to diffusion or particles having an attractive force specific to another substance.

When the composition of the liquid substance SB is changed due to a reaction inside the patch PA, a portion of the substance may be absorbed into the patch PA due to a concentration difference between the patch PA and a substance outside the patch PA (when a substance in contact with the patch PA is present, the corresponding substance), or the substance may be released from the patch PA to the substance outside the patch PA.

### 3.1.2 Second embodiment

Hereinafter, an embodiment in which the containing function of the patch PA and the function of providing a reaction space for a substance are performed together for at least a predetermined amount of time will be described. More specifically, the patch PA may perform a function of providing a space for at least a portion of the liquid substance SB contained in the patch PA to react.

The patch PA may contain a substance and provide a reaction space for the contained substance. In this case, the reaction space provided by the patch PA may be the micro-cavities formed by the mesh structural body NS of the patch PA or a surface region of the patch PA. Particularly, when a substance contained in the patch PA and a substance applied on a surface of the patch PA react, the reaction space may be the surface region of the patch PA.

The reaction space provided by the patch PA may serve to provide a specific environmental condition. While a reaction occurs in the liquid substance SB placed in the patch PA, an environmental condition of the reaction may be adjusted by the patch PA. For example, the patch PA may serve as a buffer solution.

By containing a substance through a mesh structure, the patch PA does not require a container, separately. When the reaction space of the patch PA is a surface of the patch PA, a reaction may be easily observed through the surface of the patch PA. For this, the shape of the patch PA may be deformed into a shape that facilitates the observation.

The liquid substance SB contained in the patch PA may be denaturalized or react with a different type of substance. The composition of the liquid substance SB contained in the patch PA may be changed with time.

The reaction may refer to a chemical reaction in which a chemical formula is changed, a physical state change, or a biological reaction. In this case, the liquid substance SB contained in the patch PA may be a substance formed of a single component or a mixture including a plurality of components.

### 3.2 Providing of movement path (channeling)

Hereinafter, the patch PA that performs a function of providing a substance movement path will be described. More specifically, as described above, the patch PA may capture, absorb, release, and/or contain a substance having fluidity. Various embodiments of the patch PA that performs the function of providing a substance movement path may be implemented by each of the above-described functions of the patch PA or a combination thereof. However, a few embodiments will be disclosed for a better understanding.

### 3.2.1 Third embodiment

The patch PA may be implemented to perform functions described in Section 2.2.4.1 (that is, the section related to delivery) and Section 2.2.4.2 (that is, the section related to absorption) among the above-described functions of the patch PA. In this case, the absorption function and the delivery function may be provided together or sequentially provided.

The patch PA may perform the absorption and delivery functions together to provide a substance movement path. Particularly, the patch PA may absorb an external substance and provide the absorbed external substance to an external region, thereby providing a movement path to the external substance.

The providing the movement path of the external substance by the patch PA may be performed by absorbing the external substance and releasing the external substance. More specifically, the patch PA may come into contact with the external substance, absorb the external substance, come into contact with the external region, and deliver the external substance to the external region. In this case, the capturing of the external substance and the delivery of the captured external substance to the external region by the patch PA may be performed through a process similar to those of the above-described absorption and delivery.

The external substance absorbed into the patch PA and provided may be in a liquid phase or a solid phase.

In this way, the patch PA may allow a portion of the external substance to be provided to another external substance. The external substance and the other external substance may simultaneously come into contact with the patch PA. The external substance and the other external substance may come into contact with the patch PA at different time points.

The external substance and the other external substance may come into contact with the patch PA at different time points. When the external substances come into contact with the patch PA at different time points, the external substance may come into contact with the patch PA first, and after the external substance and the patch PA are separated, the patch PA and the other external substance may come into contact. In this case, the patch PA may temporarily contain a substance captured from the external substance.

The patch PA may simultaneously provide a substance movement path and additionally provide a time delay. The patch PA may perform a function of suitably adjusting an amount of substance provided to another external substance and a speed of such providing.

Such a series of processes may be carried out in one direction with respect to the patch PA. As a specific example, absorption of a substance may be performed through a surface of the patch PA, an environment may be provided in an inner space of the patch PA, and the substance may be released through another surface facing the surface.

### 3.2.2 Fourth embodiment

The patch PA may perform the absorbing and releasing of a substance among the above-described functions of the patch PA and the providing a reaction space for the substance simultaneously. In this case, the absorption and release of the substance and the providing the reaction space may be performed simultaneously or sequentially.

According to an embodiment, in performing the processes of absorbing and releasing an external substance, the patch PA may provide a reaction space to the absorbed external substance for at least a predetermined amount of time. The patch PA may provide a specific environment for at least some time to the liquid substance SB captured in the patch PA, including the absorbed external substance.

The liquid substance SB that has been captured in the patch PA and the external substance captured in the patch PA may react inside the patch PA. The external substance absorbed into the patch PA may be affected by an environment provided by the patch PA. The substance released from the patch PA may include at least a portion of a substance generated through the reaction. The external substance may be released from the patch PA after the composition, characteristics, and the like of the external substance are changed.

The absorbed substance may be released from the patch PA. The external substance being absorbed into the patch PA and being released from the patch PA may be understood as the external substance passing through the patch PA. The external substance that has passed through the patch PA may lose integrity due to a reaction inside the patch PA or an influence of an environment provided by the patch PA.

The above-described processes of absorption of an external substance, reaction of a substance, and providing the substance may be carried out in one direction. In other words, the absorption of a substance may be performed at one position of the patch PA, the providing an environment may be performed at another position of the patch PA, and the release of the substance may be performed at yet another position of the patch PA.

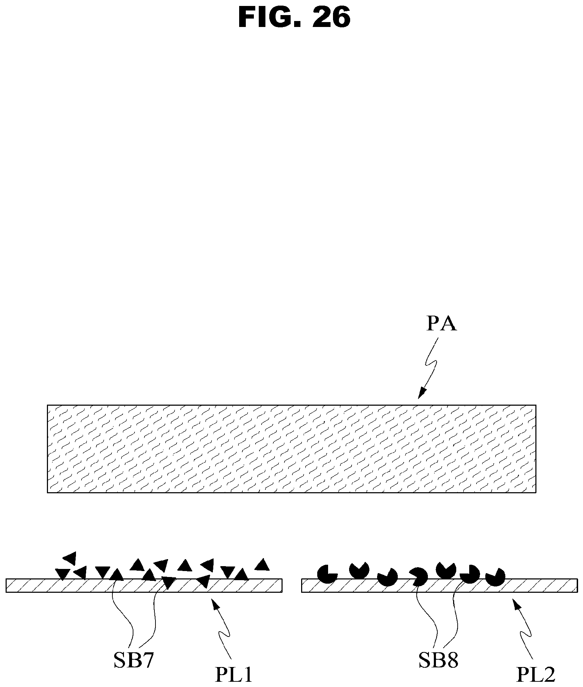

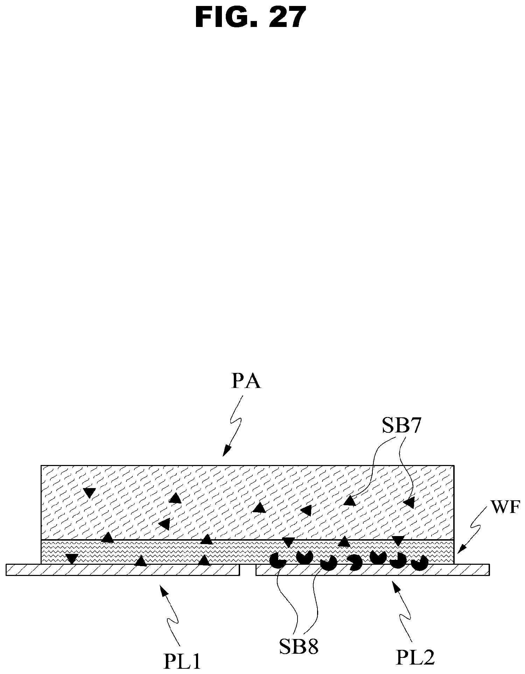

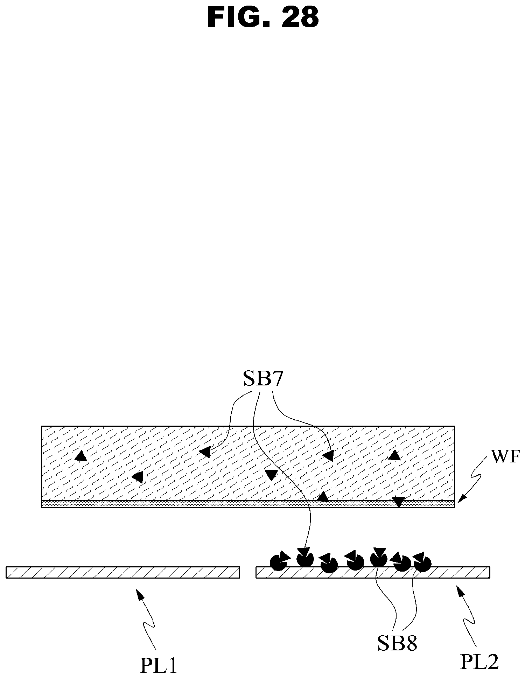

FIGS. 26 to 28 illustrate providing a substance movement path between two plates PL as an embodiment of the patch PA according to the present application. According to FIGS. 26 to 28, the patch PA may provide a substance movement path between a plate PL1 on which a seventh substance SB7 is applied and a plate PL2 on which an eighth substance SB8 is applied. As a specific example, when the seventh substance SB7 is capable of binding to the eighth substance, and the eighth substance is fixed to the plate PL2, the patch PA may come into contact with the plates PL1 and PL2 so that the seventh substance SB7 is moved through the patch PA and bound to the eighth substance SB8. The seventh substance SB7 and the eighth substance SB8 may be connected to the patch PA through a water film WF formed by the patch PA coming into contact with the plates PL1 and PL2.

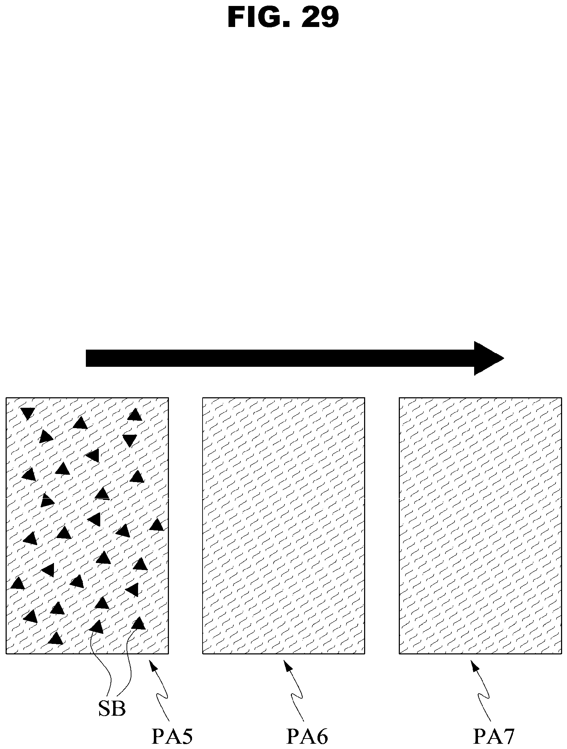

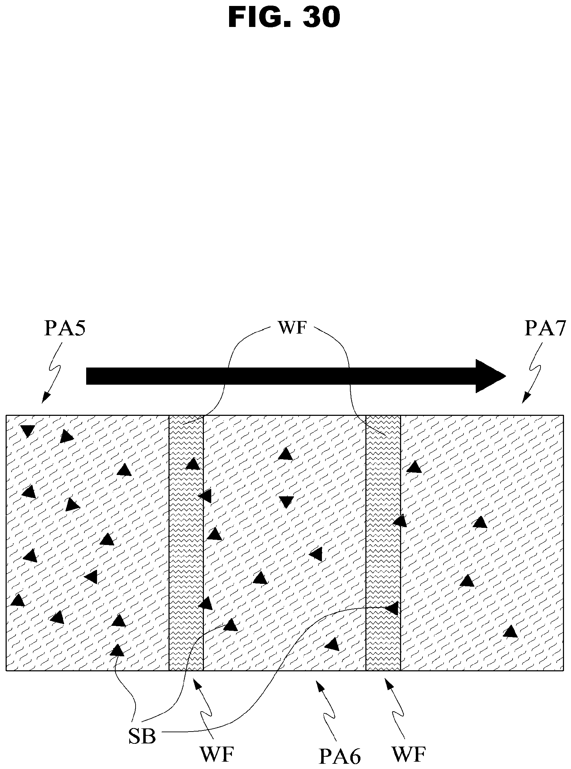

FIGS. 29 and 30 illustrate providing a substance movement path between two patches as an embodiment of the patch PA according to the present application. According to FIGS. 29 and 30, a patch PA6 configured to provide the movement path may be in contact with a patch PA5 configured to contain a substance to be moved, and a patch PA7 configured to receive the substance to be moved. The patch PA6 configured to provide the movement path may come into contact with the patch PA5 configured to contain the substance to be moved and the patch PA7 configured to receive the substance to be moved, and the substance to be moved may be moved to the patch PA7 configured to receive the substance to be moved. The movement of the substance between the patches may be performed by a water film WF formed in the vicinity of a contact region between the patches.

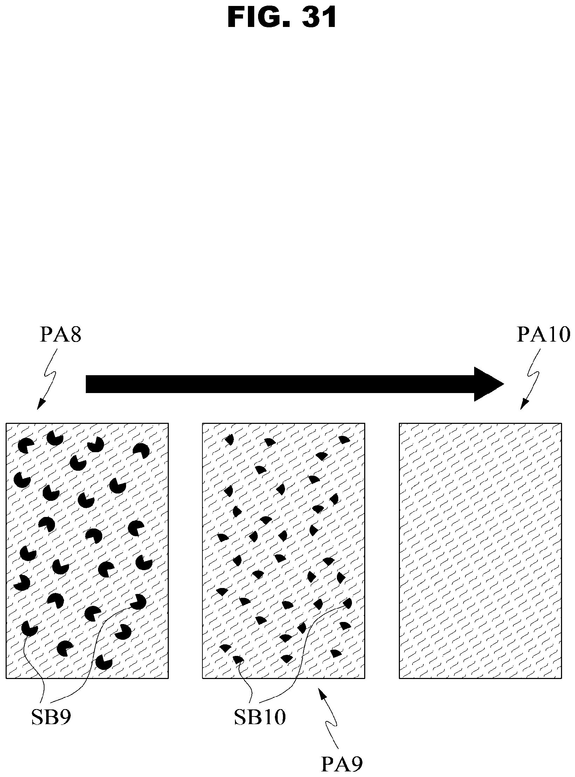

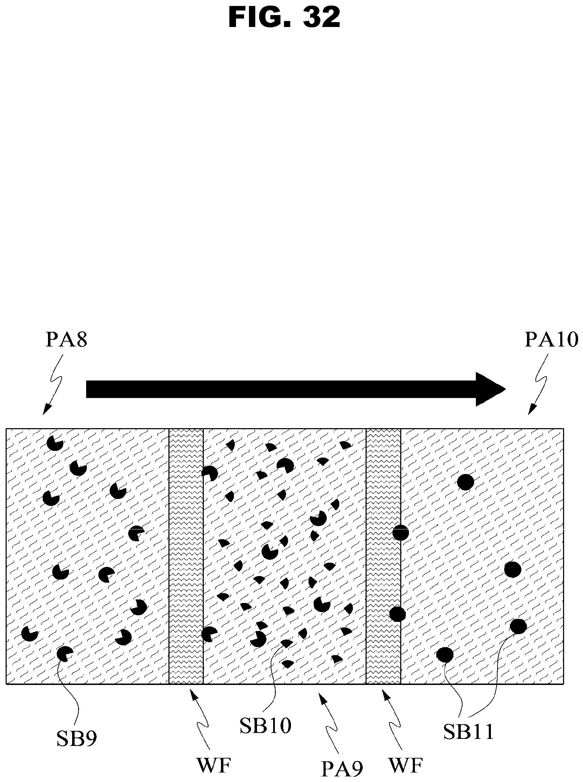

FIGS. 31 and 32 illustrate providing a substance movement path between two patches as an embodiment of the patch according to the present application. According to FIGS. 29 and 30, a patch PA9 configured to provide the movement path may be in contact with a patch PA8 configured to contain a ninth substance SB9 and a patch PA10 configured to receive a substance. The patch PA9 providing the movement path may come into contact with the patch PA8 configured to contain the ninth substance SB9 to absorb the ninth substance SB9. The absorbed ninth substance SB9 may react with a tenth substance SB10 contained in the patch PA9, which is configured to provide the movement path, and generate an eleventh substance. An eleventh substance SB11 may be provided from the patch PA9 configured to provide the movement path to the patch PA10 configured to receive the substance. The movement of a substance between the patches PA may be performed through a water film WF formed in the vicinity of a contact region between the patches PA.

### 3.3 Multi-patch

A patch PA may be solely used, or a plurality of patches PA may be used together. In this case, the plurality of patches PA being able to be used together includes a case in which the plurality of patches PA are sequentially used as well as a case in which the plurality of patches PA are used simultaneously.

When the plurality of patches PA are used simultaneously, the patches PA may perform different functions. Although each patch PA of the plurality of patches PA may contain the same substance, the plurality of patches PA may also contain different substances.

When the plurality of patches PA are used simultaneously, the patches PA may not come into contact with each other such that substance movement does not occur between the patches PA, or a desired function may be performed in a state in which substances contained in the patches PA are exchangeable.

Although the plurality of patches PA used together may be manufactured in shapes similar to each other or in the same size, the plurality of patches PA may be used together even when the plurality of patches PA have different shapes. Each patch PA constituting the plurality of patches PA may be manufactured such that densities of the mesh structural bodies NS are different or components constituting the mesh structural bodies NS are different.

### 3.3.1 Contact with plurality of patches

When a plurality of patches PA are used, the plurality of patches PA may come into contact with a single target region TA. The plurality of patches PA may come into contact with the single target region TA and perform a desired function.

When a plurality of target regions TA are present, the plurality of patches PA may come into contact with different target regions TA. When the plurality of target regions TA are present, the plurality of patches PA may respectively come into contact with corresponding target regions TA and perform a desired function.

The plurality of patches PA may come into contact with a substance applied on the target region TA. In this case, the substance applied on the target region TA may be fixed or have fluidity.

The desired function may be a function of providing or absorbing the substance. However, each patch PA does not necessarily provide the same substance or absorb the same substance, and the patches PA may provide different substances to the target region TA or absorb different components from a substance placed in the target region TA.

The desired function may be different for each patch PA constituting the plurality of patches PA. For example, one patch PA may perform the function of providing a substance to the target region TA, and another patch PA may perform the function of absorbing the substance from the target region TA.

The plurality of patches PA may include different substances, and the different substances may be provided to a single target region TA and used to induce a desired reaction. When a plurality of components of a substance is required for the desired reaction to occur, the plurality of components may be contained in a plurality of patches PA respectively and provided to the target region TA. Such use of the plurality of patches PA may be particularly useful when properties of substances required for a desired reaction are lost or altered when the substances required for the reaction being mixed for reasons such as being contained in a single patch PA.

According to an embodiment, when the plurality of patches PA include substances formed of different components, and the substances formed of different components have different specific binding relationships, the substances formed of different components may be provided to the target region TA. The plurality of patches PA may be used to detect a plurality of specific bindings from the substances applied on the target region TA, by providing the substances including different components.

According to another embodiment, the plurality of patches PA may include substances formed of the same component, but each patch PA may have a different concentration with respect to the substance formed of the same component. The plurality of patches PA including the substances formed of the same component may come into contact with the target region TA and be used to determine an influence in accordance with a concentration of the substance included in the plurality of patches PA.

When the plurality of patches PA are used as described above, the patches PA may be grouped into more efficient forms and used. In other words, the configuration of the plurality of patches PA being used may be changed every time the plurality of patches PA are used. The plurality of patches PA may be manufactured in the form of a cartridge and used. In this case, the form of each patch PA being used may be suitably standardized and manufactured.

The plurality of patches PA in the form of a cartridge may be suitable when patches PA configured to contain a plurality of types of substances are manufactured to be used by being chosen as necessary.

Particularly, when attempting to detect a specific reaction of each substance from the target region TA using a plurality of types of substances, a combination of specific reactions to be detected may be changed every time the detection is performed.

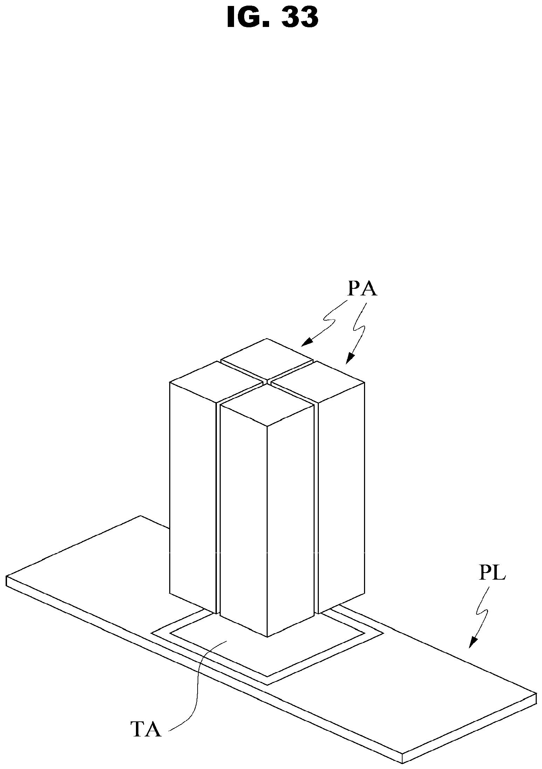

FIG. 33 illustrates a case in which the plurality of patches PA are used together as an embodiment of the patch PA according to the present application. According to FIG. 33, the plurality of patches PA according to an embodiment of the present application may simultaneously come into contact with a target region TA placed on a plate PL. The patches PA constituting the plurality of patches PA may have a standardized form. The plurality of patches PA may include a first patch and a second patch, and a substance contained in the first patch may be different from a substance contained in the second patch.

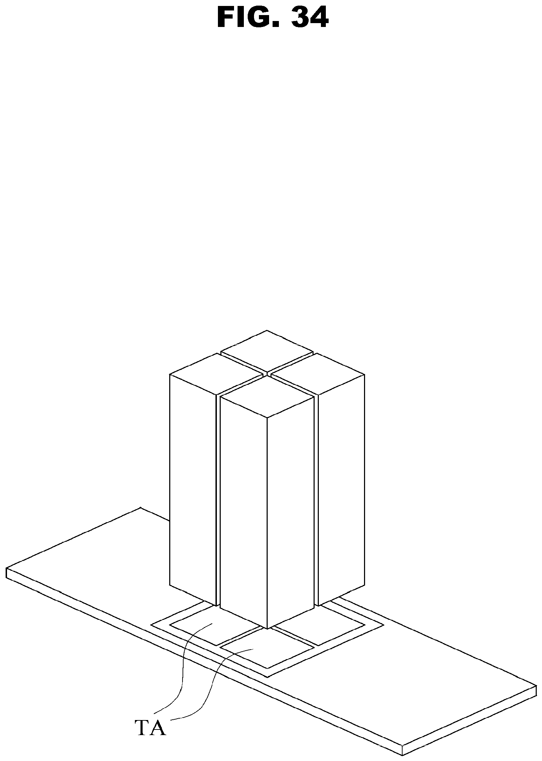

FIG. 34 illustrates a case in which the plurality of patches PA are used and the plate PL includes a plurality of target regions TA. According to FIG. 34, the plurality of patches PA according to an embodiment of the present application may simultaneously come into contact with the plurality of target regions TA placed on the plate PL. The plurality of patches PA may include a first patch PA and a second patch PA, the plurality of target regions TA may include a first target region and a second target region, and the first patch may come into contact with the first target region and the second patch may come into contact with the second target region.

### 3.3.2 Fifth embodiment

The plurality of patches PA may perform a plurality of functions. As described above, the patches PA may simultaneously perform a plurality of functions, and the patches PA may also simultaneously perform different functions. However, embodiments are not limited to the above, and the functions may also be combined and performed in the plurality of patches PA.

First, in the case in which the patches PA simultaneously perform the plurality of functions, the patches PA may perform both containing and release of a substance. For example, the patches PA may contain different substances and release substances contained in the target regions TA. In this case, the contained substances may be simultaneously or sequentially released.

Next, in the case in which the patches PA simultaneously perform different functions, the patches PA may separately perform containing and release of a substance. In this case, only some of the patches PA may come into contact with a target region TA and release a substance to the target region TA.

### 3.3.3 Sixth embodiment

When a plurality of patches PA are used, as described above, the plurality of patches PA may perform a plurality of functions. First, the patches PA may simultaneously perform containing, releasing, and absorbing of substances. Alternatively, the patches PA may also separately perform the containing, releasing, and absorbing of the substances. However, embodiments are not limited thereto, and the functions may also be combined and performed in the plurality of patches PA.

For example, at least some of the plurality of patches PA may contain a substance and release the contained substance to the target region TA. In this case, at least a remainder of the plurality of patches PA may absorb a substance from the target region TA. Some of the plurality of patches PA may release a substance that binds specifically to a substance placed in the target region TA. In this case, specific binding may be detected by absorption of a substance that has not formed specific binding from the substance placed in the target region TA using another patch PA.

### 3.3.4 Seventh embodiment

When a plurality of patches PA are used, the patches PA may simultaneously perform containing and release of a substance and providing an environment. Alternatively, the patches PA may separately perform the containing and release of a substance and providing an environment. However, embodiments are not limited thereto, and the functions may also be performed in combination in the plurality of patches PA.

For example, a patch PA among the plurality of patches PA may release a substance contained therein to the target region TA. In this case, another patch PA may provide an environment to the target region TA. Here, the providing an environment may be implemented in the form in which an environmental condition of a substance contained in the other patch PA is provided to the target region TA. More specifically, a reacting substance may be provided to the target region TA by the patch PA, and the other patch PA may come into contact with the target region TA and provide a buffering environment.

As another example, the plurality of patches PA may be in contact with each other. In this case, at least one patch PA may contain a substance and release the substance contained therein to another patch PA configured to provide an environment. In the present embodiment, the patch PA configured to provide an environment may release a substance, come into contact with at least one other patch PA that is not in contact with the patch PA configured to provide an environment, and absorb a substance from the patch PA.

### 4. Circulating Tumor Cell (CTC) diagnosis

### 4.1 CTC

A patch PA according to the present application may be used in cancer diagnosis. Particularly, the patch PA may be used in CTC testing.

Tumor cells CE are known to destroy normal cells CE and metastasize through blood vessels or lymphatic vessels. Theblood vessels and the lymphatic vessels are connected to organs located in the human body. The tumor cells CE can move to tissues which are in distance (such as a lymphatic gland, liver, or a lung) along a flow of blood or lymph and form new tumor tissue.

In relation to this, CTC testing is performed on tumor cells CE that float in the blood of a person subject to diagnosis (hereinafter, "circulating tumor cell CE"). Circulating tumor cell CE refers to tumor cells CE that move along the blood or lymph.

CTC testing has an advantage of being capable of detecting tumor cells CE even when tumor cells CE are not detected in computerized tomography (CT) or an imaging examination. That is, even when a metastasized tumor tissue has not grown to an extent that the tumor tissue may be detected in an imaging examination, the tumor cells CE moving through the blood for metastasis may be detected when CTC testing is used.

Also, CTC testing has an advantage of being capable of simply diagnosing cancer by using the blood without collecting a biopsy specimen. In relation to the fact that cancer is a disease that requires continuous monitoring even after a full recovery, being able to perform cancer diagnosis through a simple procedure is a very important advantage.

Hereinafter, prior to describing various embodiments related to a method in which a patch PA is used to detect circulating tumor cell CE, a method of diagnosing the circulating tumor cell CE will be described first.

### 4.2 Diagnostic method

In order to detect circulating tumor cell CE and diagnose cancer, general diagnostic methods that are performed to detect cells CE may be applied.

For example, there is a method in which the morphology of circulating tumor cell CE is detected. By identifying The cells CE distributed in theblood and considering the sizes and morphology of the cells CE, detecting the presence oftumor cells CE as well as sorting out the danger group of tumor cells CE may be performed.

Also, for more accurate detection of tumor cells CE, staining cells CE distributed in the blood may be additionally performed. For example, staining a cytoplasm of cells CE distributed in the blood or staining a nucleus of the cells CE may be performed.

In another example, there is a method in which immunoassay is performed on the circulating tumor cell CE. Using a tumor marker of the tumor cells CE, detection of the presence of tumor cells CE in the blood may be performed. The tumor marker may be an antibody AB that reacts specifically with epithelial cells CE or tumor cells CE.

In yet another example, there is a method in which culturing of the circulating tumor cell CE is performed.

While normal cells CE generally stop cell CE division at an arbitrary time point and go through apoptosis, tumor cells CE proliferate infinitely. Also, speed of cell division of the tumor cells CE is known to be higher than that of the normal cells CE.

Due to such differences, blood may be cultured to detect tumor cells CE in the blood. More specifically, tumor cells CE may be detected by identifying a speed of cell division of the cells CE in the blood and a time point at which the cell division has ended.

In still another example, there is a method in which extracting DNA from the circulating tumor cell CE and a PCR process using the extracted DNA is performed.

Diagnosis of cancer may be performed by amplifying a target DNA using primer to which a base sequence of DNA expressed in a tumor tissue is reflected, and identifying whether extracted DNA has been amplified by using a label or the like mentioned above. In some cases, the method for diagnosis of cancer using DNA mentioned above may also be used to determine the type of cancer expressed in the sample SA.

### 5. Performance of diagnosis

### 5.1 Preparation of sample SA

### 5.1.1 Providing sample SA

The cancer diagnosis according to the present application may be performed on blood in order to detect circulating tumor cell CE. Although not essential, in consideration of an average proportion of circulating tumor cell CE found in the blood of cancer patients, about 60 ml of blood may be used for an effective cancer diagnosis.

The sample SA may be provided on a plate PL. Also, the sample SA may be provided in a single layer on the plate PL if necessary. To provide the sample SA in a single layer, smearing the sample SA on the plate PL or printing the sample SA by adjusting a discharge speed and a discharge position of the sample SA may be used.

When the sample SA is provided in a single layer, some cells CE in the sample SA (for example, white blood cells and red blood cells) may be arranged in a two-dimensional array.

When the sample SA is provided in a single layer on the plate PL, the number of overlapping cells CE may be reduced in comparison to when the sample SA is discharged using a dropping pipet or provided in multiple layers on the plate PL. As a result, when the sample SA is provided in a single layer, the cell-counting result of the tumor cells CE in the sample SA may become more accurate.

The sample SA provided on the plate PL may be fixated. For example, a sample SA smeared on the plate PL may be fixated on the plate PL. As another example, a sample SA printed on the plate PL may be fixated on the plate PL. As yet another example, a sample SA discharged using a dropping pipet may be fixated on the plate PL.

The sample SA being fixated on the plate PL refers to a state in which a force of resistance has been generated to allow the sample SA to stay on the plate PL until a force of a reference strength is applied to the sample SA. As a result, even when the patch PA and the plate PL are contacted or separated, the sample SA may not be absorbed into the patch PA.

The sample SA may be fixated on the plate PL using any methods that is used in the art related to the present application. For example, a method of providing methanol in the sample SA and volatilizing the methanol may be used to fixate the sample SA on the plate PL.

The cancer diagnosis according to the present application may be performed on a DNA sample extracted from the blood. The extracted DNA may comprise DNA acquired by decomposing a cell membrane of circulating tumor cell CE, which float in the blood.

In relation to this, for extraction of DNA, a lysis patch PA that comprises a lysozyme for decomposing a cell membrane may be used. This will be described in more detail below.

The cancer diagnosis according to the present application may also be performed to detect DNA of tumor cells CE that float in the blood (hereinafter "CtDNA").

The CtDNA may be released from tumor cells CE that have ruptured and died. A portion of CtDNA released to the blood may not be removed by macrophages and, due to its characteristics of proliferating rapidly and having a large size, may stay in blood instead. The CtDNA staying in blood may be used in cancer diagnosis by detecting DNA in blood.

Detecting DNA of tumor cells CE that float in the blood is the same as detecting DNA of tumor cells CE from an extracted DNA sample in that cancer diagnosis is performed using DNA. Therefore, the above-described detecting DNA of tumor cells CE floating in the blood may be performed similarly to the detecting DNA of tumor cells CE from an extracted DNA sample.

Hereinafter, for detecting circulating tumor cell CE, a method in which cancer diagnosis is performed on the blood will be described in detail. In describing it will be assumed but not restricted that the sample SA is provided on the plate PL.

Also, in describing detecting DNA for cancer diagnosis, a method for detecting DNA of circulating tumor cell CE by pre-processing the blood will be described in detail. However, the method for detecting DNA which floats in blood in order to detect the CtDNA will be easily understood through understanding of the method for detecting DNA by pre-processing the blood.

### 5.1.2 Cell filtering

The cancer diagnosis according to the present application may be performed on a sample SA that has gone through filtering. In performing the cancer diagnosis, a filtering procedure may be additionally performed in which sortingis carried out using the size, density, specific marker, or the like of tumor cells CE.

The filtering has an advantage of improving the detection efficiency through sorting out cells that are highly likely to become tumor cells CE considering that circulating tumor cell CE which should be detected in the cancer diagnosis according to the present application are distributed in very small amounts in blood..

However, the above description merely clarifies that the cancer diagnosis according to the present application may be performed on a sample SA that has gone through the filtering procedure if necessary, and does not necessarily mean that the filtering procedure must be performed.

Hereinafter, a patch PA that may be used in performing the cancer diagnosis according to the present application will be described.

### 5.2 Preparation of patch

### 5.2.1 Morphology

For the cancer diagnosis according to the present application, the patch PA may be used in analyzing the morphology of cells CE included in the blood. By contacting it to the plate PL, the patch PA may enable a substance contained in the patch PA to move to the plate PL.

The cancer diagnosis may be performed by analyzing the morphology in relation to sample SA. More specifically, the cancer diagnosis may be performed by identifying the size and the shape of a nucleus of cells CE in the blood.

However, for more accurate morphological diagnosis, the tumor cells CE may be stained.

The patch may contain a staining reagent. Here, the stain may be changed in various ways in accordance with a purpose of a blood test or a staining technique to be performed. Typical examples of the staining reagent may be staining solutions which are used in Romanowsky staining techniques such as acetocarmine, methylene blue, eosin, acid fuchsin, safranin, Janus Green B, hematoxylin, Giemsa solution, Wright solution, and Wright-Giemsa solution, or Leishman staining solution, Gram staining solution, carbol-fuchsin, and Ziehl-Neelsen solution.

Of course, the staining reagent in the present disclosure is not limited to the above-mentioned examples, and various other substances for staining blood may also be used as a staining reagent if necessary. For example, the staining reagent may be 4,6-diamidino-2-phenylindole (DAPI), which exhibits a fluorescence.

The patch PA may contain a single staining reagent. In another embodiment, the patch PA may simultaneously contain two or more types of stains. In relation to this, in a staining technique using multiple types of staining reagents, the patch PA may contain only some of the above multiple types of staining reagents.

The patch PA may contain a washing solution. For example, the washing solution may be a tris buffered saline (TBS) or phosphate buffered saline (PBS) with Tween 20. The patch PA which contains the washing solution may absorb a substance on the plate PL by contacting with and being separated from the plate PL.

The patch PA may contain a buffer solution. The patch PA may perform a function of providing an environment by contacting with the plate, and an environment suitable for the staining may be created in the sample SA located on the plate PL. A solution having an optimal pH for each staining technique may be used as the buffer solution.

### 5.2.2 Immunochemistry

The patch PA may be used in performing immunoassay on blood for cancer diagnosis according to the present application. By contacting it with the plate PL, the patch PA may enable a substance contained in the patch PA to move to the plate PL.

The patch PA may contain an antigen. The patch PA may contain a biopsy specimen (that is, sample SA) that includes an antigen.

The patch PA may contain an antibody AB. For example, an antigen which the antibody AB detects may be an epithelial cell adhesion molecule (EpCAM) or cytokeratin that is known as being distributed on surfaces of the tumor cells CE.

According to an indirect technique, the antibody AB contained in the patch PA may be a primary antibody AB or a secondary antibody AB. The patch PA may contain either the primary antibody AB or the secondary antibody AB or may contain both the primary antibody AB and the secondary antibody AB.

The patch PA may contain a substrate SU. The substrate SU may perform a reaction that is catalyzed by an enzyme, and a substrate SU may be changed in accordance with an enzyme or a detecting means being used. For example, the substrate SU may be 3-ethylbenzothiazoline-6-sulphonic acid (ABTS), 3,3',5,5'-Tetramethylbenzidine (TMB), or the like.

The patch PA may contain a washing solution. As described above, the patch PA which contains the washing solution may absorb a substance on the plate PL by contacting with and then being separated from the plate PL. In this case, the sample SA fixated on the plate PL and some substances bound to the sample SA may not be absorbed.

The patch PA may contain a buffer solution. The patch PA may perform a function of providing an environment by contacting with the plate PL , and an environment suitable for each step of the immunoassay may be created in the sample SA located on the plate PL. As an example of the buffer solution, a peroxide buffer may be used upon chemiluminescence detection.

The patch PA containing the washing solution (hereinafter, "washing patch PA") and the patch PA containing the buffer solution (hereinafter, "buffer patch PA") may be partially similar to a washing patch PA and a buffer patch PA that are used in morphological diagnosis.

### 5.2.3 Culture

A patch PA may be used in performing a method of culturing cells CE for the cancer diagnosis according to the present application. By contacting it with the plate PL, the patch PA may enable a substance contained in the patch PA to move to the plate PL.

The patch PA may contain a nutrient substance NT (hereinafter, "nutrient patch PA"). The nutrient substance NT may be a component required for culturing cells CE. For example, the patch PA may contain amino acids, vitamins, trace elements, and the like. As another example, the patch PA may contain proteins, peptides, hormones, minerals, and the like.

The patch PA may contain a washing solution. As described above, the patch PA which contains the washing solution may absorb a substance on the plate PL by contacting with and then being separated from the plate PL.

The patch PA may contain a buffer solution. The patch PA may perform a function of providing an environment by contacting with the plate PL. In other words, due to contact between the patch PA and the plate PL, an environment suitable for cell culturing may be created in the sample SA located on the plate PL. For example, the patch PA may be used to adjust a pH composition of a sample that is changed as cells CE are cultured.

The patch PA containing the washing solution (hereinafter, "washing patch PA") and the patch PA containing the buffer solution (hereinafter, "buffer patch PA") may be partially similar to a washing patch PA and a buffer patch PA that are used in morphological diagnosis and immunoassay.

### 5.2.4 PCR

A patch PA may be used in performing a PCR method for amplifying DNA for the cancer diagnosis according to the present application. By contacting it with the plate PL, the patch PA may enable a substance contained in the patch PA to move to the plate PL.

The patch PA may contain a lysozyme. The patch PA may enable the lysozyme contained therein to move to the sample SA by contacting with the plate PL, and as a result, a cell membrane of circulating tumor cell CE in the sample SA may be destroyed.

When the cell membrane of the circulating tumor cell CE is destroyed, DNA inside the tumor cells CE may be discharged to the sample. Consequently, the patch PA may enable the DNA of the circulating tumor cell CE to be discharged to the plate PL.

The patch PA may contain a primer. The primer may be produced to correspond to some known DNA sequences of a cancer patient. The primer may move from the patch PA to the plate PL, and in the annealing step, the primer may bind to some sequences of DNA in the sample SA.

The patch PA may contain a deoxynucleoside triphosphate (dNTP). The dNTP may move from the patch PA to the plate PL, and in the extension step, the dNTP may bind to a target DNA of the sample SA.

The patch PA may contain a DNA polymerase. The DNA polymerase may perform a function of enabling the dNTP to bind to the DNA which is bound to the primer. For example, the DNA polymerase may be Taq polymerase.

The patch PA may contain a buffer solution. The patch PA may perform a function of providing an environment by contacting with the plate PL , and an environment suitable for each step of the PCR may be created in the sample SA located on the plate PL.

The patch PA which contains the buffer solution (hereinafter, "buffer patch PA") may be partially similar to a buffer patch PA used in the morphological diagnosis, the immunoassay, and the cell culture diagnosis.

The patch PA may contain a coenzyme. The coenzyme may be changed in accordance with the above DNA polymerase. For example, when the DNA polymerase is Taq polymerase, the coenzyme may be MgCl₂. The patch PA may contact with the plate PL and provide an environment for activating the DNA polymerase.

Hereinafter, a few diagnostic methods that are performed for the cancer diagnosis according to the present application will be described.

### 5.3 Diagnostic methods

### 5.3.1 Morphology

In the cancer diagnosis according to the present application, a morphological diagnosis using a patch PA and a plate PL may be performed.

More specifically, taking into consideration that the shape of the nucleus of tumor cells CE is different from that of normal cells CE, cancer may be diagnosed through the shape of the nucleus of the tumor cells CE. Alternatively, taking into consideration that the size of tumor cells CE is larger than that of normal cells CE, cancer may be diagnosed through an external morphology of the tumor cells CE.

In case of cancer diagnosis in which the morphological analysis is performed, it can be performed by smearing the sample SA, acquiring an image of the sample SA, and analyzing the acquired image of the sample SA. However, for more accurate diagnosis, staining of the sample SA may be performed.

A method of performing the staining of the sample SA will be briefly described below.

The morphological diagnosis using the patch PA and the plate PL may be performed through staining. The sample SA provided on the plate PL may be fixated, and a staining matter may be provided. Providing the staining matter may be performed by a patch PA which contains a staining reagent (hereinafter, "staining patch PA").

When the staining reagent contained in the patch PA moves to the sample SA, a cytoplasm or nucleus of the cells CE in the sample may be stained. Alternatively, when the staining reagent contained in the patch PA moves to the blood, the DNA located inside the cells CE in the sample SA may also be stained.

More specifically, a patch PA that contains a nucleus staining reagent may stain a nucleus of the cells CE in the blood. A basic PH staining reagent is mostly used as the nucleus staining reagent, and typical examples thereof include methylene blue, toluidine blue, hematoxylin, and the like.

A patch PA that contains a cytoplasm staining reagent may stain a cytoplasm of cells CE in blood or an outer structure of the cells CE. An acidic pH staining reagent is mostly used as the cytoplasm staining reagent, and typical examples thereof include eosin, acid fuchsin, orange G, and the like.

Further, the shape of stained cells CE and/or position of a nucleus of the cells CE can be identified by staining DNA inside the cells CE in the blood. For this, the patch PA may contain membrane-permeable DAPI. The DAPI may stain the cells CE by permeating through a cell membrane and binding to DNA distributed in the cell membrane.

By providing a buffer solution to the sample SA after the staining reagent is provided to the sample SA, a suitable environment that is required for staining may be created. Providing the buffer solution may be performed by a buffer patch PA which contains the buffer solution. This may beto improve the efficiency of staining the sample SA.

By providing a washing solution to the sample SA after the staining reagent is provided to the sample SA, remaining staining matter which is not required after staining may be removed. Providing the washing solution may be performed by a washing patch PA which contains the washing solution. This may be to acquire a clearer image of the sample SA.

The morphological diagnosis using stained blood may be performed by various means. For example, the morphological diagnosis may be performed by acquiring an image related to the stained blood and analyzing the acquired image. In the analysis of the image, a process may be performed by counting the number of tumor cells CE danger group in the acquired image and analyzing and sorting the types of the tumor cells CE.

Here, it may be preferable that the plate PL is prepared with a material through which light that is emitted from a light source may permeate as readily as possible. Also, the light source may emit white light or light having a wavelength in a specific band.

### 5.3.2 Immunochemistry

In the cancer diagnosis according to the present application, an immunoassay using a patch PA and a plate PL may be performed. Unless otherwise indicated, "Antibody AB" which will be described below may bind specifically to proteins attached to surfaces of circulating tumor cell CE.

The immunoassay using the patch PA and the plate PL may be performed by a direct technique. A sample SA (or an antigen) provided on the plate PL may be fixated, and antibodies AB that bind specifically to antigens to be detected may be provided. This may be performed by a patch that contains the above-described antibodies AB (hereinafter, "antibody patch PA"). Labels for identification may have been attached to the antibodies AB. After the antibodies AB are provided, a procedure for removing antibodies AB which are not bound to the antigens provided on the plate PL may be performed. This may be performed by the above-described patch PA which contains a washing solution (hereinafter, "washing patch PA").

The immunoassay using the patch PA and the plate PL may be performed by an indirect technique. A sample SA (or antigen) provided on the plate PL may be fixated, and a primary antibody AB that binds specifically to antigens to be detected may be provided. This may be performed by the above-described antibody patch PA. The primary antibody AB may not have a label for identification attached. After the primary antibody AB is provided, a secondary antibody AB that binds specifically to the primary antibody AB may be provided on the plate PL. This may be performed by the above-described antibody patch PA. The second antibody AB may have a label for identification attached. After the secondary antibody AB is provided, a procedure for removing the primary antibody AB which is not bound to the antigens provided on the plate PL and the secondary antibody AB which is not bound to the primary antibody AB may be performed. This may be performed by the above-described washing patch PA.

The immunoassay using the patch PA and the plate PL may be performed by a sandwich technique. Antibodies AB that bind specifically to antigens to be detected may be provided on the plate PL. On the above plate PL on which the antibodies AB are provided a sample SA may be provided. After the sample SA is provided, antibodies AB that bind specifically to the antigens may be provided on the plate PL. This may be performed by the above-described antibody patch PA. After the antibodies AB are provided, a procedure for removing antibodies AB, which are not bound to the antigens provided on the plate PL, may be performed. This may be performed by the above-described patch which contains a washing solution (hereinafter, "washing patch PA"). The antibodies AB mentioned above may have labels for identification attached.

Cancer detection through the immunoassay method may be performed using various means. For example, color development through an enzyme-substrate SU reaction may be detected, fluorescence of a fluorescence label may be detected, or light through a chemical reaction may be detected.

A method of using an enzyme-substrate SU reaction or using a fluorescence label will be described in more detail below as a typical example.

For example, by detecting color development through the enzyme-substrate SU reaction the presence of antigens in the sample SA may be identified. The antibodies AB in the above-described direct technique or the secondary antibody AB in the above-described indirect technique may have enzymes attached as labels. After antibodies AB are provided to the sample SA, a substrate SU may be provided to the sample SA. This may be performed by a patch PA that includes the substrate SU (hereinafter, "substrate patch PA"). The enzymes attached to the antibodies AB mentioned above may catalyze a chemical reaction of the substrate SU and generate a product. Color development may be detected from the sample through the generated product.

When color development is detected by the generated product, by measuring the color development, specific binding between the antibodies AB and the antigens may be quantitatively measured. Measuring of the color development may be performed by detecting light that has been emitted from a light source and has passed through the plate PL. In other words, measuring of the color development may be performed by measuring light absorption. When measuring the color development a spectrophotometer may be used. In this case, the plate PL may be a transparent and flat plate.

As another example, by detecting fluorescence of the fluorescence label the presence of antigens in the sample SA can be identified. The antibodies AB in the above-described direct technique or the secondary antibody AB in the above-described indirect technique may have fluorescent substancesattached as labels.

When the fluorescence detecting is used, fluorescence of the sample SA may be measured in order to quantitatively measure specific binding between the antibodies AB and the antigens. Measuring of the fluorescence may be performed by making light be incident on the plate PL and measuring fluorescence emitted from the plate PL. When measuring the fluorescence a fluorometer to which a filter is attached may be used. Preferably, when measuring the fluorescence an opaque black plate or an opaque white plate may be used as the plate PL.

The above-described color development or fluorescence may be detected through an image. An image of each part may be acquired, and the acquired images may be combined into a single image. From the image positions at which target antigens/antibodies AB are distributed, shape of cells CE, distribution of target proteins, or the like may be identified.

### 5.3.3 Culture

In the cancer diagnosis according to the present application, culturing cells CE may be performed using a patch PA and a plate PL. A nutrient patch PA which will be described below may contain nutritional components required for culturing tumor cells CE.

In culturing of cells CE using the patch PA and the plate PL, the sample SA may be provided on the plate PL, and a nutrient substance NT may be provided to the sample SA.Providing the nutrient substance NT to the sample SA may be performed by the above-described nutrient patch PA.

When a predetermined time has elapsed after the nutrient substance is provided to the sample SA, cells CE in the sample may proliferate.

Analysis on the proliferated cells CE may be performed by various means. For example, an image of a sample SA on which culturing cells CE has been completed for a predetermined amount of time may be acquired, and the image may be analyzed. For another example, an image of a sample SA on which the culturing cells CE is in progress may be acquired, and the image may be analyzed.

The analysis of the image may be performed in consideration of a speed of cell division of the cells CE and a time at which the cell division of the cells CE has ended. In other words, taking into consideration that a speed of cell division of tumor cells CE is faster than that of normal cells CE and the tumor cells CE may proliferate infinitely while division of the normal cells CE ends at an arbitrary point in time, cells CE that continuously divide while dividing at a relatively higher speed may be identified in order to identify whether cancer has developed.

Even in this case, it may be preferable for the plate PL to be prepared with a material through which light emitted from a light source may permeate as readily as possible.

### 5.3.4 PCR

In the cancer diagnosis according to the present application, a PCR may be performed using a patch PA and a plate PL. A primer which will be described below may be produced to correspond to a DNA sequence of a cancer patient.

The PCR using the patch PA and the plate PL may be performed on a sample SA provided on the plate PL. The sample SA may be a DNA sample that has been extracted from the blood. The DNA sample may be heated at the temperature in which two strands of DNA in the DNA sample are separated (hereinafter, "denaturation temperature").

A primer may be provided to the heated DNA sample. Providing of the primer may be performed by a patch PA that contains the primer. The DNA sample to which the primer has been provided may be adjusted to the temperature in which the primer can bind to the DNA (hereinafter, "annealing temperature"). The primer may have a label for identification attached.

After the primer binds to the DNA, a dNTP, a DNA polymerase, a buffer solution, and a coenzyme may be provided to the sample. This may be performed by a patch PA that contains the dNTP, a patch PA that contains the DNA polymerase, a patch PA that contains the buffer solution, and a patch PA that contains the coenzyme. Alternatively, this may be performed by a patch PA that contains at least two or more of the above-mentioned dNTP, DNA polymerase, buffer solution, and coenzyme. When temperature of the sample mentioned above is adjusted to the temperature at which the DNA bounds to the primer may extend (hereinafter, "extension temperature"), the DNA bound to the primer may extend.

The cancer diagnosis through the PCR process may be performed by various means. For example, fluorescence of a fluorescence label may be detected, or electrophoresis may also be used. Also, in accordance with a point in time at which the sample SA is analyzed, detecting a target DNA may be performed from a sample SA on which the PCR process has been completed, or a target DNA may be detected from a sample SA on which the PCR process is in progress.

An identification label attached to the above-described primer may be a fluorescent substance. Also, the fluorescent substance may be a substance that exhibits fluorescence until fluorescence is consumed or a substance that is designed to exhibit fluorescence when the primer and the DNA are bound.

Since the detection means using the fluorescence label has already been described in detail above in relation to the immunoassay, detailed description thereof will be omitted.

As described above, cancer diagnosis according to an embodiment of the present application may be performed through the morphological diagnosis, the immunoassay, the culture progress diagnosis, and the DNA diagnosis (that is, PCR testing).

Hereinafter, to assist in understanding the present application, a method of performing cancer diagnosis using a patch PA will be described in detail for each diagnostic method using a few embodiments.

### 6. Embodiments related to diagnostic processes using patch

### 6.1 Morphology

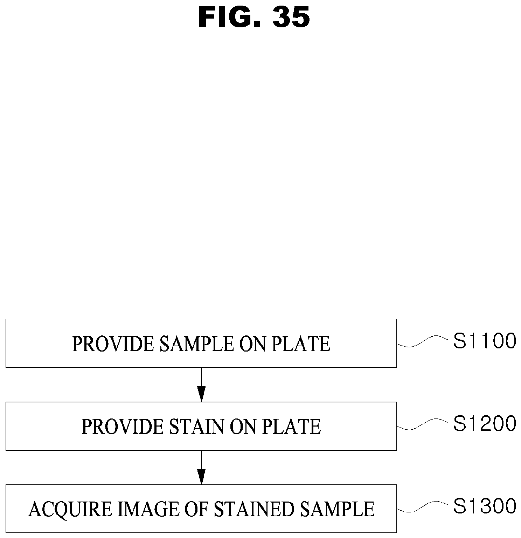

FIG. 35 is a view for describing performance of morphological diagnosis using a staining technique in cancer diagnosis according to an embodiment of the present application. The morphological diagnosis according to an embodiment of the present application may be performed through providing a sample SA on a plate PL (S1100), providing a staining reagent on the plate PL (S1200), and acquiring an image of the stained sample SA (S1300). This may correspond to performing morphological diagnosis using a simple staining technique.

In the morphological diagnosis for the cancer diagnosis according to the present application, as described above, a nucleus and/or a cytoplasm of tumor cells CE may be stained.

The providing the sample SA on the plate PL (S1100) may refer to placing the sample SA on the plate PL. For example, the sample SA may be smeared or printed on the plate PL. Also, the sample SA may be fixated on the plate PL. The sample SA may have a predetermined force of resistance and be fixated on the plate PL so that absorption of the sample SA into a patch PA due to contact or separation between the patch PA and the plate PL is prevented.

A staining patch PA may be used for the providing the staining reagent on the plate PL (S 1200). A single staining reagent may be contained in the patch PA.

Due to contact between the patch PA and the plate PL, a staining reagent that has been contained in the patch PA may move to the plate PL. To provide a staining reagent on the plate PL, the patch PA and the plate PL may be brought into contact. Also, the patch PA and the plate PL may be separated after having been in contact.

In the present step, to implement a predetermined environment in the sample SA, the buffer patch PA may also be used as necessary.

Also, in the present step, washing may be performed to remove a non-required staining reagent provided in the sample SA. The washing may be performed by the washing patch PA which contains a washing solution.

After the providing the staining reagent, the acquiring of the image of the stained sample SA located on the plate PL (S1300) may be performed. When an image of the sample SA is acquired, analysis may be performed on the image.

By analyzing the image, the sizes and morphologies of cells CE included in the blood may be compared. Since the size of tumor cells CE is larger than that of normal cells CE and the morphology of the tumor cells CE is different from that of the normal cells CE, the tumor cells CE may be detected through the image analysis, and the number of tumor cells CE may be counted.

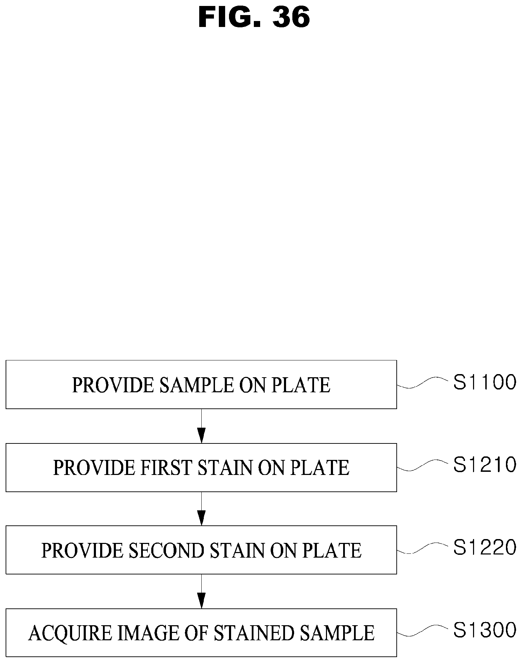

FIG. 36 is a view for describing performance of morphological diagnosis using a staining technique in which a plurality of staining reagents are used in cancer diagnosis according to an embodiment of the present application.

In the providing the staining reagent on the plate PL (S 1200), at least two or more stains may be delivered. In this case, to deliver at least two or more stains to the sample SA, a plurality of staining patches PA that each contain one of the plurality of staining reagents may be used.

However, hereinafter, for convenience of description, description will be given which assumes that two staining patches PA, i.e., a first staining patch PA and a second staining patch PA, are used to stain a sample using two staining reagents.

However, the number of staining patches PA is not limited to two in the present embodiment, and three or more staining patches PA may also be used. Since those of ordinary skill in the art may easily understand that, without inventive thinking, a modified example in which three or more staining patches PA are used is applicable to the following description, such a modified example should be understood as belonging to the present embodiment.

The providing the staining reagent on the plate PL (S 1200) may include providing a first staining reagent on the plate PL (S1210) and providing a second staining reagent on the plate PL (S 1220).

Here, the first staining reagent and the second staining reagent may stain different target substances in the blood. For example, the first staining reagent may be any one of a basic staining reagent that stains a nucleus and an acidic staining reagent that stains a cytoplasm, and the second staining reagent may be the other of the two. Alternatively, the opposite may be possible. Specifically, the first staining reagent may be methylene blue, and the second staining reagent may be eosin. Of course, it should be noted that the types of the first staining reagent and the second staining reagent are not limited to the above-given examples.

When the first staining patch PA is brought into contact with the plate PL, the first staining reagent may move to the plate PL. Therefore, the staining patch PA that contains the first staining reagent may stain a first target substance included in the sample SA. When the second staining patch PA is contacted with the plate PL, the second staining reagent may move to the plate PL. Therefore, the staining patch PA that contains the second staining reagent may stain a second target substance included in the sample SA. Here, the first target substance may be any one of a nucleus and a cytoplasm of cells CE, and the second target substance may be the other of the two.

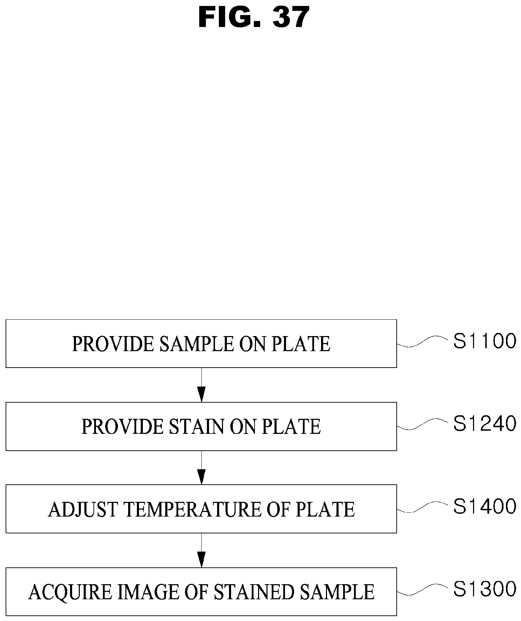

FIG. 37 is a view for describing performance of morphological diagnosis using a DAPI staining technique in cancer diagnosis according to an embodiment of the present application.

In providing a staining reagent on the plate PL (S 1240), a fluorescence staining reagent may be provided on the plate PL. For example, the fluorescence staining reagent may be DAPI.

A patch PA that contains the staining reagent may be contacted with the plate PL, and due to contact between the patch PA and the plate PL, the staining reagent contained in the patch PA may move to the plate PL.

The staining reagent may permeate through a cell membrane and be introduced into the cells CE. Although the efficiency of staining using DAPI may be further improved when the cell membrane is destroyed, the staining reagent may be introduced into the cells CE even when the cell membrane is present in the case of the DAPI staining technique.

A pH value of the sample SA may be adjusted as necessary. The pH of the sample SA may be adjusted by a buffer patch PA that contains a buffer solution.

Also, temperature of the sample SA may be adjusted. Preferably, after the staining reagent is provided to the sample SA, the temperature of the sample SA may be maintained at approximately 37 °C for about 15 minutes so that a sufficient reaction time is provided to the sample SA.

The acquiring of the image of the stained sample SA (S1300) may be performed. However, in the present embodiment, taking into consideration that a staining reagent being used is a fluorescence reagent, a fluorescence image may be acquired. Therefore, light in an arbitrary wavelength band may have to be provided to the sample SA for acquiring the fluorescence image.

Since the fluorescence image using the DAPI staining technique has a high resolution, there may be an advantage in that a more accurate cancer diagnosis is possible.

### 6.2. Immunochemistry

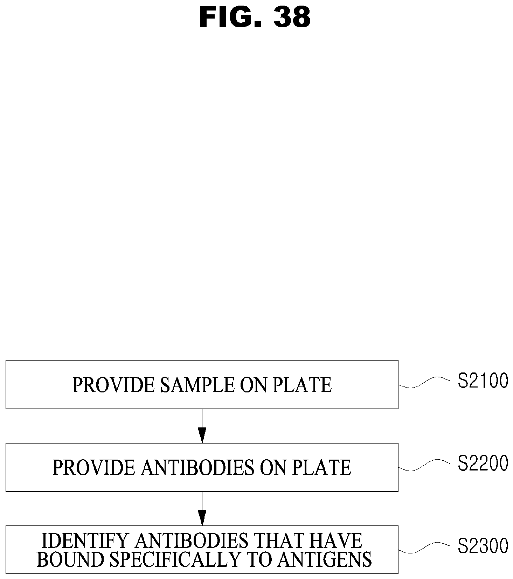

FIG. 38 is a view for describing performance of immunoassay in cancer diagnosis according to an embodiment of the present application. An immunoassay method according to an embodiment of the present application may be performed through providing a sample SA on a plate PL (S2100), providing antibodies AB on the plate PL (S2200), and identifying antibodies AB that have bound specifically to antigens (S2300). This may correspond to performing immunoassay through a direct technique.

In performing immunoassay for the cancer diagnosis according to the present application, as described above, antibodies AB that bind specifically to the EpCAM or cytokeratin may be used.

The providing the sample SA on the plate PL (S2100) may be performed similarly to the above-described Step S1100.

An antibody patch PA may be used to provide the antibodies AB on the plate PL (S2200). Due to contact between the patch PA and the plate PL, the antibodies AB contained in the patch PA may move to the plate PL. The antibodies AB may bind specifically to antigens included in the sample SA provided on the plate PL.

When the antibodies AB are provided on the plate PL, the identifying of the antibodies AB that have bound specifically to the antigens (S2300) may be performed. Prior to the present step, a washing patch PA may be used as necessary to remove antibodies AB that failed to bind to the sample SA.

The antibodies AB that have bound specifically to the antigens may be identified through various methods.

For example, a substrate SU-enzyme reaction may be used to identify the antibodies AB that have bound specifically to the antigens.

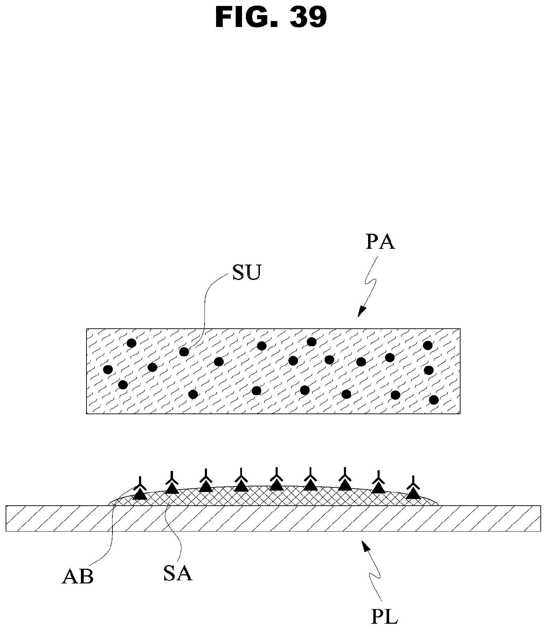

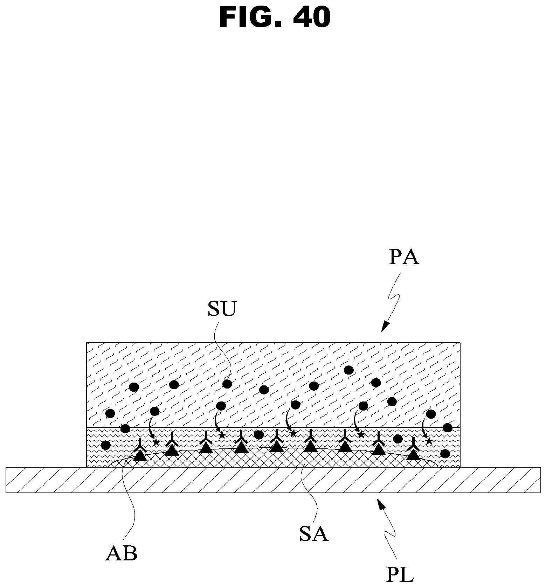

FIGS. 39 and 40 are views for describing a method of detecting cancer using a substrate SU-enzyme reaction when immunoassay is performed in cancer diagnosis according to an embodiment of the present application.

When the antibodies AB are provided by the patch PA, an enzyme may be bound to the antibodies AB. Labels bound to the antibodies AB may be enzymes. The sample SA, to which the antibodies AB are provided by the patch PA, may be bound to some of the antibodies AB provided by the patch PA.

A substrate patch PA may be provided to the sample SA. When the substrate patch PA is provided to the sample SA, the enzymes attached to the antibodies AB, which are bound to the sample, may react with the substrate SU. More specifically, a product may be generated due to a reaction that is catalyzed by the substrate. The presence of circulating tumor cell CE in the sample SA may be identified based on whether the product is generated.

In this case, the substrate patch PA may perform a function of providing a reaction space in which the enzyme and the substrate SU may react. This has been described in detail above in the description of functions of the patch PA.

As another example, a fluorescent substance may be used to identify antibodies AB that have bound specifically to the antigens.

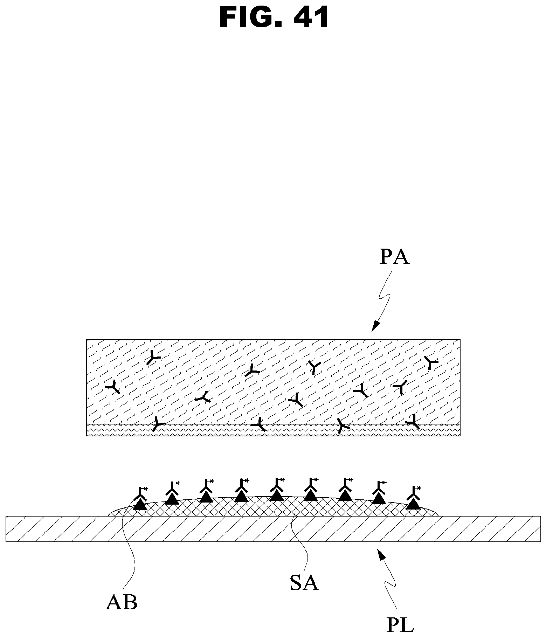

FIG. 41 is a view for describing a method of detecting cancer using a fluorescent substance when immunoassay is performed in cancer diagnosis according to an embodiment of the present application.

When antibodies AB are provided by a patch PA, a fluorescent substance may be bound to the antibodies AB. Labels bound to the antibodies AB may be a fluorescent substance. The sample SA to which the antibodies AB are provided by the patch PA may be bound to some of the antibodies AB provided by the patch PA.

The contact between the patch PA and the plate PL may be released. The contact may be released to prevent fluorescence development by antibodies AB contained in the patch PA (that is, antibodies AB that have not yet bound to the sample SA).

After the contact between the patch PA and the plate PL is released, the presence of tumor cells CE in the sample SA may be identified in accordance with whether light is emitted from the fluorescent substance attached to the antibodies AB which are bound to the sample SA.

When the above-described fluorescent substance is used to identify the antibodies AB that bind specifically to the antigens, a fluorescence image of the sample SA may be acquired to identify positions at which the antibodies AB are attached. The positions at which the antibodies AB are attached may be analyzed to count tumor cells CE distributed in the blood.

A plurality of images of the sample SA may be acquired with time. Diagnosis on a plurality of targets may be performed through a comparison between changes in a previously-acquired image and a subsequently-acquired image from among the plurality of images.

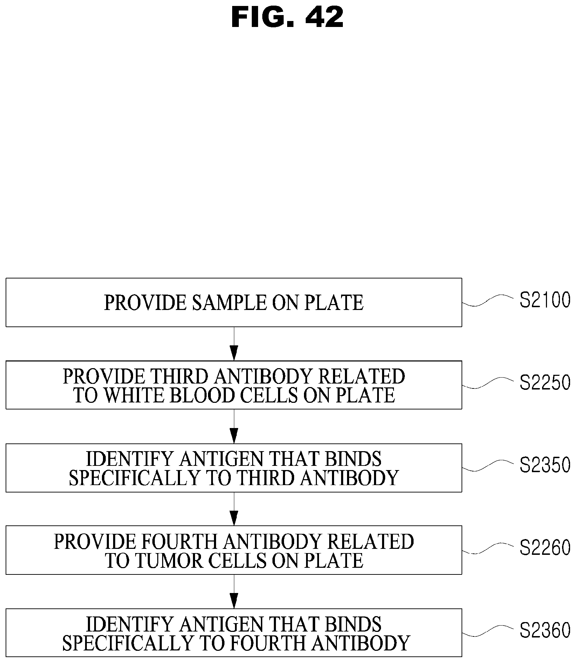

FIG. 42 is a view for describing a method of performing immunoassay using multiple types of antibodies AB in cancer diagnosis according to an embodiment of the present application.

For example, when the sample SA is provided on the plate PL (S2100), a third antibody AB that binds specifically to white blood cells (for example, CD45) may be provided to the sample SA (S2250). The providing the third antibody AB may be performed by a patch PA that contains the third antibody AB.

After the third antibody AB is provided to the sample SA, antigens that bind specifically to the third antibody AB may be identified (S2350). That is, after the providing the third antibody AB, an image of the sample SA may be acquired, and the image may be analyzed to count the number of white blood cells included in the sample SA.

A fourth antibody AB related to tumor cells CE may be provided on the plate PL (S2260). The providing the fourth antibody AB may be performed by a patch PA that contains the fourth antibody AB.

After the fourth antibody AB is provided to the sample SA, antigens that bind specifically to the fourth antibody AB may be identified (S2360). That is, after the providing the fourth antibody AB, an image of the sample SA may be acquired, and the image may be analyzed. In relation to the image, by identifying a position at which fluorescence is displayed in a currently-acquired image and then performing analysis in consideration of a position at which fluorescence is displayed in a previously-acquired image, the number of tumor cells CE included in the sample SA may be counted.

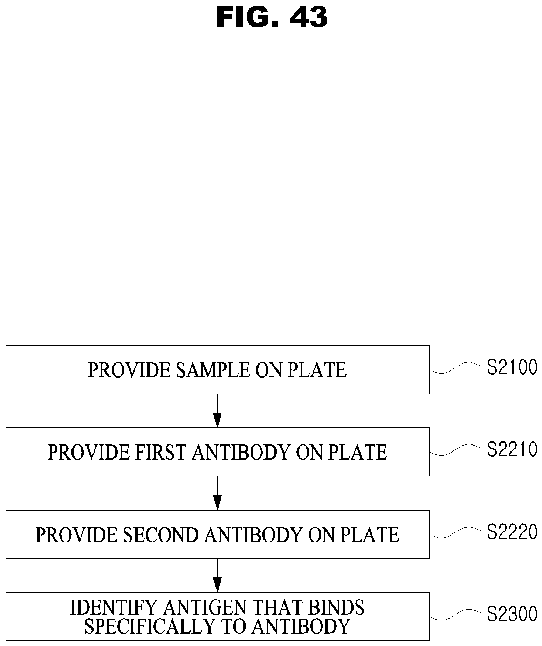

FIG. 43 is a view for describing a method of performing immunoassay using a primary antibody AB and a secondary antibody AB in cancer diagnosis according to an embodiment of the present application. This may correspond to performing immunoassay through an indirect technique.

In a modified example of the above-described embodiment, the providing the antibodies AB on the plate PL (S2200) may include providing a first antibody AB on the plate PL (S2210) and providing a second antibody AB on the plate PL (S2220).

The first antibody AB may be an antibody AB that binds specifically to tumor cells CE. The second antibody AB may be an antibody AB that binds specifically to the first antibody AB. In this case, the specific binding between the first antibody AB and the second antibody AB may be species-specific binding instead of epitope-specific binding. The immunoassay method using the first antibody AB and the second antibody AB may be used for convenience of producing a specific antibody AB.

The first antibody AB may be provided by a patch PA that contains the first antibody AB. The second antibody AB may be provided by a patch PA that contains the second antibody AB. Alternatively, the first antibody AB and the second antibody AB may be provided by a patch PA that contains both the first antibody AB and the second antibody AB.

To provide the first antibody AB on the plate PL, a patch PA which contains the first antibody AB may be brought into contact with the plate PL. To provide the second antibody AB on the plate PL, a patch PA which contains the second antibody AB may be contacted with the plate PL.

Alternatively, to provide the first antibody AB and the second antibody AB on the plate PL, the patch PA that contains both the first antibody AB and the second antibody AB may be contacted with the plate PL. To provide the first antibody AB and the second antibody AB on the plate PL, the patch PA which contains the first antibody AB may be contacted with the plate PL, and the patch PA which contains the second antibody AB may be contacted with the first antibody AB.

After the providing the antibodies AB on the plate PL, a washing patch PA may be used as necessary to remove antibodies AB that have been provided by the patch PA but have not bound to the sample SA.

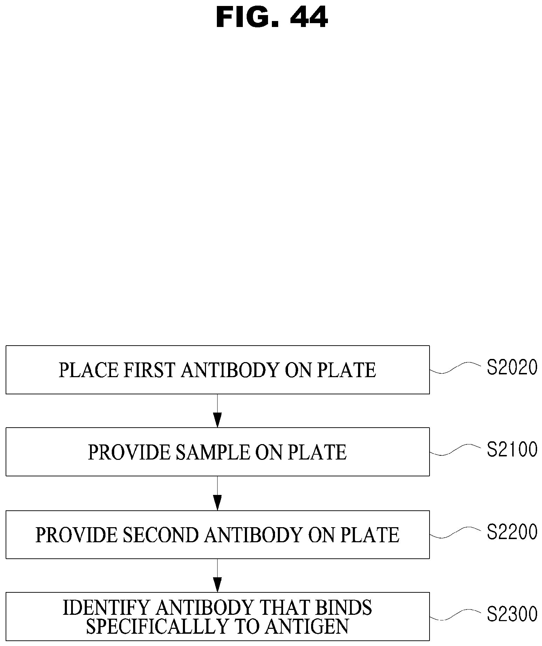

FIG. 44 is a view for describing a method of performing immunoassay using a first antibody AB that has been applied on a plate PL and second antibody AB that is provided to a sample SA in cancer diagnosis according to an embodiment of the present application. This corresponds to performing immunoassay through a sandwich technique.

By a conventional method, a first antibody AB may be placed on the plate PL (S2020). For example, a method in which the first antibody is applied on the plate and then frozen and dried may be applied. The first antibody AB may be an antibody AB that binds specifically to tumor cells CE.

The first antibody AB may be fixated on the plate PL. The fixating of the first antibody AB may be performed through a method of drying the antibody AB or using a coating buffer solution. Alternatively, the fixating of the antibody AB on the plate PL may include forming a thin film on the plate PL. The thin film may be manufactured in advance to be attached to the plate PL.

The sample SA may be provided on the plate PL on which the first antibody AB is located (S2100). The providing the sample SA on the plate PL may include applying the blood. The applying of the blood may include applying the blood in a thin layer in consideration to a reaction area with the antibody AB fixated on the plate PL. Preferably, the blood may be applied in a single layer.

A second antibody AB may be provided on the plate PL (S2200). The second antibody AB may be an antibody AB that binds specifically to white blood cells. Alternatively, the second antibody AB may be an antibody AB that binds specifically to tumor cells CE. In this case, the second antibody AB may be different from the first antibody AB.

The second antibody AB may be provided by the above-described direct technique or indirect technique. In other words, the providing the second antibody AB on the plate PL may include providing an antibody AB that binds specifically to antigens which are to be detected, or may include providing a primary antibody AB that bind specifically to antigens which are to be detected and a second antibody AB that binds specifically to the primary antibody AB.

The above-described first antibody AB and/or second antibody AB may be provided by a patch PA. In this case, an immunoassay method may be applied in accordance with the above-described direct technique or indirect technique.

After the providing the sample SA on the plate PL and/or the providing the antibodies AB on the plate PL which have been described above, a washing patch PA may be used as necessary to remove antibodies AB that have been provided by the patch PA but have not bound to the sample SA.

### 6.3 Culture

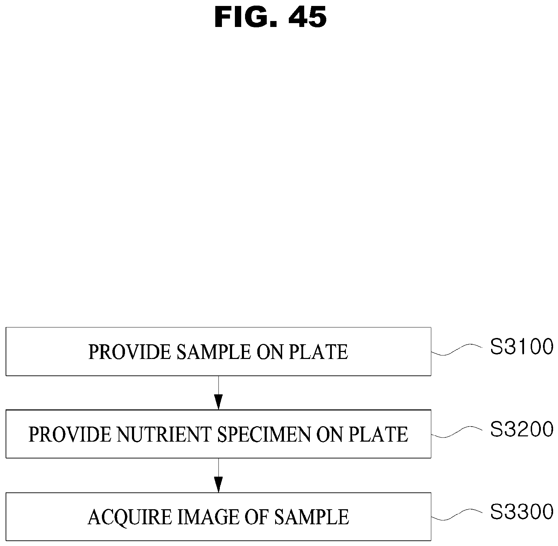

FIG. 45 is a view for describing performance of culture of cells CE in cancer diagnosis according to an embodiment of the present application.

A process of culturing cells CE according to an embodiment of the present application may include providing a sample SA on a plate PL (S3100), providing a nutrient substance NT on the plate (S3200), and acquiring an image of the sample SA (S3300). This may be intended to detect circulating tumor cell CE included in the sample SA.

The providing the sample SA on the plate PL (S3100) may be performed similarly to the above-described Step S1100.

The nutrient substance NT may be provided to the sample SA located on the plate PL (S3200). The nutrient substance NT may be provided by a patch PA that contains at least some reagents that are used in culturing tumor cells CE.

As described above, the patch PA may contain amino acids, vitamins, trace elements, and the like. Alternatively, as another example, the patch PA may contain proteins, peptides, hormones, minerals, and the like.

Due to contact between the patch PA and the plate PL, the nutrient substance NT contained in the patch PA may move to the plate PL. When the nutrient substance NT is provided, cells CE included in the sample SA which is located on the plate PL may proliferate.

In the present step, a washing process may be performed on the plate PL as necessary. This may be a procedure performed by the above-described washing patch PA.

For analysis of the proliferated cells CE, an image of the sample SA may be acquired (S3300). As described above, an image of the sample SA on which culturing of cells CE has been completed or an image of the sample SA on which culturing of cells CE is in progress may be acquired.

A plurality of images of the cells CE may be acquired. An image of the sample SA may be acquired once prior to the start of culturing of cells CE and may be acquired at least one or more times at a desired point in time. In image analysis, the reason for acquiring an image prior to the start of culturing of cells CE may be to estimate a proliferation rate of cells CE using the image prior to cell culturing.

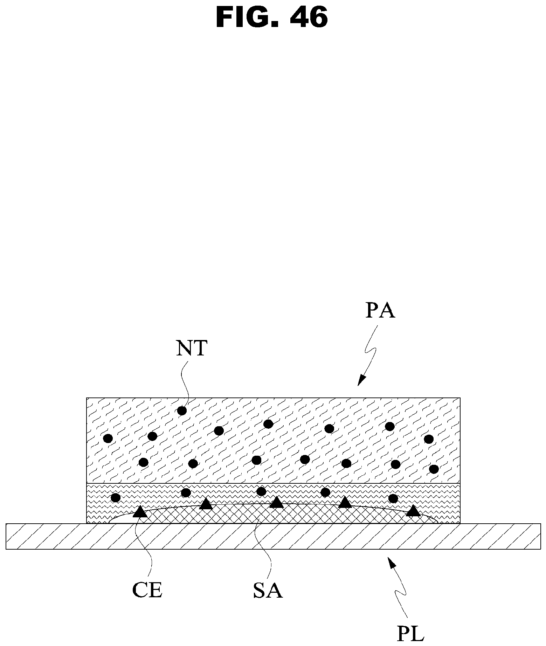

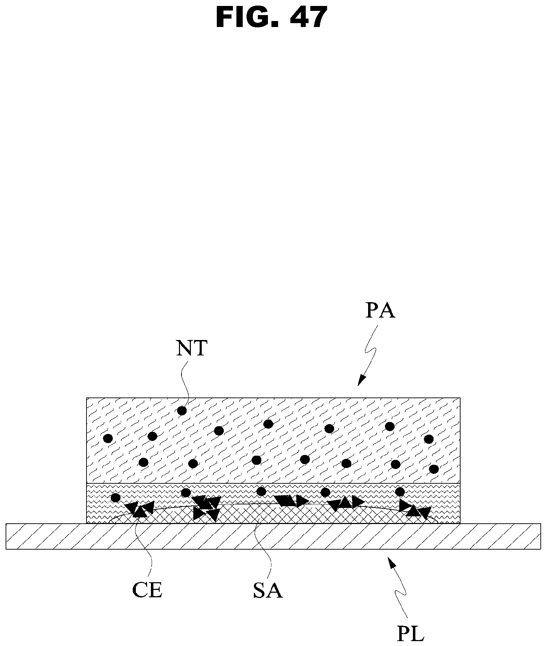

FIGS. 46 and 47 are views for describing a process in which cells CE included in a sample proliferate in cancer diagnosis according to an embodiment of the present application.

Referring to FIG. 46, in culturing of cells CE according to an embodiment of the present application, a sample SA may be applied on a plate PL. Some cells CE may be included in the applied sample SA. Due to contact between the nutrient patch PA and the plate PL, the nutrient substance NT that has been contained in the nutrient patch PA may move to the plate PL.

When the nutrient substance NT that has been contained in the nutrient patch PA is provided to the sample SA applied on the plate PL, the cells CE included in the sample SA may proliferate.

As illustrated in FIG. 47, proliferation speeds of some cells CE may be different. A speed of cell division of tumor cells CE may be higher than that of normal cells CE. On the basis of such a comparison, the tumor cells CE included in the sample SA may be detected. This may be a means in which cells which are dividing more are presumed to be tumor cells CE.

### 6.4 PCR

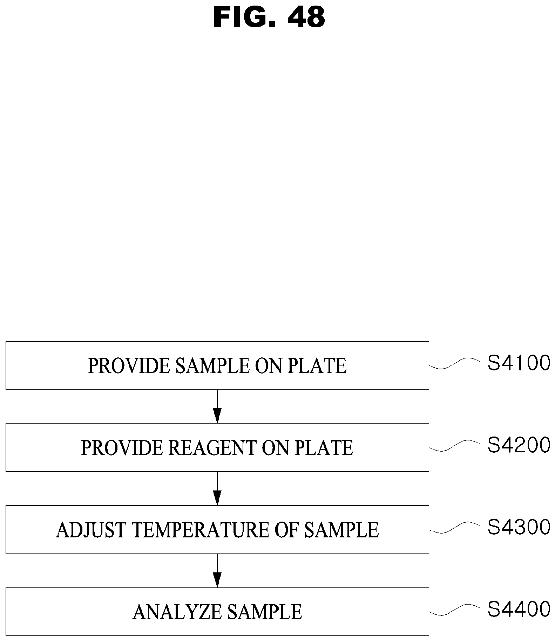

FIG. 48 is a view for describing performance of a PCR process in cancer diagnosis according to an embodiment of the present application. The PCR process according to an embodiment of the present disclosure may include providing a sample SA on a plate PL (S4100), providing reagents on the plate PL (S4200), adjusting a temperature of the sample SA (S4300), and analyzing the sample SA (S4400). This may be intended to detect DNA of circulating tumor cell CE included in the sample SA.

The providing the sample SA on the plate PL (S4100) may be performed similarly to the above-described Step S1100. However, a sample SA on which a pre-processing process for extracting DNA from the blood has been completed may be used.

Reagents may be provided on the plate PL on which the sample SA has been provided (S4200). The reagents may be provided by a patch PA that contains at least some of a plurality of reagents used in the PCR process.

The patch PA may be a patch PA that contains a dNTP, a patch PA that contains a primer, or a patch PA that contains a DNA polymerase. Also, the patch PA may be a patch PA that contains a buffer solution or a patch PA that contains a coenzyme. Alternatively, the patch PA may be a patch PA that contains two or more of the above-mentioned dNTP, primer, DNA polymerase, buffer solution, and coenzyme.

The patch PA and the plate PL may be contacted so that the reagents included in the patch PA are provided on the plate PL. Due to the contact between the patch PA and the plate PL, the reagents contained in the patch PA may move to the plate PL.

When a plurality of patches PA are used to provide reagents on the plate PL, the plurality of patches PA may sequentially be brought into contact with the plate PL, or one patch PA from among the plurality of patches PA may be brought into contact with the plate PL and then a patch PA different from the one patch PA may be contacted with the one patch PA.

When reagents are provided on the plate PL, the temperature of the sample SA may be adjusted (S4300). The temperature of the sample SA may be adjusted by increasing or decreasing the temperature of the plate PL or maintaining the temperature of the plate PL at a desired temperature. The temperature of the sample SA may be adjusted by increasing or decreasing the temperature of the patch PA or maintaining the temperature of the patch PA at the desired temperature. The temperature of the sample SA may be adjusted by increasing or decreasing the temperatures of the patch PA and the plate PL or maintaining the temperatures of the patch PA and the plate PL at desired temperatures.

The temperature of the sample SA may be adjusted to the above-described denaturation temperature, annealing temperature, and extension temperature. Due to adjustment of the temperature of the sample SA, the double helix structure of the DNA included in the sample SA may be separated, a primer may bind to the separated DNA, and a dNTP may bind to the DNA bound with the primer so that the DNA may extend.

When the temperature of the sample SA is adjusted (S4300), the sample SA may be analyzed (S4400). Here, the analyzing of the sample SA may include detecting a fluorescent substance attached to the primer. Alternatively, the analyzing of the sample SA may include acquiring an image of the sample SA.

The analysis may be performed after the PCR process on the sample SA is completed. Due to performing analysis in a state in which DNA amplification on the sample SA has been completed, more accurate data may be acquired.

The analysis may be performed in the middle of the PCR process on the sample SA. More specifically, the analysis on the sample SA may be continuously performed while the PCR process is performed, or, on the basis of one cycle of the PCR process (for example, the denaturation step, the annealing step, and the extension step are sequentially performed), the analysis may be repeatedly performed every cycle at an arbitrary point in time. This form of analysis compares the amounts of amplified DNA with the amount of time that has passed and enables quantitative analysis on target DNA in real time.

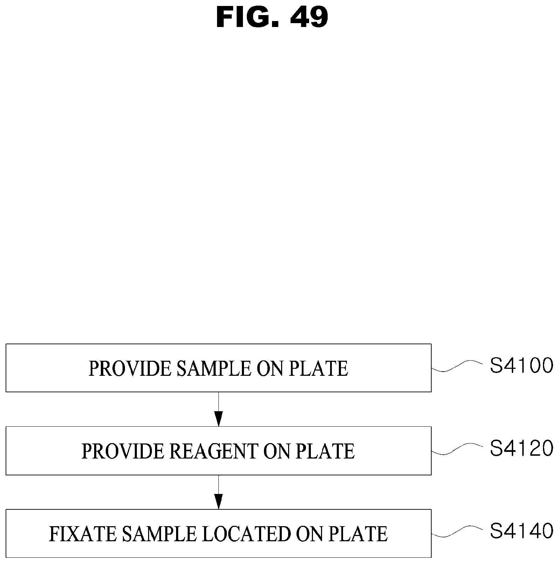

FIG. 49 is a view for describing a method of using a patch PA to decompose a cell membrane of cells CE included in a sample SA when a PCR process is performed in cancer diagnosis according to an embodiment of the present application.

In a modified example of the above-described embodiment, providing reagents on the plate PL (S4120) and fixating the sample SA located on the plate PL (S4140) may be additionally performed after the providing the sample SA on the plate PL (S4100) described above.

After the sample SA is provided on the plate PL (S4100), reagents may be provided on the plate PL (S4120). The sample SA may be blood that has not gone through pre-processing. The reagent may be a substance for breaking down cell membrane of circulating tumor cell that float in the blood. For example, the reagent may be a lysozyme for destroying the cell membrane of the cells CE. The reagent may be provided by the patch PA. The reagent may be contained in the patch PA.

After the reagents are provided on the plate PL (S4120), the sample SA located on the plate PL may be fixated (S4140). The sample SA located on the plate PL may be the blood and DNA of cells CE included in the blood. The sample SA may be in a state in which a portion of a cell membrane of the cells CE is floating.

After the sample SA is fixated on the plate PL (S4140), the PCR process may be performed on the sample SA. The PCR process to be performed afterwards may be performed using any method of PCR process such as the method disclosed herein and a method generally performed by those of ordinary skill in the art.

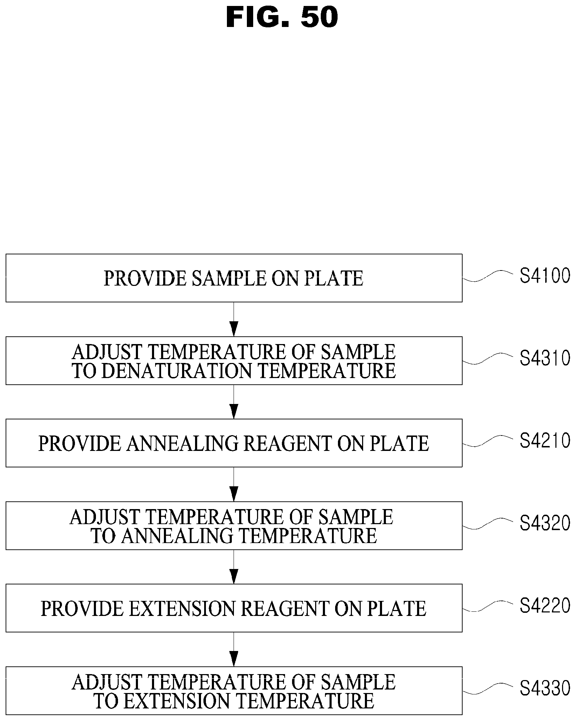

FIG. 50 is a view for describing performance of a PCR process in cancer diagnosis according to an embodiment of the present application. In the present embodiment, description will be given which assumes that reagents are provided on the plate PL by a plurality of patches PA.

When the sample SA is provided on the plate PL (S4100), the temperature of the sample SA may be adjusted to the denaturation temperature (S4310). As described above, the temperature of the sample SA may be adjusted by adjusting a temperature of the plate PL and/or temperature of the patch PA. When the temperature of the sample SA is maintained at the denaturation temperature for a predetermined amount of time, the double helix structure of the DNA included in the sample SA may be separated.

Annealing reagents may be provided on the plate PL (S4310). Here, the annealing reagents may refer to at least some of reagents used in the annealing step. For example, the annealing reagents may be primers.

The annealing reagents may be provided by a patch PA. Due to contact between the patch PA and the plate PL, the reagents contained in the patch PA may be movable to the plate PL, and accordingly, the reagents may be provided on the plate PL by the patch PA.

The patch PA may contain annealing reagents. For example, the patch PA may contain primers. In this case, the primers may be produced to correspond to sequences commonly included in DNA of cancer patients.

When the annealing reagents are provided on the plate PL (S4310), the reagents may also be provided to the sample SA located on the plate PL.

The temperature of the sample SA may be adjusted to the annealing temperature (S4320). To adjust the temperature of the sample SA to the annealing temperature, as described above, the temperatures of the plate PL and/or the patch PA may be adjusted.

A target that is subject to temperature adjustment to adjust the temperature of the sample SA to the annealing temperature may be different from a target that is subject to temperature adjustment to adjust the temperature of the sample SA to the extension temperature. For example, even when the plate PL has been heated to adjust the temperature of the sample SA to the denaturation temperature, the patch PA may be cooled and brought into contact with the plate PL in order to adjust the temperature of the sample SA to the annealing temperature.

When the temperature of the sample SA is adjusted to the annealing temperature (S4320), the DNA included in the sample SA may react with the annealing reagents. For example, the DNA included in the sample SA may bind to the primers.

After the temperature of the sample SA has been maintained at the annealing temperature for a predetermined amount of time, extension reagents may be provided on the plate PL (S4220). Here, the extension reagents may refer to some of reagents used in the extension step. For example, the annealing reagents may be a dNTP, a DNA polymerase, a buffer solution, and a coenzyme.

The extension reagents may be provided by the patch PA.

For example, due to contact between the patch PA and the plate PL, the reagents contained in the patch PA may be movable to the plate PL, and accordingly, the reagents may be provided on the plate PL by the patch PA.

As another example, the patch PA may be brought into contact with another patch PA that is already in contact with the plate PL so that the reagents contained in the patch PA are movable to the other patch PA. Accordingly, the reagents may be provided to the plate PL in contact with the other patch PA.

The patch PA may contain the extension reagents. For example, the patch PA may contain a dNTP, a DNA polymerase, a buffer solution, and a coenzyme.

The temperature of the sample SA may be adjusted to the extension temperature (S4330). The temperature of the sample SA may be adjusted by adjusting temperatures of the patch PA and/or the plate PL. When the temperature of the sample SA is adjusted to the extension temperature, the sample SA may react with the extension reagents. For example, the DNA bound to the primers may extend.

### 7. Multi-test

### 7.1 Meaning

Cancer is an incurable disease of which complete treatment has not yet been developed. Survival rates greatly differ between early cancer, middle cancer, and terminal cancer. Therefore, accurate diagnosis of whether cancer has developed is an important factor in cancer diagnosis.

In relation to this, in cancer diagnosis according to the present application, for accuracy of diagnosis, multiple diagnoses may be performed on a single sample SA.

An embodiment in which multiple diagnostic processes are performed on a sample SA and advantageous effects thereof will be described in detail below.

### 7.2. Embodiment of multi-test

### 7.2.1 First embodiment

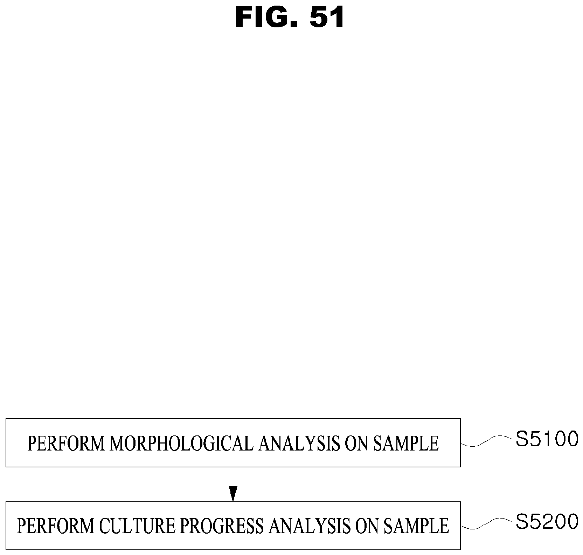

FIG. 51 is a view for describing multiple types of diagnostic methods for a sample SA in cancer diagnosis according to an embodiment of the present application.

The cancer diagnosis according to an embodiment of the present application may be performed using a method which includes performing morphological analysis of a sample SA (S5100) and analyzing culture progress of the sample SA (S5200).

The above-described embodiments related to morphological analysis may be applied to the morphological analysis on the sample SA.

The above-described embodiments related to culture progress analysis may be applied to the culture progress analysis on the sample SA.

The performing of the morphological analysis on the sample SA refers to analyzing the morphology of cells CE included in the sample SA. By analyzing the morphology of the cells CE included in the sample SA, cells CE presumed to be tumor cells CE may be identified.

Although staining may be performed on the sample SA as necessary, description will be given which assumes that an image of an unstained sample SA is acquired in the morphological analysis of the present embodiment.

The performing of the culture progress analysis after the morphological analysis on the sample SA refers to culturing the sample SA on which the morphological analysis has been performed.

In the culture analysis, cells CE suspected as tumor cells CE may be identified by culturing the sample SA. In this case, locations of cells CE confirmed, through the morphological analysis of the sample SA, as cells highly likely to become tumor cells CE may be identified, and the culture progress of the cells CE at the corresponding locations may be identified.

The culture progress analysis on the sample SA may include identifying whether cells CE included in the sample SA continuously divide. In other words, on the basis of the above-mentioned characteristic of tumor cells CE that the tumor cells CE proliferate infinitely, cells CE which divide continuously may be detected as tumor cells CE.

The method of cancer diagnosis according to the present embodiment will be described in more detail below.

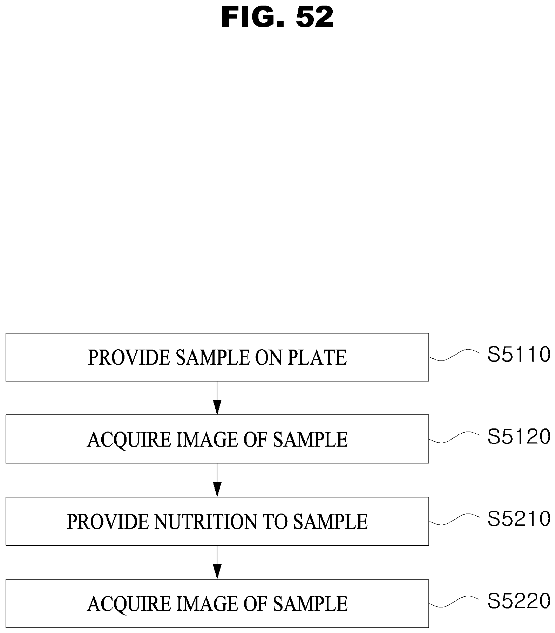

FIG. 52 is a view for describing multiple types of diagnostic methods for a sample SA in cancer diagnosis according to an embodiment of the present application.

When a sample SA is provided on the plate PL (S5110), an image of the sample SA may be acquired (S5120).

Step S5110 may be performed similarly to the above-described Step S1100. Step S5120 may be similar to the above-described Step S1300.

As described above, the sample SA may be applied in a single layer on the plate PL. The sample SA may not have been stained. Although this is not essential, this may be intended to prevent culturing of a sample SA on which an unnecessary specimen (e.g., a stain) is provided in the culture progress analysis which will be performed afterwards.

Also, as necessary, the sample SA provided on the plate may not be fixated on the plate in the present embodiment. This may be intended to prevent destruction of membranes of cells CE in the fixating process.

The image of the sample SA may be analyzed. Through the image of the sample SA, cells CE presumed to be circulating tumor cell CE may be identified, and precise analysis may be attempted on the corresponding cells CE.

The precise analysis may be performed using a method in which the culture progress analysis is performed on the corresponding cells CE.

Nutrition may be provided to the sample SA (S5210). The nutrition may be provided due to contact between the patch PA and the sample SA. As described above, the patch PA may be a nutrient patch PA which contains a nutrient substance NT.

When the nutrition is provided to the sample SA, an image of the sample SA may be acquired (S5220).

Step S5210 may be performed similarly to the above-described Step S3200. Step S5220 may be performed similarly to the above-described Step S3300.

The image of the sample SA may be acquired in real time while the nutrition is being provided to the sample SA. In this case, the image acquired in real time has an advantage of allowing instantaneous changes of the cells CE to be observed.

Alternatively, an image of the sample SA may be acquired after an arbitrary amount of time has elapsed after the providing nutrition to the sample SA. The image of the sample SA may be compared with an image for the above-described morphological analysis. For example, by analyzing changes in sizes of specific cells CE and a relative difference in culture speeds of the specific cells CE and other cells CE, tumor cells CE included in the sample SA may be detected.

### 7.2.2 Second embodiment

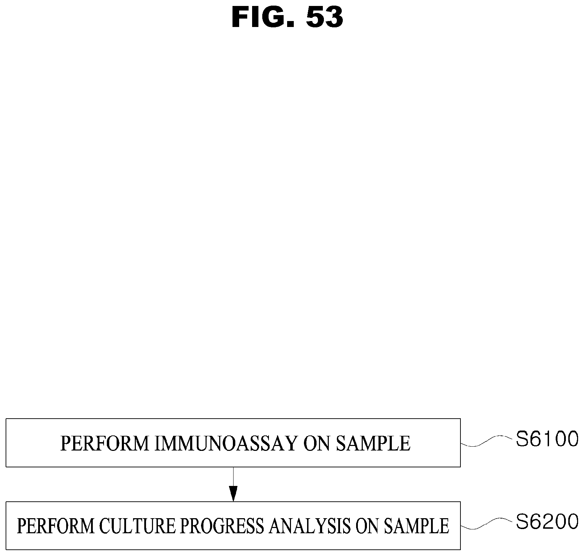

FIG. 53 is a view for describing multiple types of diagnostic methods for a sample SA in cancer diagnosis according to an embodiment of the present application.

The cancer diagnosis according to an embodiment of the present application may be performed using a method which includes performing an immunoassay of a sample SA (S6100) and performing culture progress analysis of the sample SA (S6200).

The above-described embodiments related to an immunoassay may be applied to the immunoassay on the sample SA.

The above-described embodiments related to culture progress analysis may be applied to the culture progress analysis on the sample SA.

The performing of the immunoassay on the sample SA refers to analyzing an immune system of cells CE included in the sample SA. Using antibodies AB related to proteins distributed on surfaces of cells CE included in the sample SA, characteristics of the cells CE (for example, whether the cells CE are tumor cells CE) may be analyzed.

According to circumstances, the morphology of the cells CE may also be analyzed using antibodies AB that are bound to proteins on surfaces of cells CE due to binding between antigens and the antibodies AB. This corresponds to a method in which schematic external morphology of the cells CE is estimated using labels bound to the antibodies AB.

The performing of the culture progress analysis on the sample SA includes culturing the sample SA on which the above-described immunoassay has been performed. Since the culture progress analysis on the sample SA has already been described above, detailed description thereof will be omitted.

The method of cancer diagnosis according to the present embodiment will be described in more detail below.

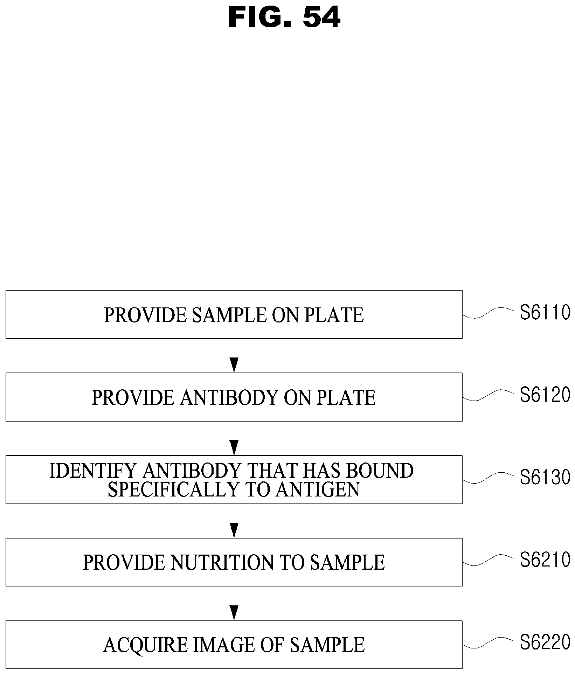

FIG. 54 is a view for describing multiple types of diagnostic methods for a sample SA in cancer diagnosis according to an embodiment of the present application.

When a sample SA is provided on the plate PL (S6110), antibodies AB may be provided on a plate PL (S6120). The antibodies AB provided on the plate PL may bind specifically to antigens included in the sample SA. The patch PA may contain the antibodies AB. The antibodies AB may be provided on the plate PL by the patch PA.

The providing the antibodies AB may be performed using any of the above-described direct technique, indirect technique, and sandwich technique.

After the antibodies AB are provided on the plate PL, antibodies AB that have bound specifically to antigens may be identified (S6130). For example, when a fluorescent substance is attached to the antibodies AB, fluorescence of the sample SA may be detected. As another example, when an enzyme is attached to the antibodies AB, a substrate SU may be provided to the sample SA, and color development of the sample SA may be detected. Using the above-described methods, the presence of antibodies AB that have bound specifically to antigens included in the sample SA may be identified, and in this way, whether cancer has developed may be identified.

Step S6110 may be performed similarly to the above-described Step S2100, Step S6120 may be performed similarly to the above-described Step S2200, and Step S6130 may be performed similarly to the above-described Step S2300.

Since providing nutrition to the sample SA provided on the plate PL (S6210) and acquiring an image of the sample SA (S6220) may be performed similarly to the above-described Steps S5210 and S5220, detailed description thereof will be omitted.

### 7.2.3 Third embodiment

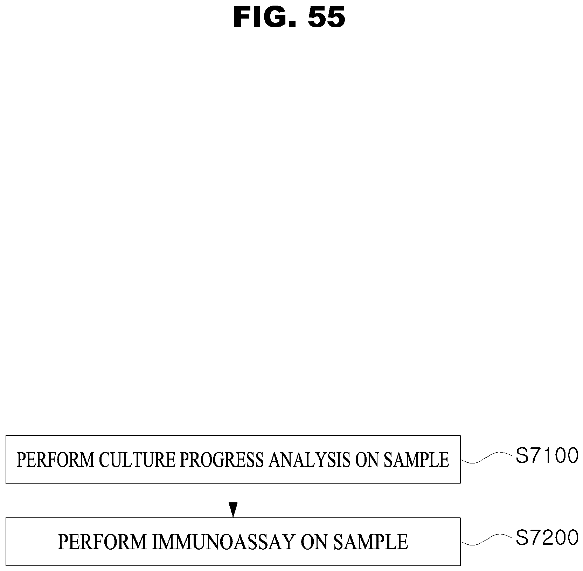

FIG. 55 is a view for describing multiple types of diagnostic methods for a sample SA in cancer diagnosis according to an embodiment of the present application.

After culture progress analysis on the sample SA is performed, any one of morphological analysis, immunoassay, and DNA analysis may be performed on the sample SA.

When the culture progress analysis is performed, cells CE included in the sample SA may proliferate. Therefore, when cancer diagnosis using another method is performed after the culture progress analysis is performed, a sample SA that includes proliferated cells CE may be analyzed, and a more accurate cancer diagnosis may be possible.

To assist in understanding the present application, a case in which culture progress analysis on a sample SA is performed (S7100) and then immunoassay on the sample SA is performed (S7200) will be described below as an example.

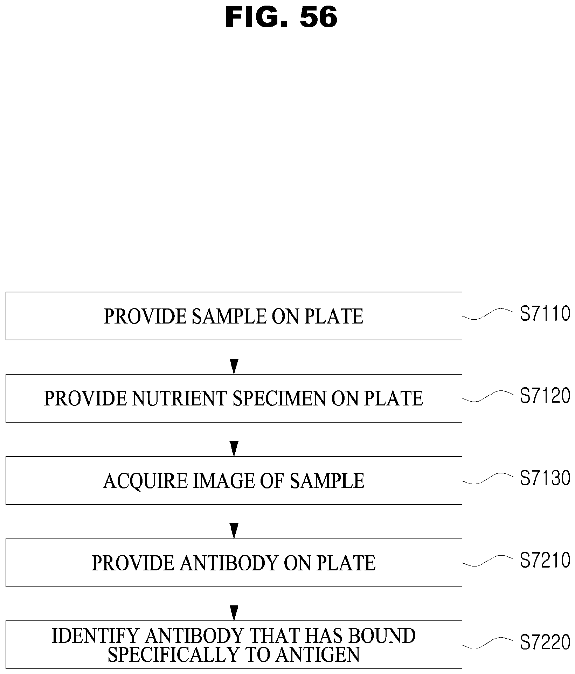

FIG. 56 is a view for describing multiple types of diagnostic methods for a sample SA in cancer diagnosis according to an embodiment of the present application.

When a sample SA is provided on the plate PL (S7110), a nutrient substance NT may be provided on the plate PL (S7120). The providing the nutrient substance NT may be performed by the patch PA. The patch PA may contain the nutrient substance NT.

The nutrient substance NT provided on the plate PL may also be provided to the sample SA located on the plate PL and an environment that allows cells CE included in the sample SA to proliferate may be provided.

In addition, a buffer patch PA may be used to suitably adjust the pH and/or salt concentration of the sample SA. The buffer patch PA may be brought into contact with the plate PL or brought into contact with the nutrient patch PA.

After the nutrient substance NT is supplied to the plate PL for an arbitrary amount of time, an image of the sample SA may be acquired (S7130) and analyzed.

Alternatively, an image of the sample SA may be acquired (S7130) while the nutrient substance NT is supplied to the plate PL so that cells CE included in the sample SA are analyzed in real time.

Alternatively, an image of the sample SA may not be acquired. That is, instead of being a procedure for acquiring an image of the sample SA, the culture progress analysis on the sample SA may be a procedure for proliferating cells CE in a sample SA to be used in various diagnoses which will be performed afterwards. In this way, by performing various diagnoses using the proliferated cells CE, the efficiency of detecting circulating tumor cell CE distributed in the blood may be increased.

Since providing antibodies AB on the plate PL (S7210) and identifying antibodies AB that have bound specifically to antigens (S7220) may be performed similarly to the above-described Steps S6120 and S6130, detailed description thereof will be omitted.

### 7.2.4 Fourth embodiment

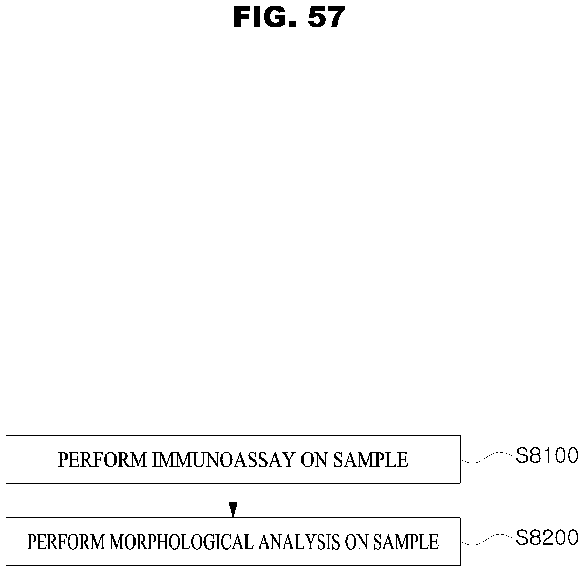

FIG. 57 is a view for describing multiple types of diagnostic methods for a sample SA in cancer diagnosis according to an embodiment of the present application.

The cancer diagnosis according to an embodiment of the present application may be performed using a method in which an immunoassay of a sample SA is performed (S8100) and morphological analysis of the sample SA is performed (S8200).

The above-described embodiments related to an immunoassay may be applied to the immunoassay on the sample SA.

The above-described embodiments related to morphological analysis may be applied to the morphological analysis on the sample SA.

The morphological analysis on the sample SA may be performed first, and then the immunoassay on the sample SA may be performed. Alternatively, like the present embodiment, the immunoassay on the sample SA may be performed first, and then the morphological analysis on the sample SA may be performed.

When staining of the sample SA is performed while the morphological analysis on the sample SA is performed, some of the cells CE included in the sample SA may be destroyed. This is related to the fact that cells CE may undergo necrosis due to a dye. Therefore, it is understood that, when staining of the sample SA is performed during the morphological analysis, a more accurate examination of the sample SA may be possible if the immunoassay on the sample SA is performed first.

The immunoassay on the sample SA (S8100) may be performed using any of the above-described direct technique, indirect technique, and sandwich technique.

The morphological analysis on the sample SA (S8200) may be performed using the above-described acquisition of an image of an unstained sample SA or acquisition of an image of a sample SA that is stained using any one of the simple staining technique, the Romanowsky staining technique, and the DAPI staining technique.

However, staining techniques are not limited thereto, and staining using other staining techniques not disclosed herein may also be used in the above-described morphological analysis.

The embodiments in which multiple types of diagnoses on the sample SA are performed in performing cancer diagnosis according to the present application have been described above.

However, the above-described embodiments are merely a few exemplary embodiments, and the scope of the present application is not limited by the above-described embodiments.

Also, although, for convenience of description, embodiments in which two types of diagnoses are sequentially performed has been described, cases in which cancer diagnosis is performed using three or more types of diagnoses may also be easily practiced by those of ordinary skill in the art.

### 8. Multi-region

In cancer diagnosis according to the present application, when multiple types of diagnostic methods are applied to a single sample SA, the multiple types of diagnostic methods may be simultaneously performed.

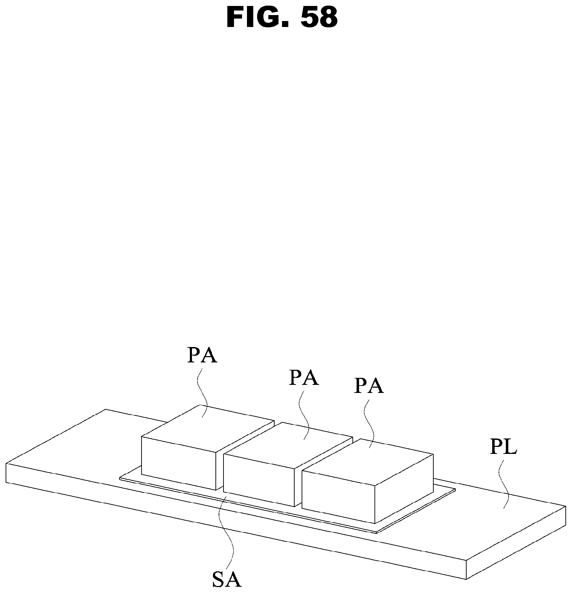

FIG. 58 is a view for describing multiple types of diagnostic methods for a sample SA in cancer diagnosis according to an embodiment of the present application.

A plurality of patches PA may be brought into contact with the plate PL. The plurality of patches PA may contain different reagents.

For example, the plurality of patches PA may include a first patch PA, a second patch PA, and a third patch PA.

The first patch PA may contain a stain. The staining reagent may be a specimen for staining a nucleus and/or cytoplasm of cells CE included in the sample SA.

The second patch PA may contain antibodies AB. The antibodies AB may be antibodies AB that react specifically with tumor cells CE included in the sample SA.

The third patch PA may contain a nutrient substance NT. The nutrient substance NT may be a specimen used for proliferation of cells CE included in the sample SA through division of the cells CE.

The first patch PA may be brought into contact with a first region of the plate PL. The second patch PA may be brought into contact with a second region of the plate PL. The third patch PA may be brought into contact with a third region of the plate PL. As a result, cells CE included in the sample SA located in the first region may be stained, some of the antibodies AB may bind to surfaces of cells CE included in the sample SA located in the second patch PA, and cells CE included in the sample SA located in the third patch PA may proliferate.

Images of the first region, the second region, and the third region of the plate PL may be acquired. The images of the first region, the second region, and the third region may be separately acquired or acquired in a single frame. Alternatively, the images of the first region, the second region, and the third region may also be acquired in a form in which an image of each part of a single image is acquired separately, and the acquired images are combined into the single image.

The multiple types of diagnostic methods disclosed in Multi-region section and the multiple types of diagnostic methods disclosed in Multi-test section may be combined with each other. For example, after cell CE culture analysis is performed on a region in which the sample SA is provided, the region of the sample SA on which the cell CE culture analysis has been performed may be divided, and multiple types of diagnoses may be performed in divided regions.

### 9. Diagnostic device

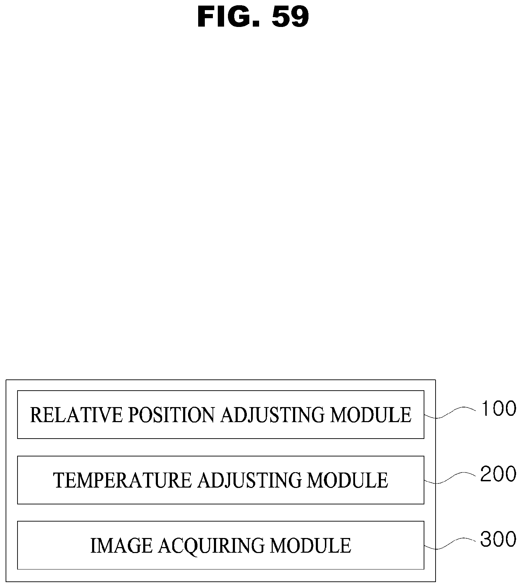

FIG. 59 is a block diagram of a diagnostic device according to an embodiment of the present application.

A diagnostic device according to the present application may include a relative position adjusting module 100, a temperature adjusting module 200, and an image acquiring module 300. The diagnostic device according to the present application may also include more or less elements.

The relative position adjusting module 100 may perform a function of relatively moving the patch PA and the plate PL to each other. The relative position adjusting module 100 may relatively move the patch PA and the plate PL to each other in a horizontal direction and/or a vertical direction.

The horizontal direction may refer to a direction parallel to a surface at which the plate PL and the patch PA come into contact. The vertical direction may refer to a direction perpendicular to the surface at which the plate PL and the patch PA come into contact.

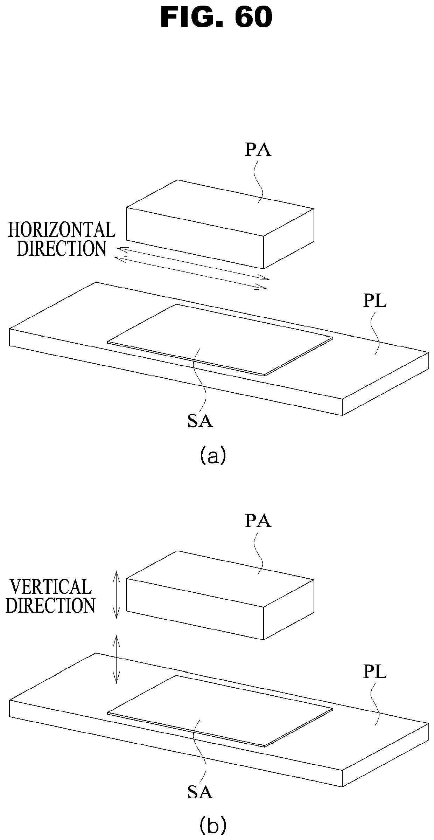

FIG. 60 is a conceptual diagram illustrating an example in which a structure of a diagnostic device is moved due to a relative movement operation of the relative position adjusting module 100 according to an embodiment of the present application.

Referring to FIG. 60(a), the relative position adjusting module 100 may relatively move the patch PA and the plate PL to each other in the horizontal direction and change a relative position of the patch PA on the plate PL.

The relative position adjusting module 100 may relatively move the patch PA and the plate PL to each other in the horizontal direction and perform a function of changing a patch PA that is disposed to be capable of coming into contact with the sample SA. The changing of the patch PA that is disposed to be capable of coming into contact with the sample SA may allow a liquid substance provided from another patch PA to be delivered to the sample SA.

Referring to FIG. 60(b), the relative position adjusting module 100 may relatively move the patch PA and the plate PL to each other in the vertical direction and control whether the plate PL and the sample SA are brought into contact. The bringing of the patch PA and the sample SA into contact may become involved in delivery of a substance captured in the patch PA to the sample SA.

The relative position adjusting module 100 may include a moving power source configured to relatively move the patch PA and the plate PL to each other in the horizontal direction and a other moving power source configured to relatively move the patch PA and the plate PL to each other in the vertical direction. Alternatively, the relative position adjusting module 100 may use a single moving power source to move the patch PA and the plate PL in the horizontal and/or vertical directions.

The temperature adjusting module 200 may perform a function of controlling a temperature. The temperature adjusting module 200 may perform heating or cooling of the plate PL and/or the patch PA. Also, the temperature adjusting module 200 may perform a function of adjusting a temperature of the sample SA and maintaining a constant temperature.

For example, the temperature adjusting module 200 may be used to adjust a temperature of the sample SA to the above-described denaturation temperature, annealing temperature, and/or extension temperature.

The temperature adjusting module 200 may perform an exothermic reaction and an endothermic reaction. Consequently, the temperature adjusting module 200 may include a heating element or a thermoelectric element. The temperature adjusting module 200 is not limited thereto, and any substance capable of heating may be used as the temperature adjusting module 200 without limitations.

The temperature adjusting module 200 may further include a temperature sensor as necessary. The temperature sensor may be used to identify a current temperature of a target that is subject to temperature adjustment.

The image acquiring module 300 may perform a function of acquiring an image of the sample SA. For example, the image acquisition may be performed by a method in which an image of a portion of a plate PL or an entire plate PL is acquired, a method in which an image of a portion of a patch PA or an entire patch PA is acquired, or a method in which an image of the sample SA is directly acquired.

The image acquiring module 300 may include a means for acquiring an image. For example, the image acquiring module 300 may include an image generator configured to generate an image, such as an image sensor including a complementary metal oxide semiconductor (CMOS) image sensor and a charge-coupled device (CCD) image sensor, a predetermined light generator configured to generate light that transmits through the sample SA, and/or an optical system configured to form an image of light that has transmitted through the sample SA.

The image acquiring module 300 may directly acquire an image of a sample SA smeared on a plate PL. Here, the image acquiring module 300 may receive light that has been irradiated from a light source and transmitted through the plate PL on which the sample SA is smeared, and acquire an image of the sample SA.

For example, the image acquiring module 300 may be disposed on a surface of a slide glass on which a sample SA is smeared (hereinafter referred to as "front surface"), and the light source may be disposed at a surface opposite the front surface of the slide glass, i.e., a rear surface of the slide glass. Due to such arrangements, the image acquiring module 300 may receive light that has been irradiated from the light source from the rear surface side of the slide glass and has passed through the slide glass, and acquire an image of the sample SA.

As another example, the image acquiring module 300 may be disposed on the rear surface of the slide glass, and the light source may be disposed on the front surface of the slide glass. Due to such arrangements, the image acquiring module 300 may receive light that has been irradiated from the light source from the front surface side of the slide glass and has passed through the slide glass, and acquire an image of the sample SA.

The image acquiring module 300 may detect fluorescence or acquire a fluorescence image for quantitative and/or qualitative analysis of the sample SA.

An image generated from the image acquiring module 300 may have various magnifications. For example, the image may be an image with a magnification that enlarges the sample SA, an image with a fixated magnification, or may also be an image with a magnification that reduces the sample SA as necessary.

The image acquiring module 300 may also include a power member configured to move the plate PL on which the sample SA is located or move an element of the image acquiring module 300, thereby acquiring an image of the sample SA.

The diagnostic apparatus according to the present application may perform the PCR process described above. Description of the process in terms of a mechanical aspect will be omitted since it is deemed that those of ordinary skill in the art to which the present application pertains would be able to easily understand the process without repetitive description of the details.

The above description is merely illustrative of the technical spirit of the present application, and those of ordinary skill in the art to which the present application pertains should be able to make various modifications and changes within a scope not departing from essential characteristics of the present application. Therefore, the above-described embodiments of the present application may also be implemented separately or in combination.

The embodiments disclosed herein are for describing the technical spirit of the present application instead of limiting the same, and the scope of the technical spirit of the present application is not limited by such embodiments. The scope of the present application should be interpreted on the basis of the claims below, and all technical spirits within the equivalent scope should be interpreted as belonging to the scope of the present application.

## Claims

1. A diagnostic method for performing diagnosis on tumor cells included in a sample by using a patch which is provided as a gel type having a net-like structure forming micro-cavities, the diagnostic method comprising:
placing the sample on a plate; and
providing a reagent included in the patch to the plate with using the patch that contains the reagent used to detect cancer.

2. The diagnostic method of claim 1, wherein the placing the sample on the plate includes providing the sample in a single layer on the plate.

3. The diagnostic method of claim 1, wherein the sample is blood.

4. The diagnostic method of claim 2, wherein the placing the sample on the plate includes fixating the sample on the plate.

5. The diagnostic method of claim 1, wherein the reagent contained in the patch include an antibody that reacts specifically with the tumor cells.

6. The diagnostic method of claim 5, further comprising providing a substrate on the plate in order to identify an antibody bound to the tumor cells.

7. The diagnostic method of claim 5, wherein the antibody that reacts specifically with the tumor cells is applied on the plate.

8. The diagnostic method of claim 1, wherein the reagent contained in the patch includes a staining reagent used in staining cells in order to stain the tumor cells.

9. The diagnostic method of claim 8, wherein the staining reagent targets at least one of a nucleus of the cells, a cytoplasm of the cells, and DNA distributed in the cells.

10. The diagnostic method of claim 9, further comprising adjusting a temperature of the sample in order to provide a reaction environment for the staining reagent.

11. The diagnostic method of claim 1, wherein the reagent contained in the patch includes a nutrient reagent used in cell culturing in order to diagnose the cancer by culturing the tumor cells.

12. The diagnostic method of claim 1, wherein the providing the reagent contained in the patch includes contacting the patch and the plate.

13. The diagnostic method of claim 1, further comprising acquiring an image of the plate on which the sample is placed in order to perform diagnosis of the tumor cells.

14. The diagnostic method of claim 13, wherein the image of the plate on which the sample is placed is a fluorescence image.

15. The diagnostic method of claim 1, wherein the reagent contained in the patch includes a reagent used for removing a cell membrane of a cell in order to extract DNA of the tumor cells.

16. A diagnostic method for performing diagnosis on tumor cells included in a sample by using a patch which is provided as a gel type having a net-like structure forming micro-cavities, the diagnostic method comprising:
placing the sample on a plate;
providing a first reagent contained in a first patch to the plate with using the first patch containing the first reagent used to detect cancer; and
providing a second reagent contained in a second patch to the plate with using the second patch containing the second reagent used to detect the cancer.

17. The diagnostic method of claim 16, wherein the providing the reagent contained in the first patch is performed prior to the providing the reagent contained in the second patch.

18. The diagnostic method of claim 16, wherein:
the first reagent includes an antibody that reacts specifically with the tumor cells; and
the second reagent includes an antibody that binds to the antibody reacting specifically with the tumor cells.

19. The diagnostic method of claim 16, wherein:
the first reagent includes an antibody that reacts specifically with the tumor cells; and
the second reagent includes a nutrient reagent that is used in cell culturing in order to diagnose the cancer by culturing the tumor cells.

20. The diagnostic method of claim 16, wherein:
the first reagent includes an antibody that reacts specifically with the tumor cells; and
the second reagent includes a staining reagent that is used for cell staining in order to stain the tumor cells.

21. The diagnostic method of claim 16, wherein:
the first reagent includes an antibody that reacts specifically with the tumor cells; and
the second reagent includes an antibody that reacts specifically with white blood cells.

22. The diagnostic method of claim 21, further comprising, between the providing the reagent contained in the first patch and the providing the reagent contained in the second patch, acquiring an image of the sample.

23. The diagnostic method of claim 16, wherein:
the first reagent includes a reagent used for removing a cell membrane of a cell in order to extract DNA of the tumor cells; and
the second reagent includes a reagent used in a polymerase chain reaction (PCR).

24. The diagnostic method of claim 23, further comprising adjusting a temperature of the sample in order to cause the PCR.

25. A diagnostic apparatus for performing diagnosis on tumor cells included in a sample by using a patch which is provided as a gel type having a net-like structure forming micro-cavities and contains reagent used to detect cancer, the diagnostic device comprising:
a relative movement adjusting module configured to relatively move the patch and a region in which the sample is provided to each other to provide the reagent contained in the patch to the sample; and
an image acquiring module configured to acquire an image of the sample for cancer diagnosis.

26. The diagnostic apparatus of claim 25, further comprising a temperature adjusting module configured to adjust the temperature of the sample.
